# EUROPEAN PATENT APPLICATION

(11) **EP 4 834 361 A2**
(43) Date of publication of application: **04.11.2026**
(21) Application number: 26188674.1
(22) Date of filing: 19.12.2024
(51) Int. Cl.: A61K 39/00

(54) **BROADLY NEUTRALIZING ANTIBODIES AGAINST SARS-COV-2 AND SARS-COV VARIANTS**

(30) Priority: 21.12.2023 US 202363613327 P
(62) Document number(s) of the earlier application(s) in accordance with Art. 76 EPC: 24846914.0 / 4 828 727
(71) Applicant: The United States of America, as represented by The Secretary, Department of Health and Human Services, Bethesda, MD 20892-7788 (US)
(72) Inventor: WANG, Lingshu, Bethesda, Maryland, 20814 (US); DORIA-ROSE, Nicole Amy, Bethesda, Maryland, 20892 (US); KOUP, Richard Alan, Bethesda, Maryland, 20892 (US); MISASI, John Nicholas, Bethesda, Maryland, 20892 (US); DOUEK, Daniel Ceasar, Bethesda, Maryland, 20892 (US); SCHRAMM, Chaim Aryeh, New York, New York, 10025 (US); MUSAYEV, Maryam, Bethesda, Maryland, 20892 (US); BUSH, Sabrina Marie, Bethesda, Maryland, 20892 (US); MCKIM, Quenelle Washington, Bethesda, Maryland, 20892 (US); YANG, Eun Sung, Bethesda, Maryland, 20892 (US); SHI, Wei, Bethesda, Maryland, 20892 (US); HENRY, Amy Ransier, Bethesda, Maryland, 20892 (US); SMITH, Sarah Catherine, Bethesda, Maryland, 20892 (US); ZHOU, Tongqing, Bethesda, Maryland, 20892 (US); PIERSON, Theodore, Bethesda, Maryland, 20892 (US)
(74) Representative: Vossius & Partner Patentanwälte Rechtsanwälte mbB

(57) **Abstract**

Disclosed are monoclonal antibodies, antigen binding fragments, and multi-specific antibodies that specifically bind a coronavirus spike protein, such as SARS-CoV-2. Also disclosed is the use of these antibodies and multi-specific antibodies for inhibiting a coronavirus infection, such as a SARS-CoV-2 infection. In addition, disclosed are methods for detecting a coronavirus, such as SARS-CoV-2, in a biological sample, using the disclosed antibodies and multi-specific antibodies.

## Description

### CROSS REFERENCE TO RELATED APPLICATIONS

This claims the benefit of U.S. Provisional Application No. 63/613,327, filed December 21, 2023, which is incorporated herein by reference in its entirety.

### FIELD

This relates to monoclonal antibodies and antigen binding fragments that specifically bind a severe acute respiratory syndrome coronavirus (SARS-CoV)-2 spike protein, and their use for inhibiting a SARS-CoV-2 infection and detecting SARS-CoV-2 in a biological sample.

### SEQUENCE LISTING

The Sequence Listing is submitted as an XML file in the form of the file named "Sequence.xml" (204,472 bytes), which was created on October 30, 2024, which is incorporated by reference herein.

### BACKGROUND

SARS-CoV-2, the virus that causes COVID-19, continues to infect millions of people and is a major cause of death among the elderly. Monoclonal antibodies targeting the SARS-CoV-2 spike protein have been used clinically, either given prophylactically to prevent infection, or as a treatment to prevent severe disease. The former is especially important for immunocompromised persons, including cancer patients, for whom vaccines may be ineffective or contraindicated. However, SARS-CoV-2 variants emerge frequently, and often bear amino acid sequence changes in the spike protein that confer escape from such monoclonals. Indeed, as of November 2023, all previously U.S. Food and Drug Administration (FDA)- authorized monoclonal products, including the therapeutic antibodies bamlaminivab and bebtelovimab, and the prophylactic formulation EVUSHELD^{™} (mix of tixagevimab and cilgavimab), are no longer authorized because recent variants are not neutralized by these products. Therefore, new monoclonals needed for clinical use that have broader cross-reactivity to viral variants.

### SUMMARY

Isolated monoclonal antibodies or antigen binding fragments thereof are disclosed that specifically binds to a coronavirus spike protein. These monoclonal antibodies or antigen binding fragments can neutralize at least one coronavirus.

In some embodiments, the antigen or antigen binding fragment includes a heavy chain variable (V_{H}) region and a light chain variable region (V_{L}) comprising a heavy chain complementarity determining region (HCDR)1, a HCDR2, and a HCDR3, and a light chain complementarity determining region (LCDR)1, a LCDR2, and a LCDR3 of the V_{H} and V_{L} set forth as any one of:
a) SEQ ID NOs: 1 and 5, respectively (A18-618-448.1);
b) SEQ ID NOs: 9 and 13, respectively (F768-104_B2);
c) SEQ ID NOs: 17 and 21, respectively (E184-105_F3);
d) SEQ ID NOs: 25 and 29, respectively (A63-652-32.1);
e) SEQ ID NOs: 33 and 37, respectively (F768-104_G7);
g) SEQ ID NOs: 41 and 45, respectively (E184-105_H1);
f) SEQ ID NOs: 49 and 53, respectively (F768-104_D10);
h) SEQ ID NOs: 57 and 61, respectively (A63-652-1.4);
i) SEQ ID NOs: 65 and 69, respectively (E184-105_E5);
j) SEQ ID NOs: 73 and 77, respectively (E184-105_H9);
k) SEQ ID NOs: 81 and 85, respectively (E184-105_H2);
l) SEQ ID NOs: 89 and 93, respectively (A80-102+103_G1);
m) SEQ ID NOs: 97 and 101, respectively (A80-103_E2);
n) SEQ ID NOs: 105 and 109, respectively (A80-103_B6);
o) SEQ ID NOs: 113 and 117, respectively (A18-618-452.1);
p) SEQ ID NOs: 121 and 125, respectively (A80-102+103_C1);
q) SEQ ID NOs: 129 and 133, respectively (A43-d321.10.4);
r) SEQ ID NOs: 137 and 141, respectively (F768-100-pt2_A05);
s) SEQ ID NOs: 145 and 149, respectively (A80-102_G5);
t) SEQ ID NOs: 153 and 157, respectively (A18-338-19.5);
u) SEQ ID NOs: 161 and 165, respectively (A18-338-19.6);
v) SEQ ID NOs: 169 and 173, respectively (A18-338-19.8); or
w) SEQ ID NOs: 177 and 181, respectively (A18-338-19.9).

In some embodiments, disclosed are nucleic acid molecules encoding these antibodies and antigen binding fragments, vectors including these nucleic acid molecules, and host cells including these vectors.

Pharmaceutical compositions including the antibodies, antigen binding fragments, nucleic acid molecules, and vectors are also disclosed. In more embodiments, disclosed is the use of these pharmaceutical compositions to inhibit a coronavirus infection in a subject.

In yet other embodiments, disclosed is the use of the disclosed antibodies and antigen binding fragments for the detection of a coronavirus in a biological sample.

The foregoing and other features and advantages of the invention will become more apparent from the following detailed description of several embodiments which proceeds with reference to the accompanying figures.

### BRIEF DESCRIPTION OF THE DRAWINGS

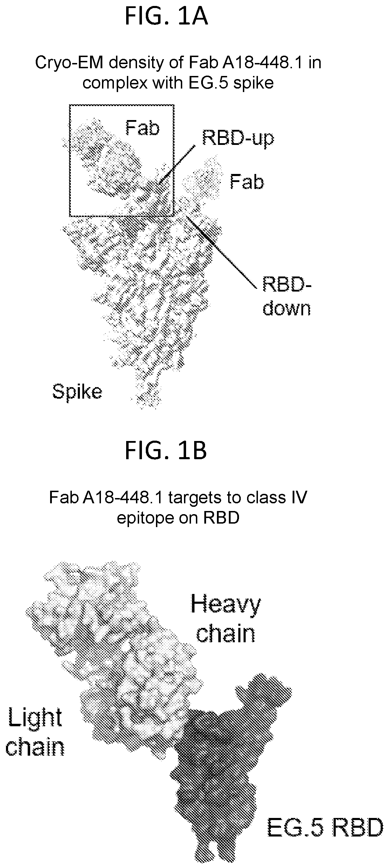

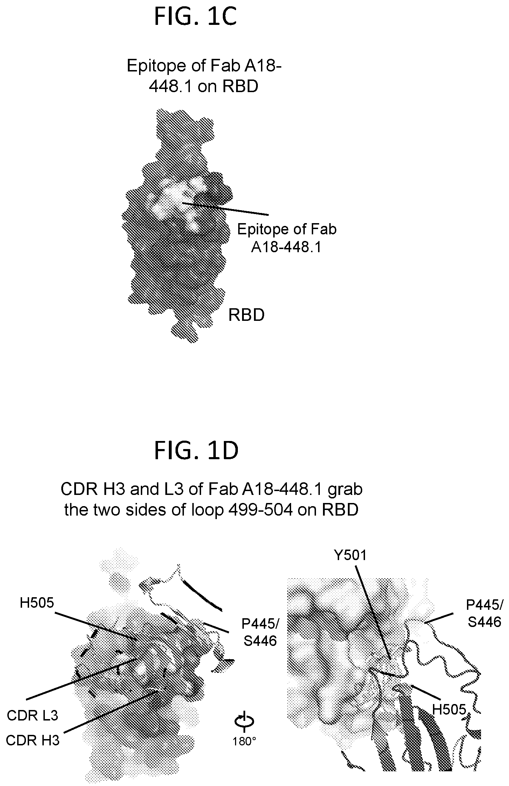

**FIGS. 1A-1D****.** Cryo-EM structure of Fab A18-448.1 in complex with EG.5 spike. FIG. 1A. Cryo-EM density of Fab A18-448.1 in complex with EG.5 spike showed that antibody binds to RBD in both RBD-up and RBD-down conformations. The boxed complex between antibody and RBD in up conformation is shown in FIG. 1B. FIG. 1B. Fab A18-448.1 targets the class IV epitope on RBD. Antibody is shown in lighter gray surface and EG.5 RBD is shown in black surface representation. FIG. 1C. Epitope of A18-448.1 on RBD is relatively small and only includes residues 404-405 and loop 499-504. RBD is shown in black surface with epitope colored gray. FIG. 1D. A18-448.1 utilizes CDR H3 and CDR L3 to bind to the RBD residues 499-504. Omicron mutational hotspots around this region, such as P445, S446 and H505, are not interacting with antibody A18-448.1.

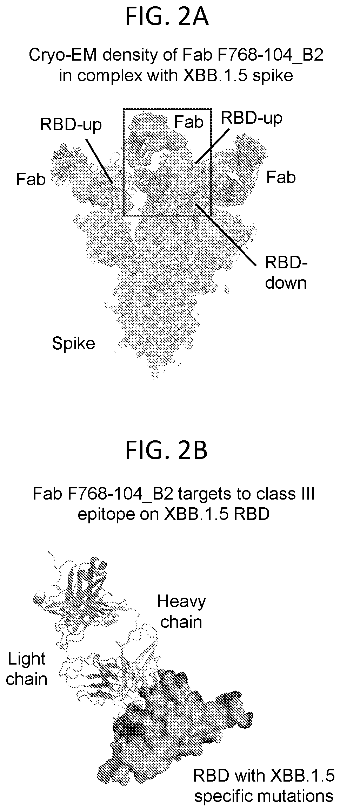

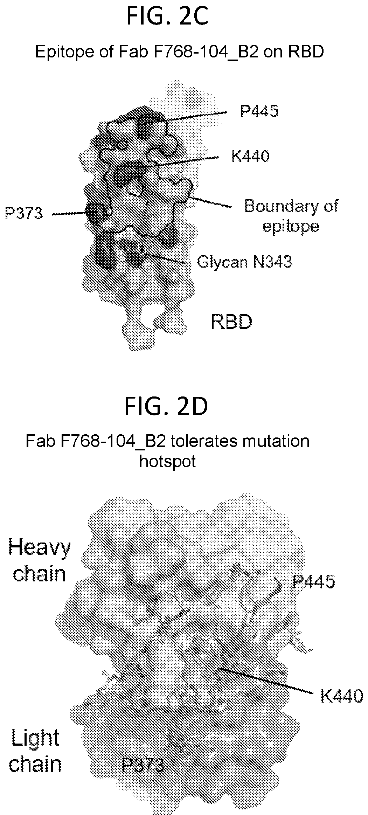

**FIGS. 2A-2D****. Cryo-EM structure of Fab F768-104_B2 in complex with XBB.1.5 spike.** **FIG. 2A****.** Cryo-EM density of Fab F768-104_B2 in complex with XBB.1.5 spike shows that F768-104_B2 binds to RBD in both RBD-up and RBD-down conformations. The boxed antibody-RBD detail is shown in FIG. 2B. FIG. 2B. Fab F768-104_B2 targets class III epitope on XBB.1.5 RBD. RBD is shown in gray surface rendering and specific XBB.1.5 mutations (relative to ancestral) are shown as dark surface. Heavy and light chains of antibody are shown in cartoon form. FIG. 2C. Epitope of F768-104_B2 glycan. Antibody is shown in surface representation with heavy and light chains of antibody in different shades. Epitope residues are shown in sticks with interacting XBB.1.5 mutations labeled. Spike amino acid K440 is in the middle of the epitope and two other mutations, P373 and P445, are at the edge of the epitope. FIG. 2D. F768-104_B2 utilizes a heavy/light chain interface cavity to tolerate variations at the mutation hotspot at RBD residue 440, which is in the middle of the epitope. While K440 points to the central cavity, two other XBB.1.5 mutations, P373 and P445, are at the edge of F768-104_B2 epitope and therefore do not impart resistance.

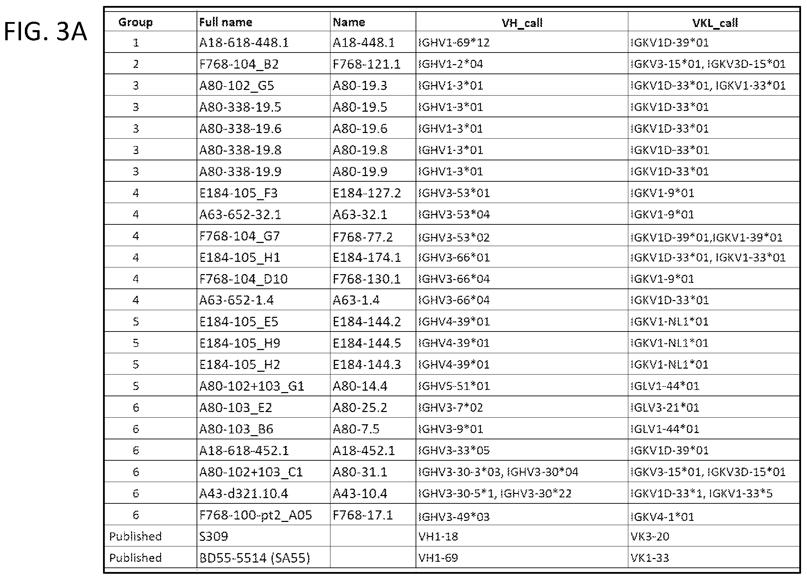

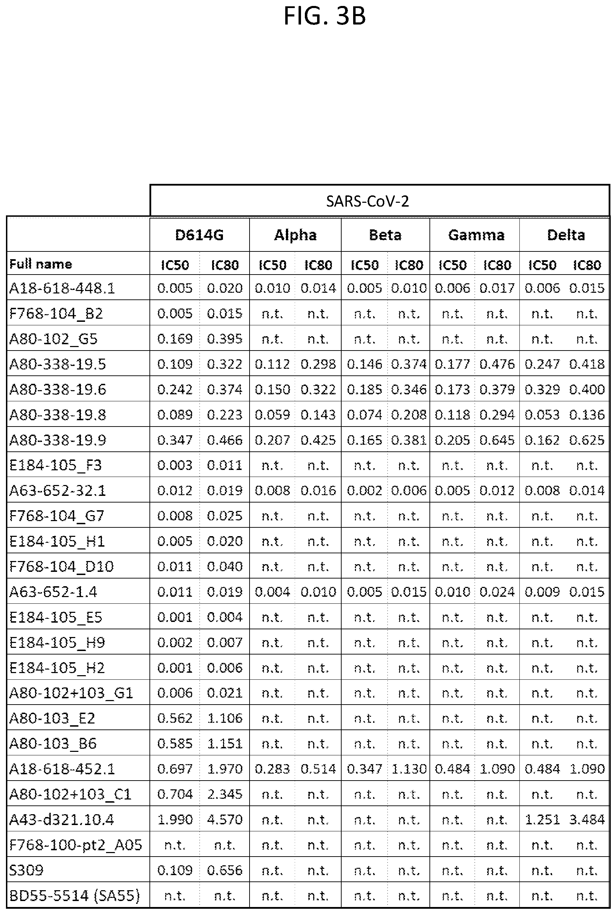

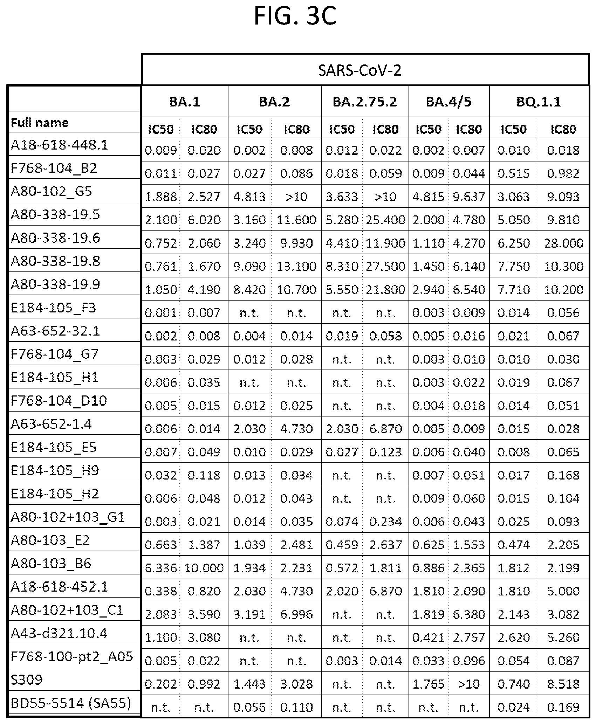

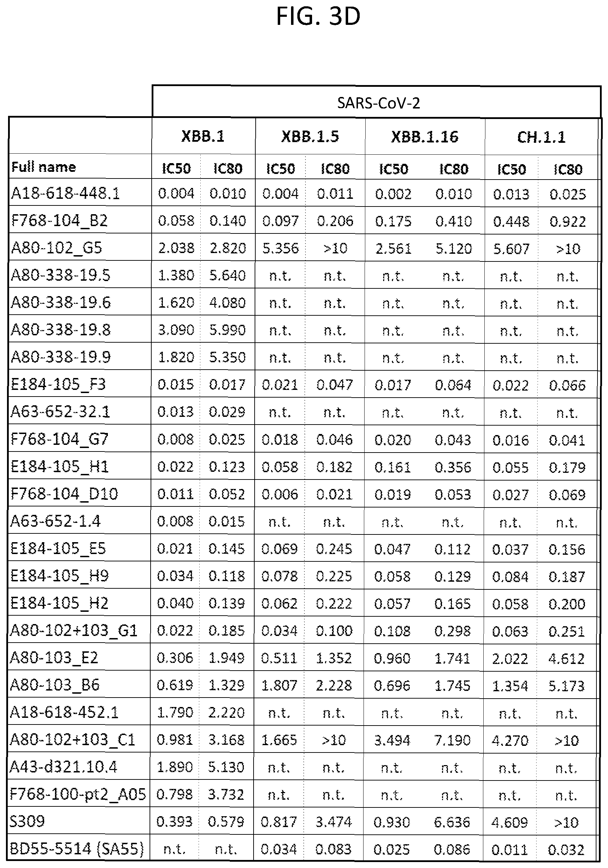

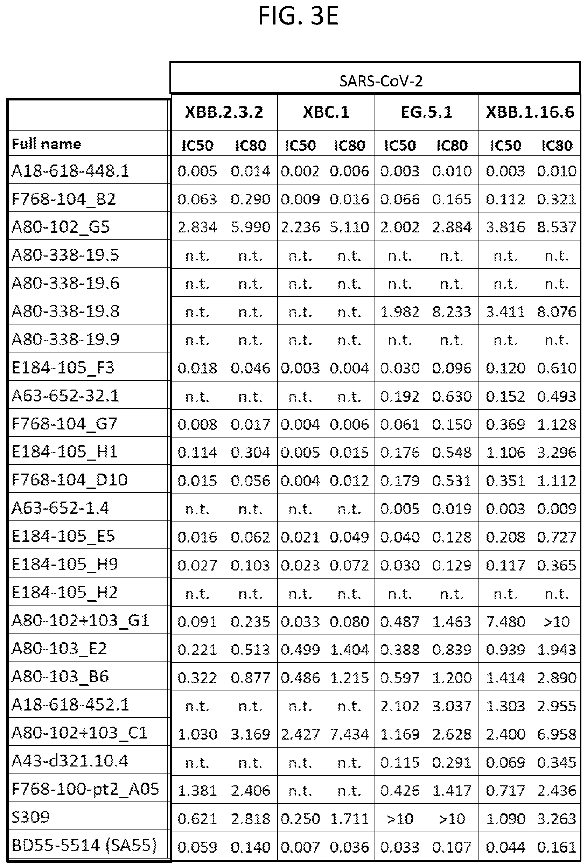

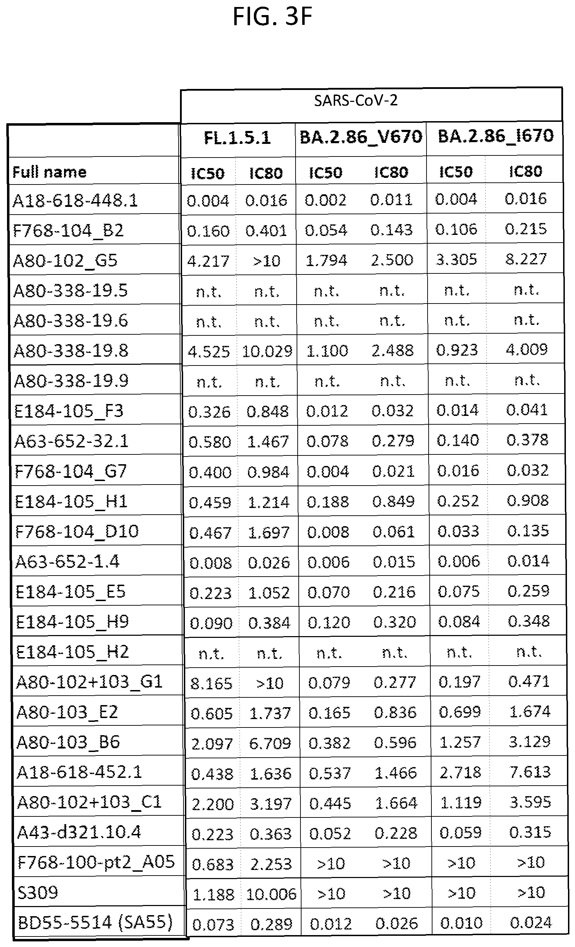

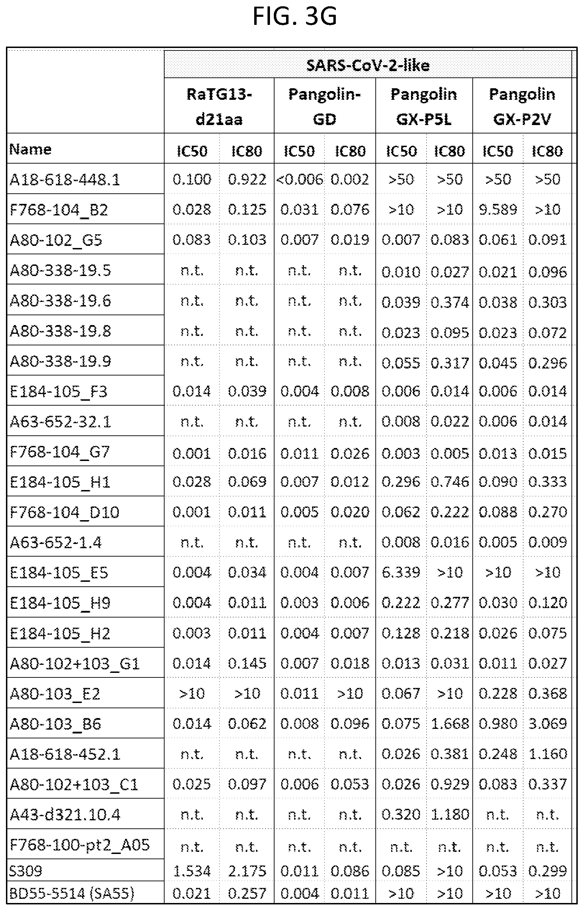

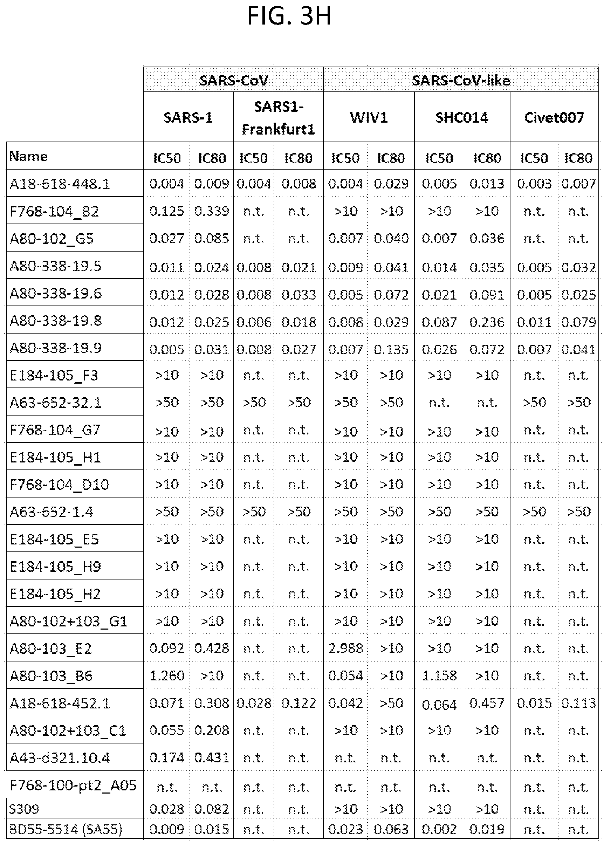

**FIGS. 3A-3H****.** Table providing the genetic derivation of antibodies, and neutralization activity against SARS-CoV-2 variants and sarbecoviruses.

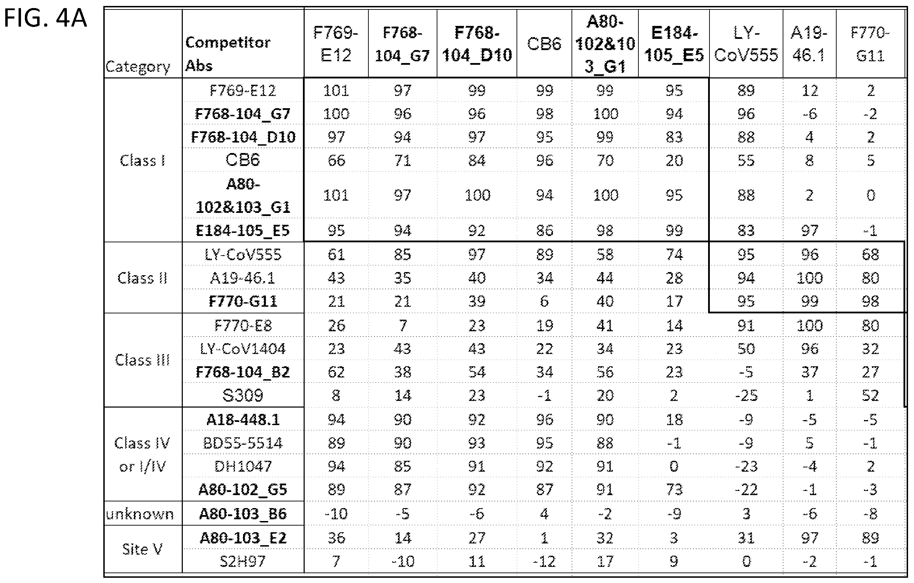

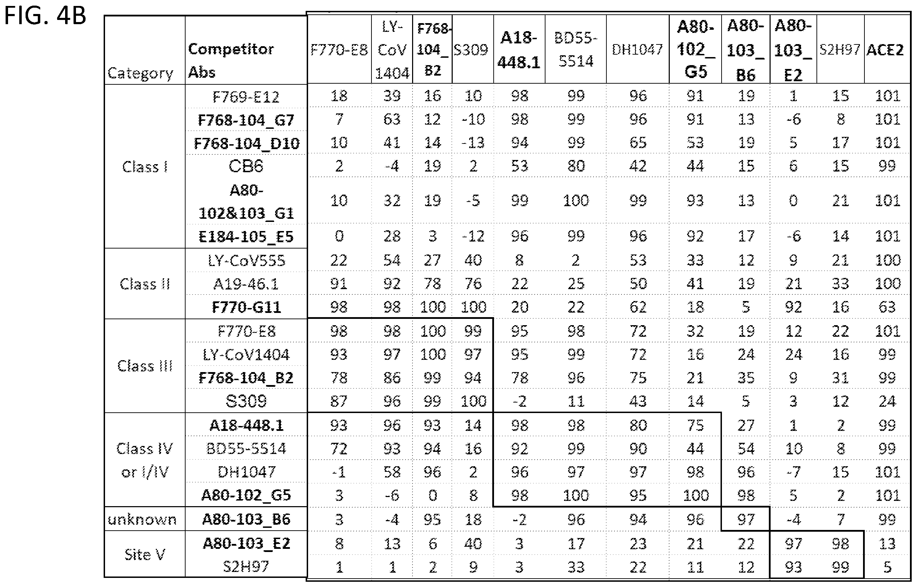

**FIGS. 4A-4B****.** Table providing epitope mapping by competition ELISA

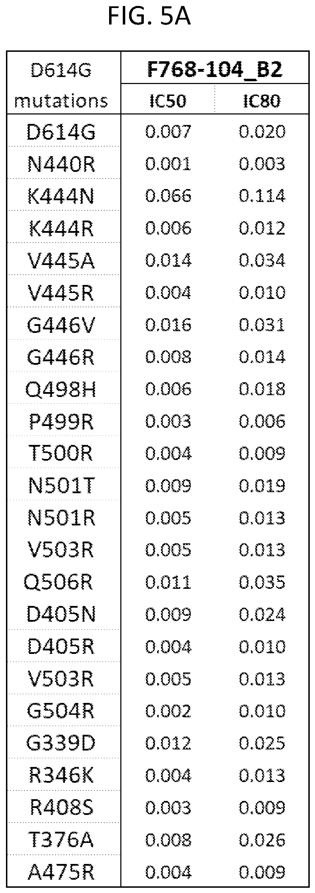

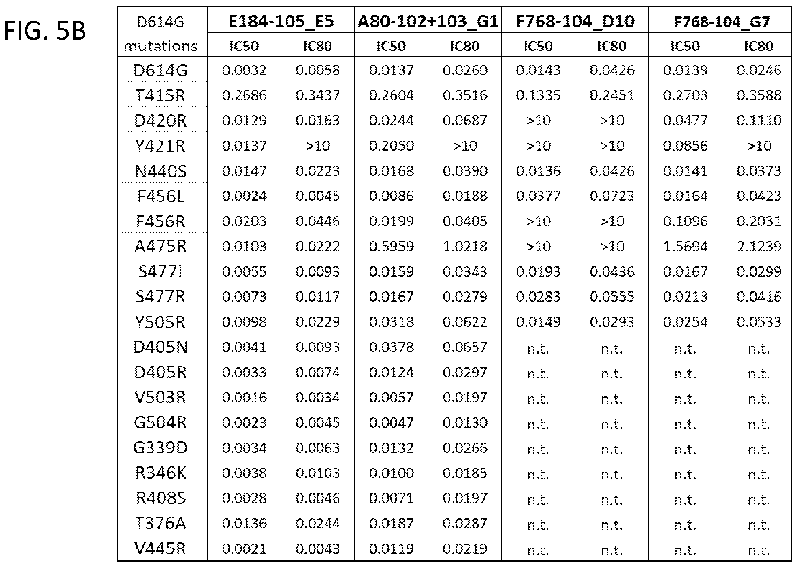

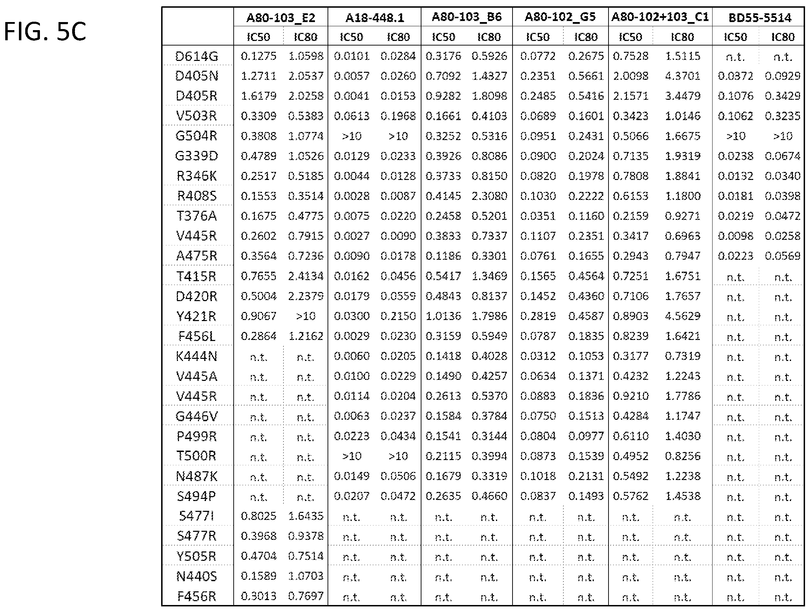

**FIGS. 5A-5C****.** Table providing mutational analysis of selected antibodies

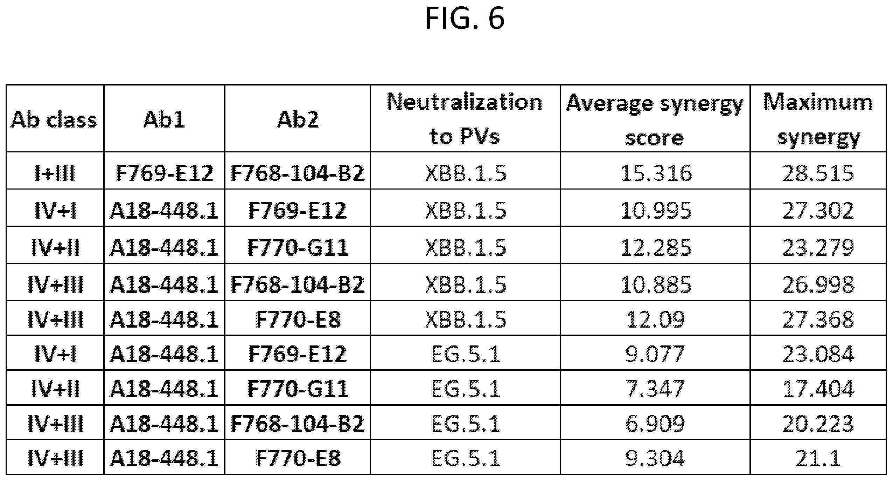

**FIG. 6****.** Table showing combinations of two mAbs synergistically neutralize SARS-CoV-2 variants.

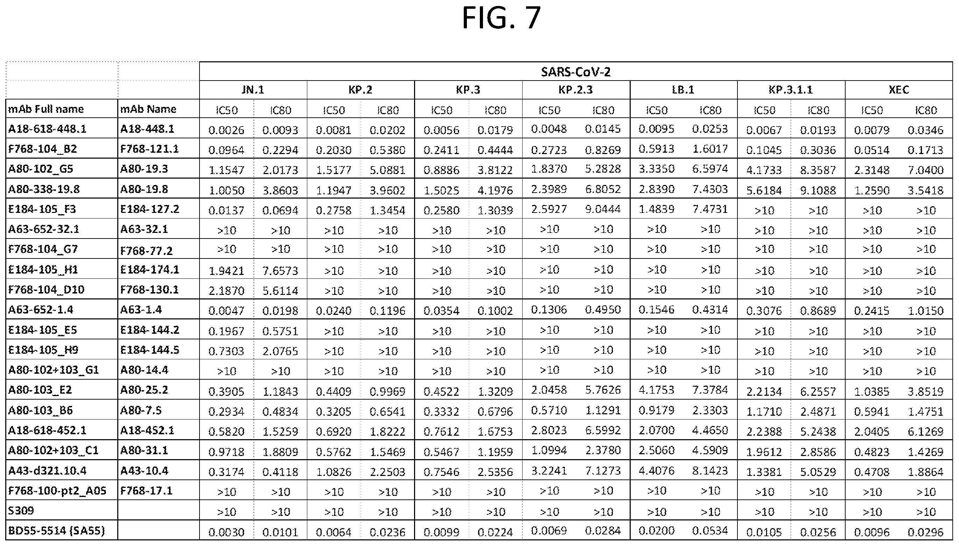

**FIG. 7****.** Table providing monoclonal antibody neutralization of SARS-CoV-2 variants. Lentiviruses pseudotyped with SARS-CoV-2 spike proteins from the indicated variants were incubated with serial dilutions of the indicated antibodies and IC₅₀ and IC₈₀ values determined on 293-TMPRSS2-ACE2 cells. Values are IC₅₀ and IC₈₀ in micrograms/milliliter.

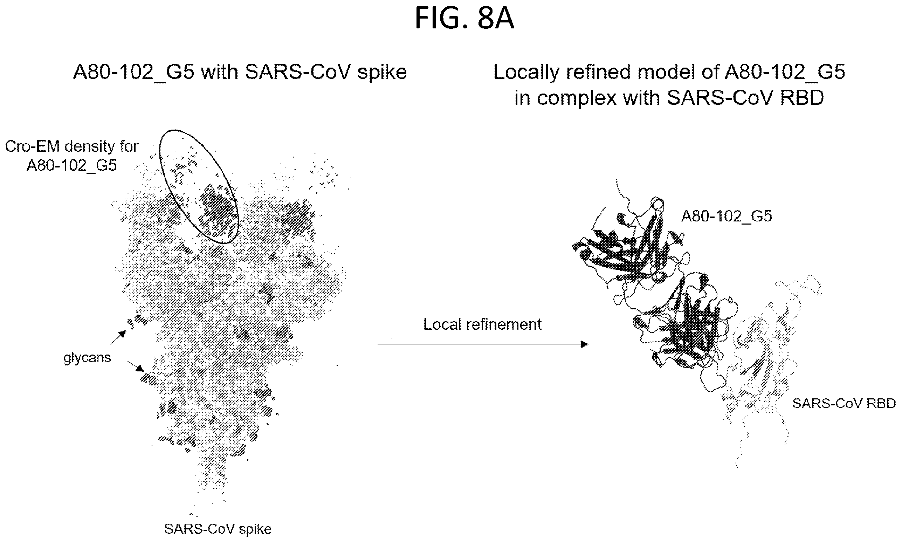

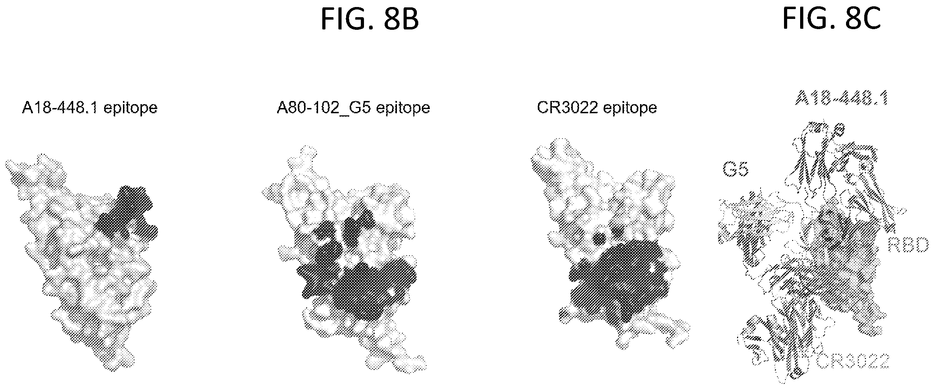

**FIGS. 8A-8C****. Comparison of binding modes and epitopes of class IV mAbs.** FIG. 8A. Structure of A80-102_G5 in complex with SARS-CoV S-2P spike. Left, cryo-EM surface rendering of antibody-spike complex. Right, antibody bound to RBD, both rendered as ribbon diagrams. FIG. 8B. Epitopes of A18-448.1, A80-102_G5, and CR3022. RBD is shown as light gray surface, with antibody binding footprints as black surfaces. FIG. 8C. Comparison of RBD binding modes between A18-448.1, A80-102_G5 and CR3022. RBD is shown as gray surface, while each antibody is shown in cartoon.

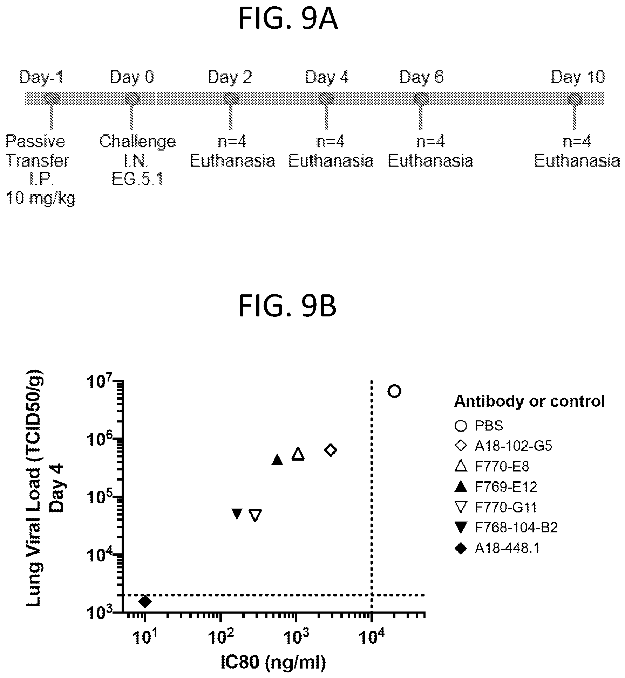

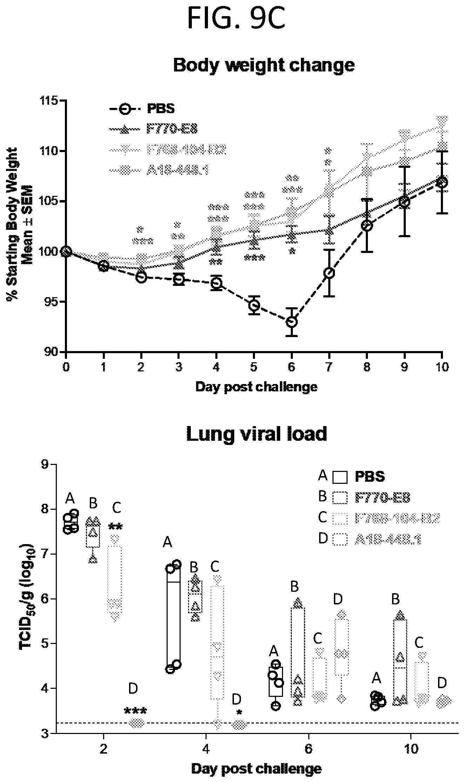

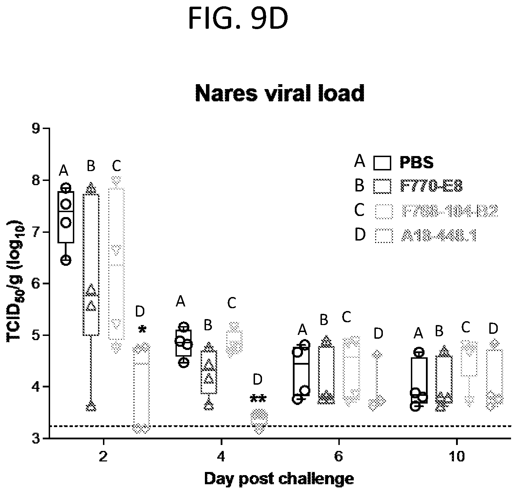

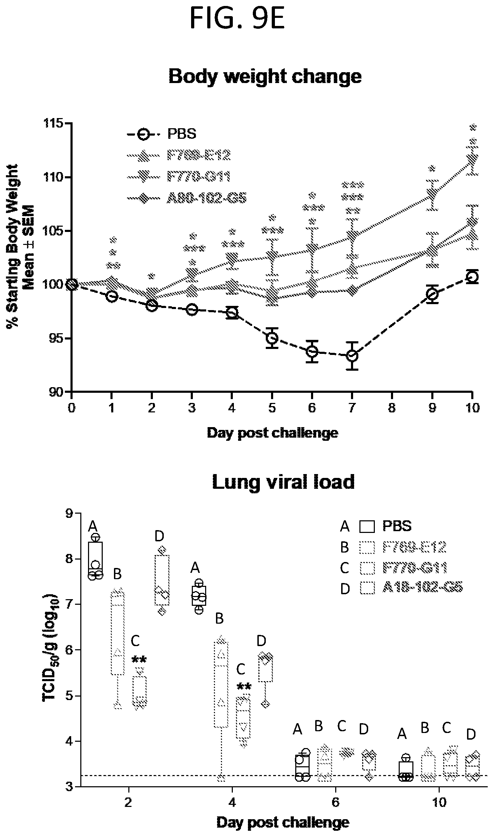

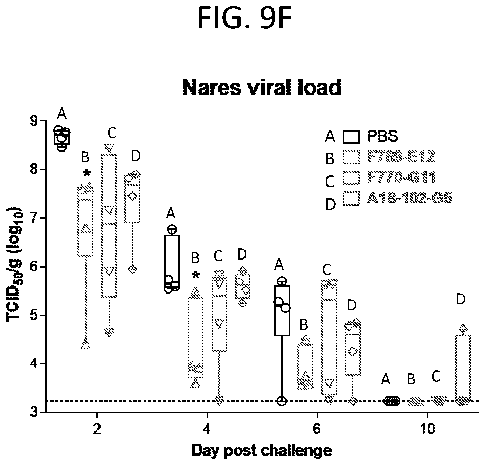

**FIGS. 9A-9F****. Antibodies targeting class I-IV epitopes protect Syrian hamsters from SARS-CoV-2 variants.** FIG. 9A. mAb passive transfer and virus challenge schema. mAbs targeting diverse epitopes were passively transferred to Syrian hamsters intraperitoneally 24 hours prior to challenge with1x10⁵ PFU of SARS-CoV-2 EG.5.1. An additional group of hamsters received an equal volume of PBS in the same manner and served as controls. All animals were weighed daily to monitor weight loss (top panel of FIG. 9C and FIG. 9E) and 4 animals per group were euthanized at days 2, 4, 6, and 10 to assess viral loads in the lung (bottom panel of FIG. 9C and FIG. 9E) and nares (FIG. 9D and FIG. 9F). Hamsters were monitored daily for weight loss over 10 days. FIG. 9B. In vitro neutralization potency predicts protection. Post-challenge (day 4) lung viral loads (TCID50/g) were highly correlated with antibody IC80 (ng/ml) against challenge virus. Each dot indicates one antibody, or the PBS (saline) control. Dotted lines indicate limits of quantification. FIGs. 9C-9F. Class I-IV mAbs protect hamsters from EG.5.1 challenge. Body weight was monitored daily (top panel of FIG. 9C and FIG. 9E) and average of data from 4-16 animals were shown. Symbols and error bars represent means and SEM, respectively. Viral load in the lung (bottom panel of FIG. 9C and FIG. 9E) or nares (FIG. 9D and FIG. 9F) of EG.5.1 challenged hamsters were quantified by TCID50 per gram. Each symbol represents an individual animal and may overlap for equivalent values. Boxes and horizontal bars denote the IQR and medians, respectively; whisker end points are equal to the maximum and minimum values. Dotted lines indicate the assay's lower limit of detection. One-way ANOVA were used to determine significance at each timepoint between treated and untreated animals. Significant p-values are indicated on graphs. Significant p-values are indicated as: * (≤0.05), **(≤0.01), ***(≤0.001).

### DETAILED DESCRIPTION

The International Committee on the Taxonomy of Viruses (ICTV, 2019) describes the *Coronaviridae* subfamily *Orthocoronavirinae* with several viruses that are pathogenic to humans. The most common human coronaviruses cause the common cold and include the alpha-coronaviruses 229E and NL63, and the beta-coronaviruses OC43 and HKU1. In addition to the coronaviruses that cause common cold symptoms, three beta-coronaviruses have been shown to be highly pathogenic in humans. These viruses, Middle East Respiratory syndrome cornonavirus (MERS), Severe acute respiratory syndrome coronavirus (SARS-CoV, a.k.a. SARS1) can produce severe symptoms that can lead to death in human patients.

At the end of 2019, a novel coronavirus was identified as the cause of a severe respiratory distress syndrome outbreak. This virus was later sequenced and identified to be highly similar to SARS-CoV and based on this result, the novel Coronavirus was renamed SARS CoV-2. The incubation period is typically between 4 to 14 days but can be as short as 1 day. Infection is characterized by fever, fatigue, cough, difficulty breathing and diarrhea. A subset of patients have significant respiratory distress, requiring hospitalization and oxygen supplementation. These patients can rapidly deteriorate and require intensive care unit admission and intubation. Severe disease is also characterized by abnormalities in multi-organ failure, blood clots and an apparent systemic inflammatory response syndrome. The elderly are at particularly high risk of severe disease.

The genome of coronavirus is a large, enveloped, positive-sense, single-stranded RNA whose genome length varies by species and encodes multiple structural and non-structural proteins, encoded in several reading frames. The spike protein (S) is expressed on the surface of the viral particle and is responsible for virus entry and infection of target cells. Transmission of Coronaviruses can occur through multiple methods, including respiratory droplets, aerosols, fecal-oral and fomite routes.

In survivors of COVID-19 and vaccine recipients, both humoral and cellular immunity are detected. While their relative contribution to protection is unknown, humoral responses including binding and neutralization correlate with protection. Humoral immunity is measured using assays for serum immunoglobulin binding to viral antigens, serum neutralization of virus particles, and binding of memory B cells to viral spike-based fluorescent probes.

At the end of 2021, the first member of the Omicron lineage, BA.1, was identified and noted to have a high level of resistance to monoclonal antibodies being used clinically. Since then many Omicron lineage members have been identified, including variants BA.1, BA.2, BA.4/BA.5, BQ.1.1, XBB.1.5, EG.5.1, and FL.1.5.1. As of late-2024 nearly all circulating strains are descendants of omicron JN.1, including KP.3.1.1 and XEC, and are resistant to all monoclonals previously available under EUA except for Pemgarda. Thus, there is a need for new monoclonals with activity against these variants, as well as sarbecoviruses that could potentially cause future outbreaks.

### I. Summary of Terms

Unless otherwise noted, technical terms are used according to conventional usage. Definitions of many common terms in molecular biology may be found in Krebs et al. (eds.), Lewin's genes XII, published by Jones & Bartlett Learning, 2017. As used herein, the singular forms "a," "an," and "the," refer to both the singular as well as plural, unless the context clearly indicates otherwise. For example, the term "an antigen" includes singular or plural antigens and can be considered equivalent to the phrase "at least one antigen." As used herein, the term "comprises" means "includes." It is further to be understood that any and all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described herein. In case of conflict, the present specification, including explanations of terms, will control. In addition, the materials, methods, and examples are illustrative only and not intended to be limiting. To facilitate review of the various aspects, the following explanations of terms are provided:
**About:** Unless context indicated otherwise, "about" refers to plus or minus 5% of a reference value. For example, "about" 100 refers to 95 to 105.

**Administration:** The introduction of an agent, such as a disclosed antibody, into a subject by a chosen route. Administration can be local or systemic. For example, if the chosen route is intravascular, the agent (such as antibody) is administered by introducing the composition into a blood vessel of the subject. Exemplary routes of administration include, but are not limited to, oral, injection (such as subcutaneous, intramuscular, intradermal, intraperitoneal, and intravenous), sublingual, rectal, transdermal (for example, topical), intranasal, vaginal, and inhalation routes.

**Amino acid substitution:** The replacement of one amino acid in a polypeptide with a different amino acid.

**Antibody and Antigen Binding Fragment:** An immunoglobulin, antigen-binding fragment, or derivative thereof, that specifically binds and recognizes an analyte (antigen) such as a coronavirus spike protein, such as a spike protein from SARS-CoV-2. The term "antibody" is used herein in the broadest sense and encompasses various antibody structures, including but not limited to monoclonal antibodies, polyclonal antibodies, multispecific antibodies (*e.g.,* bispecific antibodies), and antigen binding fragments, so long as they exhibit the desired antigen-binding activity.

Non-limiting examples of antibodies include, for example, intact immunoglobulins and variants and fragments thereof that retain binding affinity for the antigen. Examples of antigen binding fragments include but are not limited to Fv, Fab, Fab', Fab'-SH, F(ab')₂; diabodies; linear antibodies; single-chain antibody molecules (*e.g.* scFv); and multispecific antibodies formed from antibody fragments. Antibody fragments include antigen binding fragments either produced by the modification of whole antibodies or those synthesized *de novo* using recombinant DNA methodologies (see, *e.g.,* Kontermann and Dübel (Eds.), Antibody Engineering, Vols. 1-2, 2nd ed., Springer-Verlag, 2010).

Antibodies also include genetically engineered forms such as chimeric antibodies (such as humanized murine antibodies) and heteroconjugate antibodies (such as bispecific antibodies).

An antibody may have one or more binding sites. If there is more than one binding site, the binding sites may be identical to one another or may be different. For instance, a naturally-occurring immunoglobulin has two identical binding sites, a single-chain antibody or Fab fragment has one binding site, while a bispecific or bifunctional antibody has two different binding sites.

Typically, a naturally occurring immunoglobulin has heavy (H) chains and light (L) chains interconnected by disulfide bonds. Immunoglobulin genes include the kappa, lambda, alpha, gamma, delta, epsilon and mu constant region genes, as well as the myriad immunoglobulin variable domain genes. There are two types of light chain, lambda (λ) and kappa (x). There are five main heavy chain classes (or isotypes) which determine the functional activity of an antibody molecule: IgM, IgD, IgG, IgA and IgE.

Each heavy and light chain contains a constant region (or constant domain) and a variable region (or variable domain). In combination, the heavy and the light chain variable regions specifically bind the antigen.

References to "V_{H}" or "VH" refer to the variable region of an antibody heavy chain, including that of an antigen binding fragment, such as Fv, scFv, dsFv or Fab. References to "V_{L}" or "VL" refer to the variable domain of an antibody light chain, including that of an Fv, scFv, dsFv or Fab.

The V_{H} and V_{L} contain a "framework" region interrupted by three hypervariable regions, also called "complementarity-determining regions" or "CDRs" (see, *e.g.,* Kabat et al., Sequences of Proteins of Immunological Interest, 5th ed., NIH Publication No. 91-3242, Public Health Service, National Institutes of Health, U.S. Department of Health and Human Services, 1991). The sequences of the framework regions of different light or heavy chains are relatively conserved within a species. The framework region of an antibody, that is the combined framework regions of the constituent light and heavy chains, serves to position and align the CDRs in three-dimensional space.

The CDRs are primarily responsible for binding to an epitope of an antigen. The amino acid sequence boundaries of a given CDR can be readily determined using any of a number of well-known schemes, including those described by Kabat et al. (Sequences of Proteins of Immunological Interest, 5th ed., NIH Publication No. 91-3242, Public Health Service, National Institutes of Health, U.S. Department of Health and Human Services, 1991; "Kabat" numbering scheme), Al-Lazikani et al., ("Standard conformations for the canonical structures of immunoglobulins," J. Mol. Bio., 273(4):927-948, 1997; "Chothia" numbering scheme), and Lefranc et al. ("IMGT unique numbering for immunoglobulin and T cell receptor variable domains and Ig superfamily V-like domains," Dev. Comp. Immunol., 27(1):55-77, 2003; "IMGT" numbering scheme). The CDRs of each chain are typically referred to as CDR1, CDR2, and CDR3 (from the N-terminus to C-terminus), and are also typically identified by the chain in which the particular CDR is located. Thus, a V_{H} CDR3 is the CDR3 from the V_{H} of the antibody in which it is found, whereas a V_{L} CDR1 is the CDR1 from the V_{L} of the antibody in which it is found. Light chain CDRs are sometimes referred to as LCDR1, LCDR2, and LCDR3. Heavy chain CDRs are sometimes referred to as HCDR1, HCDR2, and HCDR3.

In some aspects, a disclosed antibody includes a heterologous constant domain. For example, the antibody includes a constant domain that is different from a native constant domain, such as a constant domain including one or more modifications (such as the "LS" mutation) to increase half-life.

A "monoclonal antibody" is an antibody obtained from a population of substantially homogeneous antibodies, that is, the individual antibodies comprising the population are identical and/or bind the same epitope, except for possible variant antibodies, for example, containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein. In some examples monoclonal antibodies are isolated from a subject. Monoclonal antibodies can have conservative amino acid substitutions which have substantially no effect on antigen binding or other immunoglobulin functions. (See, for example, Greenfield (Ed.), Antibodies: A Laboratory Manual, 2nd ed. New York: Cold Spring Harbor Laboratory Press, 2014.)

A "humanized" antibody or antigen binding fragment includes a human framework region and one or more CDRs from a non-human (such as a mouse, rat, or synthetic) antibody or antigen binding fragment. The non-human antibody or antigen binding fragment providing the CDRs is termed a "donor," and the human antibody or antigen binding fragment providing the framework is termed an "acceptor." In one aspect, all the CDRs are from the donor immunoglobulin in a humanized immunoglobulin. Constant regions need not be present, but if they are, they can be substantially identical to human immunoglobulin constant regions, such as at least about 85-90%, such as about 95% or more identical. Hence, all parts of a humanized antibody or antigen binding fragment, except possibly the CDRs, are substantially identical to corresponding parts of natural human antibody sequences.

A "chimeric antibody" is an antibody which includes sequences derived from two different antibodies, which typically are of different species. In some examples, a chimeric antibody includes one or more CDRs and/or framework regions from one human antibody and CDRs and/or framework regions from another human antibody.

A "fully human antibody" or "human antibody" is an antibody which includes sequences from (or derived from) the human genome, and does not include sequence from another species. In some aspects, a human antibody includes CDRs, framework regions, and (if present) an Fc region from (or derived from) the human genome. Human antibodies can be identified and isolated using technologies for creating antibodies based on sequences derived from the human genome, for example by phage display or using transgenic animals (see, *e.g.,* Barbas et al. Phage display: A Laboratory Manuel. 1st Ed. New York: Cold Spring Harbor Laboratory Press, 2004. Print.; Lonberg, Nat. Biotech., 23: 1117-1125, 2005; Lonenberg, Curr. Opin. Immunol., 20:450-459, 2008).

**Antibody or antigen binding fragment that neutralizes SARS-CoV-2:** An antibody or antigen binding fragment that specifically binds to a SARS-CoV-2 antigen (such as the spike protein) in such a way as to inhibit a biological function associated with SARS-CoV-2 that inhibits infection. The antibody can neutralize the activity of SARS-CoV-2. The SARS-CoV-2 can be Omicron or a variant thereof. In some aspects, the SARS-CoV-2 is BA.1, BA.2, BA.4/BA.5, BQ.1.1, XBB.1.5, EG.5.1, and FL.1.5.1, JN.1, KP.3.1.1, XEC, or related strains. For example, an antibody or antigen binding fragment that neutralizes SARS-CoV-2 may interfere with the virus by binding it directly and limiting entry into cells. Alternately, an antibody may interfere with one or more post-attachment interactions of the pathogen with a receptor, for example, by interfering with viral entry using the receptor. In some examples, an antibody that is specific for a coronavirus spike protein neutralizes the infectious titer of SARS-CoV-2.

In some aspects, an antibody or antigen binding fragment that specifically binds to SARS-CoV-2 and neutralizes SARS-CoV-2 inhibits infection of cells, for example, by at least 50% compared to a control antibody or antigen binding fragment.

A "broadly neutralizing antibody" is an antibody that binds to and inhibits the function of related antigens, such as antigens that share at least 85%, 90%, 95%, 96%, 97%, 98% or 99% identity antigenic surface of antigen. With regard to an antigen from a pathogen, such as a virus, the antibody can bind to and inhibit the function of an antigen from more than one class and/or subclass of the pathogen. For example, with regard to a coronavirus, the antibody can bind to and inhibit the function of an antigen, such as the spike protein from multiple SARS-CoV-2 Omicron variants.

**Biological sample:** A sample obtained from a subject. Biological samples include all clinical samples useful for detection of disease or infection in subjects, including, but not limited to, cells, tissues, and bodily fluids, such as blood, derivatives and fractions of blood (such as serum), cerebrospinal fluid; as well as biopsied or surgically removed tissue, for example tissues that are unfixed, frozen, or fixed in formalin or paraffin. In a particular example, a biological sample is obtained from a subject having or suspected of having a coronavirus infection, such as, but not limited to, a SARS-CoV-2 infection.

**Bispecific antibody:** A recombinant molecule composed of two different antigen binding domains that consequently binds to two different antigenic epitopes. Bispecific antibodies include chemically or genetically linked molecules of two antigen-binding domains. The antigen binding domains can be linked using a linker. The antigen binding domains can be monoclonal antibodies, antigen-binding fragments (*e.g.,* Fab, scFv), or combinations thereof. A bispecific antibody can include one or more constant domains, but does not necessarily include a constant domain.

**Conditions sufficient to form an immune complex:** Conditions which allow an antibody or antigen binding fragment to bind to its cognate epitope to a detectably greater degree than, and/or to the substantial exclusion of, binding to substantially all other epitopes. Conditions sufficient to form an immune complex are dependent upon the format of the binding reaction and typically are those utilized in immunoassay protocols or those conditions encountered *in vivo.* See Greenfield (Ed.), Antibodies: A Laboratory Manual, 2nd ed. New York: Cold Spring Harbor Laboratory Press, 2014, for a description of immunoassay formats and conditions. The conditions employed in the methods are "physiological conditions" which include reference to conditions (e.g., temperature, osmolarity, pH) that are typical inside a living mammal or a mammalian cell. While it is recognized that some organs are subject to extreme conditions, the intra-organismal and intracellular environment normally lies around pH 7 (*e.g.,* from pH 6.0 to pH 8.0, more typically pH 6.5 to 7.5), contains water as the predominant solvent, and exists at a temperature above 0°C and below 50°C. Osmolarity is within the range that is supportive of cell viability and proliferation.

The formation of an immune complex can be detected through conventional methods, for instance immunohistochemistry (IHC), immunoprecipitation (IP), flow cytometry, immunofluorescence microscopy, ELISA, immunoblotting (for example, Western blot), magnetic resonance imaging (MRI), computed tomography (CT) scans, radiography, and affinity chromatography.

**Conjugate:** A complex of two molecules linked together, for example, linked together by a covalent bond. In one aspect, an antibody is linked to an effector molecule; for example, an antibody that specifically binds to SARS-CoV-2, covalently linked to an effector molecule, such as a detectable label. The linkage can be by chemical or recombinant means. In one aspect, the linkage is chemical, wherein a reaction between the antibody moiety and the effector molecule has produced a covalent bond formed between the two molecules to form one molecule. A peptide linker (short peptide sequence) can optionally be included between the antibody and the effector molecule. Because conjugates can be prepared from two molecules with separate functionalities, such as an antibody and an effector molecule, they are also sometimes referred to as "chimeric molecules."

**Conservative variants:** "Conservative" amino acid substitutions are those substitutions that do not substantially affect or decrease a function of a protein, such as the ability of the protein to interact with a target protein. For example, a coronavirus-specific antibody can include up to 1, 2, 3, 4, 5, 6, 7, 8, 9, or up to 10 conservative substitutions compared to a reference antibody sequence and retain specific binding activity for spike protein binding, and/or neutralization activity. The term conservative variation also includes the use of a substituted amino acid in place of an unsubstituted parent amino acid.

Individual substitutions, deletions or additions which alter, add or delete a single amino acid or a small percentage of amino acids (for instance less than 5%, in some aspects less than 1%) in an encoded sequence are conservative variations where the alterations result in the substitution of an amino acid with a chemically similar amino acid.

The following six groups are examples of amino acids that are considered to be conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Non-conservative substitutions are those that reduce an activity or function of the antibody, such as the ability to specifically bind to a coronavirus spike protein. For instance, if an amino acid residue is essential for a function of the protein, even an otherwise conservative substitution may disrupt that activity. Thus, a conservative substitution does not alter the basic function of a protein of interest.

Contacting: Placement in direct physical association; includes both in solid and liquid form, which can take place either *in vivo* or *in vitro.* Contacting includes contact between one molecule and another molecule, for example the amino acid on the surface of one polypeptide, such as an antigen, that contacts another polypeptide, such as an antibody. Contacting can also include contacting a cell for example by placing an antibody in direct physical association with a cell.

**Control:** A reference standard. In some aspects, the control is a negative control, such as sample obtained from a healthy patient not infected with a coronavirus, such as SARS-CoV-2, for example BA.1, BA.2, BA.4/BA.5, BA.2.75, BA.4.6, BA.2.75.2, BQ.1.1, BJ.1, XBB.1, XBB.1.5, XBB.1.16, EG.5.1, FL.1.5.1, BA.2.86, JN.1, KP.2, KP.3, LB.1, KP.2.3, KP.3.1.1 or XEC. In other aspects, the control is a positive control, such as a tissue sample obtained from a patient diagnosed with a coronavirus infection, such as a SARS-CoV-2 infection. In still other aspects, the control is a historical control or standard reference value or range of values (such as a previously tested control sample, such as a group of patients with known prognosis or outcome, or group of samples that represent baseline or normal values).

A difference between a test sample and a control can be an increase or conversely a decrease. The difference can be a qualitative difference or a quantitative difference, for example a statistically significant difference. In some examples, a difference is an increase or decrease, relative to a control, of at least about 5%, such as at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, or at least about 500%.

**Coronavirus:** A family of positive-sense, single-stranded RNA viruses that are known to cause severe respiratory illness. Viruses currently known to infect human from the coronavirus family are from the alphacoronavirus and betacoronavirus genera. Additionally, it is believed that the gammacoronavirus and deltacoronavirus genera may infect humans in the future.

Non-limiting examples of betacoronaviruses include SARS-CoV-2, Middle East respiratory syndrome coronavirus (MERS-CoV), Severe Acute Respiratory Syndrome coronavirus (SARS-CoV), Human coronavirus HKU1 (HKU1-CoV), Human coronavirus OC43 (OC43-CoV), Murine Hepatitis Virus (MHV-CoV), Bat SARS-like coronavirus WIV1 (WIV1-CoV), and Human coronavirus HKU9 (HKU9-CoV). Non-limiting examples of alphacoronaviruses include human coronavirus 229E (229E-CoV), human coronavirus NL63 (NL63-CoV), porcine epidemic diarrhea virus (PEDV), and Transmissible gastroenteritis coronavirus (TGEV). A non-limiting example of a deltacoronaviruses is the Swine Delta Coronavirus (SDCV).

The viral genome is capped, polyadenylated, and covered with nucleocapsid proteins. The coronavirus virion includes a viral envelope containing type I fusion glycoproteins referred to as the spike (S) protein. Most coronaviruses have a common genome organization with the replicase gene.

**Degenerate variant:** In the context of the present disclosure, a "degenerate variant" refers to a polynucleotide encoding a polypeptide (such as an antibody heavy or light chain) that includes a sequence that is degenerate as a result of the genetic code. There are 20 natural amino acids, most of which are specified by more than one codon. Therefore, all degenerate nucleotide sequences encoding a peptide are included as long as the amino acid sequence of the peptide encoded by the nucleotide sequence is unchanged.

**Detectable marker:** A detectable molecule (also known as a label) that is conjugated directly or indirectly to a second molecule, such as an antibody, to facilitate detection of the second molecule. For example, the detectable marker can be capable of detection by ELISA, spectrophotometry, flow cytometry, microscopy or diagnostic imaging techniques (such as CT scans, MRIs, ultrasound, fiberoptic examination, and laparoscopic examination). Specific, non-limiting examples of detectable markers include fluorophores, chemiluminescent agents, enzymatic linkages, radioactive isotopes and heavy metals or compounds (for example super paramagnetic iron oxide nanocrystals for detection by MRI). Methods for using detectable markers and guidance in the choice of detectable markers appropriate for various purposes are discussed for example in Green and Sambrook (Molecular Cloning: A Laboratory Manual, 4th ed., New York: Cold Spring Harbor Laboratory Press, 2012) and Ausubel et al. (Eds.) (Current Protocols in Molecular Biology, New York: John Wiley and Sons, including supplements, 2017).

**Detecting:** To identify the existence, presence, or fact of something.

**Effective amount:** A quantity of a specific substance sufficient to achieve a desired effect in a subject to whom the substance is administered. For instance, this can be the amount necessary to inhibit a coronavirus infection, such as a SARS-CoV-2 infection, or to measurably alter outward symptoms of such an infection. The SARS-CoV-2 can be BA.1, BA.2, BA.4/BA.5, BA.2.75, BA.4.6, BA.2.75.2, BQ.1.1, BJ.1, XBB.1, XBB.1.5, XBB.1.16, EG.5.1, FL.1.5.1, BA.2.86, JN.1, KP.2, KP.3, LB.1, KP.2.3, KP.3.1.1 or XEC.

In one example, a desired response is to inhibit or reduce or prevent SARS-CoV-2 infection. The SARS-CoV-2 infection does not need to be completely eliminated or reduced or prevented for the method to be effective. For example, administration of an effective amount of can decrease the SARS-CoV-2 infection (for example, as measured by infection of cells, or by number or percentage of subjects infected by the SARS-CoV-2) by a desired amount, for example by at least 50%, at least 60%, at least 70%, at least 80%, at least 90%, at least 95%, at least 98%, or even at least 100% (elimination or prevention of detectable SARS-CoV-2 infection), as compared to a suitable control. Additional coronavirus infections can also be inhibited as a result of using the presently disclosed antibodies.

In some aspects, administration of an effective amount of a disclosed antibody or antigen binding fragment that binds to a SARS-CoV-2 spike protein can reduce or inhibit infection (for example, as measured by infection of cells, or by number or percentage of subjects infected by the SARS-CoV-2 or by an increase in the survival time of infected subjects, or reduction in symptoms associated with the infection) by a desired amount, for example by at least 10%, at least 20%, at least 50%, at least 60%, at least 70%, at least 80%, at least 90%, at least 95%, at least 98%, or even at least 100% (elimination or prevention of detectable infection), as compared to a suitable control.

The effective amount of an antibody or antigen binding fragment that specifically binds the SARS-CoV-2 spike protein that is administered to a subject to inhibit infection will vary depending upon a number of factors associated with that subject, for example the overall health and/or weight of the subject. An effective amount can be determined by varying the dosage and measuring the resulting response, such as, for example, a reduction in pathogen titer. Effective amounts also can be determined through various *in vitro, in vivo* or *in situ* immunoassays.

An effective amount encompasses a fractional dose that contributes in combination with previous or subsequent administrations to attaining an effective response. For example, an effective amount of an agent can be administered in a single dose, or in several doses, for example daily, during a course of treatment lasting several days or weeks. However, the effective amount can depend on the subject being treated, the severity and type of the condition being treated, and the manner of administration. A unit dosage form of the agent can be packaged in an amount, or in multiples of the effective amount, for example, in a vial *(e.g.,* with a pierceable lid) or syringe having sterile components.

**Effector molecule:** A molecule intended to have or produce a desired effect; for example, a desired effect on a cell to which the effector molecule is targeted, or a detectable marker. Effector molecules can include, for example, polypeptides and small molecules. Some effector molecules may have or produce more than one desired effect.

**Epitope:** An antigenic determinant. These are particular chemical groups or peptide sequences on a molecule that are antigenic, such that they elicit a specific immune response, for example, an epitope is the region of an antigen to which B and/or T cells respond. An antibody can bind to a particular antigenic epitope, such as an epitope on a SARS-CoV-2 spike protein.

**Expression:** Transcription or translation of a nucleic acid sequence. For example, an encoding nucleic acid sequence (such as a gene) can be expressed when its DNA is transcribed into RNA or an RNA fragment, which in some examples is processed to become mRNA. An encoding nucleic acid sequence (such as a gene) may also be expressed when its mRNA is translated into an amino acid sequence, such as a protein or a protein fragment. In a particular example, a heterologous gene is expressed when it is transcribed into an RNA. In another example, a heterologous gene is expressed when its RNA is translated into an amino acid sequence. Regulation of expression can include controls on transcription, translation, RNA transport and processing, degradation of intermediary molecules such as mRNA, or through activation, inactivation, compartmentalization or degradation of specific protein molecules after they are produced.

**Expression Control Sequences:** Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus, expression control sequences can include appropriate promoters, enhancers, transcriptional terminators, a start codon (ATG) in front of a protein-encoding gene, splice signals for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

**Expression vector:** A vector comprising a recombinant polynucleotide comprising expression control sequences operatively linked to a nucleotide sequence to be expressed. An expression vector comprises sufficient cis- acting elements for expression; other elements for expression can be supplied by the host cell or in an in vitro expression system. Non-limiting examples of expression vectors include cosmids, plasmids (*e.g.,* naked or contained in liposomes) and viruses (*e.g.,* lentiviruses, retroviruses, adenoviruses, and adeno-associated viruses) that incorporate the recombinant polynucleotide.

A polynucleotide can be inserted into an expression vector that contains a promoter sequence which facilitates the efficient transcription of the inserted genetic sequence of the host. The expression vector typically contains an origin of replication, a promoter, as well as specific nucleic acid sequences that allow phenotypic selection of the transformed cells.

**Fc region:** The constant region of an antibody excluding the first heavy chain constant domain. Fc region generally refers to the last two heavy chain constant domains of IgA, IgD, and IgG, and the last three heavy chain constant domains of IgE and IgM. An Fc region may also include part or all of the flexible hinge N-terminal to these domains. For IgA and IgM, an Fc region may or may not include the tailpiece, and may or may not be bound by the J chain. For IgG, the Fc region is typically understood to include immunoglobulin domains Cy2 and Cy3 and optionally the lower part of the hinge between Cγ1 and Cy2. Although the boundaries of the Fc region may vary, the human IgG heavy chain Fc region is usually defined to include residues following C226 or P230 to the Fc carboxyl-terminus, wherein the numbering is according to EU numbering. For IgA, the Fc region includes immunoglobulin domains Cα2 and Cα3 and optionally the lower part of the hinge between Cα1 and Cα2.

**Heterologous:** Originating from a different genetic source. A nucleic acid molecule that is heterologous to a cell originated from a genetic source other than the cell in which it is expressed. In one specific, non-limiting example, a heterologous nucleic acid molecule encoding a protein, such as an scFv, is expressed in a cell, such as a mammalian cell. Methods for introducing a heterologous nucleic acid molecule in a cell or organism are well known in the art, for example transformation with a nucleic acid, including electroporation, lipofection, particle gun acceleration, and homologous recombination.

**Host cell:** Cells in which a vector can be propagated and its DNA expressed. The cell may be prokaryotic or eukaryotic. The term also includes any progeny of the subject host cell. It is understood that all progeny may not be identical to the parental cell since there may be mutations that occur during replication. However, such progeny are included when the term "host cell" is used.

**IgA:** A polypeptide belonging to the class of antibodies that are substantially encoded by a recognized immunoglobulin alpha gene. In humans, this class or isotype comprises IgA₁ and IgA₂. IgA antibodies can exist as monomers, polymers (referred to as pIgA) of predominantly dimeric form, and secretory IgA. The constant chain of wild-type IgA contains an 18-amino-acid extension at its C-terminus called the tail piece (tp). Polymeric IgA is secreted by plasma cells with a 15-kDa peptide called the J chain linking two monomers of IgA through the conserved cysteine residue in the tail piece.

**IgG:** A polypeptide belonging to the class or isotype of antibodies that are substantially encoded by a recognized immunoglobulin gamma gene. In humans, this class comprises IgG₁, IgG₂, IgG₃, and IgG₄.

**Immune complex:** The binding of antibody or antigen binding fragment (such as a scFv) to a soluble antigen forms an immune complex. The formation of an immune complex can be detected through conventional methods, for instance immunohistochemistry, immunoprecipitation, flow cytometry, immunofluorescence microscopy, ELISA, immunoblotting (for example, Western blot), magnetic resonance imaging, CT scans, radiography, and affinity chromatography.

**Inhibiting or treating a disease:** Inhibiting the full development of a disease or condition, for example, in a subject who is at risk for a disease such as a SARS-CoV-2 infection. "Treatment" refers to a therapeutic intervention that ameliorates a sign or symptom of a disease or pathological condition after it has begun to develop. The term "ameliorating," with reference to a disease or pathological condition, refers to any observable beneficial effect of the treatment. Inhibiting a disease can include preventing or reducing the risk of the disease, such as preventing or reducing the risk of viral infection. The beneficial effect can be evidenced, for example, by a delayed onset of clinical symptoms of the disease in a susceptible subject, a reduction in severity of some or all clinical symptoms of the disease, a slower progression of the disease, a reduction in the viral load, an improvement in the overall health or well-being of the subject, or by other parameters that are specific to the particular disease. A "prophylactic" treatment is a treatment administered to a subject who does not exhibit signs of a disease or exhibits only early signs for the purpose of decreasing the risk of developing pathology.

The term "reduces" is a relative term, such that an agent reduces a disease or condition if the disease or condition is quantitatively diminished following administration of the agent, or if it is diminished following administration of the agent, as compared to a reference agent. Similarly, the term "prevents" does not necessarily mean that an agent completely eliminates the disease or condition, so long as at least one characteristic of the disease or condition is eliminated. Thus, a composition that reduces or prevents an infection, can, but does not necessarily completely, eliminate such an infection, so long as the infection is measurably diminished, for example, by at least about 50%, such as by at least about 70%, or about 80%, or even by about 90% the infection in the absence of the agent, or in comparison to a reference agent.

**Isolated:** A biological component (such as a nucleic acid, peptide, protein or protein complex, for example an antibody) that has been substantially separated, produced apart from, or purified away from other biological components in the cell of the organism in which the component naturally occurs, that is, other chromosomal and extra-chromosomal DNA and RNA, and proteins. Thus, isolated nucleic acids, peptides and proteins include nucleic acids and proteins purified by standard purification methods. The term also embraces nucleic acids, peptides and proteins prepared by recombinant expression in a host cell, as well as, chemically synthesized nucleic acids. An isolated nucleic acid, peptide or protein, for example an antibody, can be at least 50%, at least 60%, at least 70%, at least 80%, at least 90%, at least 95%, at least 96%, at least 97%, at least 98%, or at least 99% pure.

**Kabat position:** A position of a residue in an amino acid sequence that follows the numbering convention delineated by Kabat et al. (Sequences of Proteins of Immunological Interest, 5th Edition, Department of Health and Human Services, Public Health Service, National Institutes of Health, Bethesda, NIH Publication No. 91-3242, 1991).

**Linker:** A bi-functional molecule that can be used to link two molecules into one contiguous molecule, for example, to link a detectable marker to an antibody. Non-limiting examples of peptide linkers include glycine-serine linkers.

The terms "conjugating," "joining," "bonding," or "linking" can refer to making two molecules into one contiguous molecule; for example, linking two polypeptides into one contiguous polypeptide, or covalently attaching an effector molecule or detectable marker radionuclide or other molecule to a polypeptide, such as an scFv. The linkage can be either by chemical or recombinant means. "Chemical means" refers to a reaction between the antibody moiety and the effector molecule such that there is a covalent bond formed between the two molecules to form one molecule.

**Multispecific antibody:** A recombinant molecule containing of two or more different variable fragments (Fv). The valency of a multispecific antibody is equal to the number of Fv fragments in the molecule. The specificity of a multispecific antibody is equal to the number of unique antigen-binding domains in the multispecific. In multispecific antibodies, specificity is limited by the number of total Fv domains available. Monospecific antibodies can be made using multispecific antibodies with a valency of 3, 4, 5 or more and in which all of the Fvs are identical. Bispecific antibodies can be made using multispecific antibodies with valency of 2, 3, 4 or more, where the Fv domains are made from two different antigen binding domains. In this instance the numbers of each antigen binding domain can be equal or different and can vary in position and location. Similarly, trispecific antibodies can be made with valency of 3, 4, 5 or more, where the Fv domains are made from three different antigen binding domains. In this instance the numbers of each antigen binding domain can be equal or different and can vary in position and location. Similarly, tetraspecific antibodies can be made with valency of 4, 5, 6 or more, where the Fv domains are made from four different antigen binding domains. In this instance the numbers of each antigen binding domain can be equal or different and can vary in position and location. Multispecific antibodies include chemically or genetically linked molecules of antigen binding domains. The antigen binding domains can be linked using a linker. The antigen binding domains can be monoclonal antibodies, antigen-binding fragments (*e.g.*, Fab, scFv), or combinations thereof. A multiispecific antibody can include one or more constant domains, but does not necessarily include a constant domain.

**Nucleic acid (molecule or sequence):** A deoxyribonucleotide or ribonucleotide polymer or combination thereof including without limitation, cDNA, mRNA, genomic DNA, and synthetic (such as chemically synthesized) DNA or RNA. The nucleic acid can be double stranded (ds) or single stranded (ss). Where single stranded, the nucleic acid can be the sense strand or the antisense strand. Nucleic acids can include natural nucleotides (such as A, T/U, C, and G), and can include analogs of natural nucleotides, such as labeled nucleotides.

"cDNA" refers to a DNA that is complementary or identical to an mRNA, in either single stranded or double stranded form.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA produced by that gene produces the protein in a cell or other biological system. Both the coding strand, the nucleotide sequence of which is identical to the mRNA sequence and is usually provided in sequence listings, and non-coding strand, used as the template for transcription, of a gene or cDNA can be referred to as encoding the protein or other product of that gene or cDNA. Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns.

**Operably linked:** A first nucleic acid sequence is operably linked with a second nucleic acid sequence when the first nucleic acid sequence is placed in a functional relationship with the second nucleic acid sequence. For instance, a promoter, such as the CMV promoter, is operably linked to a coding sequence if the promoter affects the transcription or expression of the coding sequence. Generally, operably linked DNA sequences are contiguous and, where necessary to join two protein-coding regions, in the same reading frame.

**Pharmaceutically acceptable carriers:** The pharmaceutically acceptable carriers of use are conventional. Remington: The Science and Practice of Pharmacy, 22nd ed., London, UK: Pharmaceutical Press, 2013, describes compositions and formulations suitable for pharmaceutical delivery of the disclosed agents.

In general, the nature of the carrier will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. For solid compositions (*e.g*., powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, added preservatives (such as non-natural preservatives), and pH buffering agents and the like, for example sodium acetate or sorbitan monolaurate. In particular examples, the pharmaceutically acceptable carrier is sterile and suitable for parenteral administration to a subject for example, by injection. In some aspects, the active agent and pharmaceutically acceptable carrier are provided in a unit dosage form such as a pill or in a selected quantity in a vial. Unit dosage forms can include one dosage or multiple dosages (for example, in a vial from which metered dosages of the agents can selectively be dispensed).

**Polypeptide:** A polymer in which the monomers are amino acid residues that are joined together through amide bonds. When the amino acids are alpha-amino acids, either the L-optical isomer or the D-optical isomer can be used, the L-isomers being preferred. The terms "polypeptide" or "protein" as used herein are intended to encompass any amino acid sequence and include modified sequences such as glycoproteins. A polypeptide includes both naturally occurring proteins, as well as those that are recombinantly or synthetically produced. A polypeptide has an amino terminal (N-terminal) end and a carboxy-terminal end. In some aspects, the polypeptide is a disclosed antibody or a fragment thereof.

**Purified:** The term purified does not require absolute purity; rather, it is intended as a relative term. Thus, for example, a purified peptide preparation is one in which the peptide or protein (such as an antibody) is more enriched than the peptide or protein is in its natural environment within a cell. In one aspect, a preparation is purified such that the protein or peptide represents at least 50% of the total peptide or protein content of the preparation.

**Recombinant:** A recombinant nucleic acid is one that has a sequence that is not naturally occurring or has a sequence that is made by an artificial combination of two otherwise separated segments of sequence. This artificial combination can be accomplished by chemical synthesis or, more commonly, by the artificial manipulation of isolated segments of nucleic acids, for example, by genetic engineering techniques. A recombinant protein is one that has a sequence that is not naturally occurring or has a sequence that is made by an artificial combination of two otherwise separated segments of sequence. In several aspects, a recombinant protein is encoded by a heterologous (for example, recombinant) nucleic acid that has been introduced into a host cell, such as a bacterial or eukaryotic cell. The nucleic acid can be introduced, for example, on an expression vector having signals capable of expressing the protein encoded by the introduced nucleic acid or the nucleic acid can be integrated into the host cell chromosome.

**SARS-CoV-2:** Also known as 2019 novel coronavirus, SARS-CoV-2 is a positive-sense, single stranded RNA virus of the genus betacoronavirus that has emerged as a highly fatal cause of severe acute respiratory infection. The viral genome is capped, polyadenylated, and covered with nucleocapsid proteins. The SARS-CoV-2 virion includes a viral envelope with large spike glycoproteins. The SARS-CoV-2 genome, like most coronaviruses, has a common genome organization with the replicase gene included in the 5'-two thirds of the genome, and structural genes included in the 3'-third of the genome. The SARS-CoV-2 genome encodes the canonical set of structural protein genes in the order 5' - spike (S) - envelope (E) - membrane (M) and nucleocapsid (N) - 3'. Symptoms of SARS-CoV-2 infection include fever and respiratory illness, such as dry cough and shortness of breath. Cases of severe infection can progress to severe pneumonia, multi-organ failure, and death. The time from exposure to onset of symptoms is approximately 2 to 14 days.

Standard methods for detecting viral infection may be used to detect SARS-CoV-2 infection, including but not limited to, assessment of patient symptoms and background and genetic tests such as reverse transcription-polymerase chain reaction (rRT-PCR). The test can be done on patient samples such as respiratory or blood samples.

B.1.1.529, also known as the Omicron variant BA.1, is a variant of the original SARS-CoV-2 first reported to the World Health Organization on November 21, 2021. This variant has a total of 60 mutations compared to the original strain of SARS-CoV-2, specifically 50 nonsynonymous mutations, 8 synonymous mutations, and 2 non-coding mutations. Thirty-two mutations affect the spike protein (A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F), or which approximately half are located in the receptor binding domain (319-541).

Novel sub-variants with enhanced transmissibility rates, derived from either BA.2 or BA.4/BA.5, emerged and became prevalent in November 2022. Their geographical distribution is heterogeneous, but they carry an additional limited set of mutations in the spike.

BA.2.75.2, derived from BA.2, was first noted in India and Singapore and comprises R346T, F486S and D1199N substitutions 17-19. BA.4.6 was detected in various countries, and carries R346T and N658S mutations. As of November 2022, BQ.1.1 became the main circulating lineage in many countries. It also carries the R346T mutation found in BA.2.75.2, along with K444T and N460K substitutions (see Planas et al., bioRXiv, available on the internet, doi.org/10.1101/2022.11.17.516888, November 17, 2022). BQ.1.1 includes amino acid substitutions relative to WA-1 (note "-" indicates a deletion): T19I, L24-, P25-, P26-, A27S, H69-, V70-, G142D, V213G, G339D, R346T, S371F, S373P, S375F, T376A, D405N, R408S, K417N, N440K, K444T, L452R, N460K_S477N, T478K, E484A, F486V, Q498R, N501Y, Y505H, D614G, H655Y, N679K, P681H, N764K, D796Y, Q954H, and N969K. BQ1.1 is disclosed, for example, in Miller et al., /doi.org/10.1101/2022.11.01.514722, available November 2, 2022. The BJ.1 variant is believed to have evolved from an Omicron BA.2 background. BJ.1 includes the following amino acid substitutions relative to WA-1, wherein "-" indicates a deletion: T19I,L24-, P25-, P26-, A27S, V83A, G142D, Y144, H146Q, Q183E, V213E, G339H, R346T, L368I, S371F, S373P, S375F, T376A, D405N, R408S, K417N, N440K, V445P, G446S, S477N, T478K, V483A, E484A, F490V, Q493R, Q498R, N501Y, Y505H, D614G, H655Y, N679K, P681H, N764K, D796Y, G798D, Q954H, N969K and S1003I, see Roemer et al., SARS-CoV-2 post-Omicron, virological.org/t/sars-cov-2-evolution-post-omicron/911, November 25, 2022. XBB is a SARS-CoV-2 variant believe to have arisen from an inter-lineage recombination even between BJ.1 and BA.2.75. XBB includes the following amino acid substitutions relative to WA-1, wherein "-" indicates a deletion: T19I, L24-, P25-, P26-, A27S, V83A, G142D, Y144-, H146Q, Q183E, V213E, G339H, R346T, L368I, S371F, S373P, S375F, T376A, D405N, R408S, K417N, N440K, V445P, G446S, N460K, S477N, T478K, E484A, F486S, F490S, Q498R, N501Y, Y505H, D614G, H655Y, N679K, P681H, N764K, D796Y, Q954H, and N969K, see Roemer et al., SARS-CoV-2 post-Omicron, virological.org/t/sars-cov-2-evolution-post-omicron/911, November 25, 2022.

BA.2.12.1 includes the following amino acid substitutions relative to WA-1, wherein "-" indicates a deletion: T19I,L24S, P25-, P26-, A27-, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H and N969K. BA2.75 includes the following amino acid substitutions relative to WA-1, wherein "-" indicates a deletion: T19I, L24-, P25-, P26-, A27S, G142D, K147E, W152R, F157L, I210V, V213G, G257S, G339H, S371F, S373P, S375F, T376A, D405N, R408S, K417N, N440K, G446S, N460K, S477N, T478K, E484A, Q498R, N501Y, Y505H, H655Y, N679K, P681H, N764K, D796Y, Q954H and N969K. BA.4 and BA.5 spike include the following amino acid substitutions relative to WA-1, wherein "-" indicates a deletion: T19I, L24S, P25-, P26-, A27-, H69-, V70-, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K.

The BA.4 and BA.5 sub-lineages of B.1.1.529, which do not differ in their spike sequence from each other, were also first detected by genomic surveillance in South Africa. BA.4 and BA.5 have changes relative to B.1.1.529 including the L452R and F486V mutations and the R493Q reversion in the spike receptor binding domain (RBD). BA.4 and BA.5 also differ from the BA.2 sub-lineage by a deletion of spike residues 69 and 70 (Khan et al., Nature Comm. 13, Article number 4686, doi.org/10.1038/s41467-022-32396-9, (2022).

XBB.1.5 includes the following amino acid substitutions relative to WA-1, wherein "-" indicates a deletion: T19I, L24-, P25-, P26-, A27S, V83A, G142D, Y144-, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, N969K. XBB.1.16 includes the following amino acid substitutions relative to WA-1, wherein "-" indicates a deletion: T19I, L24-, P25-, P26-, A27S, V83A, G142D, Y144-, H146Q, E180V, Q183E, V213E, G252V, G339H, R346T, L368I, S371F, S373P, S375F, T376A, D405N, R408S, K417N, N440K, V445P, G446S, N460K, S477N, T478R, E484A, F486P, F490S, Q498R, N501Y, Y505H, D614G, H655Y, N679K, P681H, N764K, D796Y, Q954H, N969K. CH.1.1 includes the following amino acid substitutions relative to WA-1, wherein "-" indicates a deletion: T19I, L24-, P25-, P26-, A27S, G142D, K147E, W152R, F157L, I210V, V213G, G257S, G339H, R346T, S371F, S373P, S375F, T376A, D405N, R408S, K417N, N440K, K444T, G446S, L452R, N460K, S477N, T478K, E484A, F486S, Q498R, N501Y, Y505H, D614G, H655Y, N679K, P681H, N764K, D796Y, Q954H, N969K. XBC.1 includes the following amino acid substitutions relative to WA-1, wherein "-" indicates a deletion: T19I, P25S, G142D, Y144-, E156-, F157-, R158G, P209L, L212S, D215H, A222V, A243-, L244-, S256L, S371F, S373P, S375F, T376A, D405N, R408S, K417N, N440K, G446S, L452M, S477N, T478K, E484A, F486P, Q498R, N501Y, Y505H, D614G, H655Y, N679K, P681H, N703I, N764K, D796Y, Q954H, N969K. XBB.2.3.2 contains G184V, D253G, F486P, P521S amino acid substitutions on the XBB.1.5. EG.5.1 contains Q52H and F456L amino acid substitutions on the XBB.1.5. XBB.1.16.6 contains E180V, F456L and T478R amino acid substitutions on the XBB.1.5. FL.1.5.1 contains F456L, T478R and A701V amino acid substitutions on the XBB.1.5.

BA.2.86 has two versions, BA.2.86_V670 and BA.2.86_I670. BA.2.86_I670 is a predominant version, includes the following amino acid substitutions relative to WA-1, wherein "-" indicates a deletion: ins16MPLF, T19I, R21T, L24-, P25-, P26-, A27S, S50L, H69-, V70-, V127F, G142D, Y144-,F157S, R158G, N211-, L212I, V213G, L216F, H245N, A264D, I332V, G339H, K356T, S371F,S373P, S375F, T376A, R403K, D405N, R408S, K417N, N440K, V445H, G446S, N450D, L452W, N460K, S477N, T478K, N481K, V483-, E484K, F486P, Q498R, N501Y, Y505H, E554K, A570V, D614G, P621S, H655Y, N679K, P681R, N764K, D796Y, S939F, Q954H, N969K, P1143L. JN.1 contains L455S amino acid substitution on the BA.2.86. KP.2 contains R346T, F456L amino acid substitutions on the JN.1. KP.3 contains F456L, Q493E, V1104L amino acid substitutions on the JN.1. LB.1 contains S31 deletion and Q183H, R346T, F456L amino acid substitutions on the JN.1. KP.2.3 contains S31 deletion and H146Q, R346T, F456L, V1104L amino acid substitutions on the JN.1. KP.3.1.1 contains S31 deletion and F456L, Q493E, V1104L amino acid substitutions on the JN.1. XEC contains T22N, F59S, F456L, Q493E, V1104L amino acid substitutions on the JN.1. As of November 23, 2024, KP.3.1.1 and XEC are the predominant variants globally.

**Spike (S) protein (Coronavirus):** A class I fusion glycoprotein initially synthesized as a precursor protein of approximately 1256 amino acids in size for SARS-CoV, and 1273 for SARS-CoV-2. Individual precursor S polypeptides form a homotrimer and undergo glycosylation within the Golgi apparatus as well as processing to remove the signal peptide, and cleavage by a cellular protease between approximately position 679/680 for SARS-CoV, and 685/686 for SARS-CoV-2, to generate separate S1 and S2 polypeptide chains, which remain associated as S1/S2 protomers within the homotrimer and is therefore a trimer of heterodimers. The S1 subunit is distal to the virus membrane and contains the receptor-binding domain (RBD) that is believed to mediate virus attachment to its host receptor. The S2 subunit contains the fusion protein machinery, such as the fusion peptide, two heptad-repeat sequences (HR1 and HR2) and a central helix typical of fusion glycoproteins, a transmembrane domain, and the cytosolic tail domain.

The numbering used in the disclosed SARS-CoV-2 S proteins and fragments thereof is relative to the S protein of SARS-CoV-2, the sequence of which was deposited as NCBI Ref. No. YP_009724390.1, as available on February 1, 2022, which is incorporated by reference herein in its entirety.

The spike trimer (S) of SARS-CoV-2 is a trimer of dimers, which are S1 and S2. S2 mediates fusion of the virus and host cell membranes, while the S1 domain mediates attachment to target cells and the host-cell receptor protein, angiotensin converting enzyme (ACE)2. S1 consists of 2 major domains: the N-terminal domain (NTD) and the receptor binding domain (RBD). The receptor binding domain (RBD) of the SARS-CoV-2 Spike protein (S) contains a receptor binding motif (RBM) that binds human cellular receptor protein ACE2. RBM binding to ACE2 is required for SARS-CoV-2 to infect cells. The RBD exists in two conformations referred to as "up" or "down". When the RBD is "down", the RBM is not able to bind ACE2. However, when RBD is "up", the RBM is able to bind to ACE2. Barnes et al. defined a functional classification schema for antibodies targeting the RBD that is based upon the RBD state an antibody can bind and whether the epitope within RBD overlaps with the ACE2 receptor binding site. Class I and II antibodies have epitopes that at least partially overlap the RBM site and class III and IV do not bind the RBM region. Class I and IV are only able to engage RBD in an "up" position. In contrast, Class II and III antibodies can bind to RBD with it is in either the "up" or "down" position. See Barnes et al., Nature 588(7839):682-687. doi: 10.1038/s41586-020-2852-1, ePub October 12, 2020, incorporated herein by reference. This classification is used to quickly map epitopes of antibodies by performing competition assays with antibodies of known class and epitope.

The receptor binding domain (RBD) of the SARS-CoV-2 spike protein (S) contains a receptor binding motif (RBM) that binds human cellular receptor protein, angiotensin converting enzyme (ACE)2. RBM binding to ACE2 is required for SARS-CoV-2 to infect cells. The RBD exists in two conformations referred to as "up" or "down". When the RBD is "down", the RBM is not able to bind ACE2. However, when RBD is "up", the RBM is able to binds to ACE2. Barnes et al. defined a functional classification schema for antibodies targeting the RBD that is based upon the RBD state an antibody can bind and whether the epitope within RBD overlaps with the ACE2 receptor binding site (pubmed.ncbi.nlm.nih.gov/33045718/). Class I and II antibodies have epitopes that at least partially overlap the RBM site and class III and IV do not bind the RBM region. Class I and IV are only able to engage RBD in an "up" position. In contrast, Class II and III antibodies can bind to RBD with it is in either the "up" or "down" position.

Omicron variants seem to be converging on mutation at position 486 which impact neutralization and binding of Class I antibodies, including COV2-2196 (a major component of EVUSHELD^{™}), positions 444-446 which impact class III antibodies, including LY-CoV1404, and sometimes contain changes at 452, which impacts class II antibodies. Since the new variants combine mutations at these positions, these variants may be highly resistant to clinical antibodies that were of use in treating the original SARS-CoV-2 virus, and delta, see Wang et al., doi.org/10.1101/2022.11.23. 517532, available as biorxiv.org/content/10.1101/2022.11.23.517532v1.full, November 28, 2022.

**Sequence identity:** The identity between two or more nucleic acid sequences, or two or more amino acid sequences, is expressed in terms of the percentage identity between the sequences. Sequence identity can be measured in terms of percentage identity; the higher the percentage, the more identical the sequences. Homologs and variants of a V_{L} or a V_{H} of an antibody that specifically binds a target antigen are typically characterized by possession of at least about 75% sequence identity, for example at least about 80%, 85%, 90%, 91%, 92%, 93%, 94%, 95%, 96%, 97%, 98% or 99% sequence identity counted over the full-length alignment with the amino acid sequence of interest.

Any suitable method may be used to align sequences for comparison. Non-limiting examples of programs and alignment algorithms are described in: Smith and Waterman, Adv. Appl. Math. 2(4):482-489, 1981; Needleman and Wunsch, J. Mol. Biol. 48(3):443-453, 1970; Pearson and Lipman, Proc. Natl. Acad. Sci. U.S.A. 85(8):2444-2448, 1988; Higgins and Sharp, Gene, 73(1):237-244, 1988; Higgins and Sharp, Bioinformatics, 5(2):151-3, 1989; Corpet, Nucleic Acids Res. 16(22):10881-10890, 1988; Huang et al. Bioinformatics, 8(2):155-165, 1992; and Pearson, Methods Mol. Biol. 24:307-331, 1994., Altschul et al., J. Mol. Biol. 215(3):403-410, 1990, presents a detailed consideration of sequence alignment methods and homology calculations. The NCBI Basic Local Alignment Search Tool (BLAST) (Altschul et al., J. Mol. Biol. 215(3):403-410, 1990) is available from several sources, including the National Center for Biological Information and on the Internet, for use in connection with the sequence analysis programs blastp, blastn, blastx, tblastn, and tblastx. Blastn is used to compare nucleic acid sequences, while blastp is used to compare amino acid sequences. Additional information can be found at the NCBI web site.

Generally, once two sequences are aligned, the number of matches is determined by counting the number of positions where an identical nucleotide or amino acid residue is present in both sequences. The percent sequence identity between the two sequences is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100.

**Specifically bind:** When referring to an antibody or antigen binding fragment, refers to a binding reaction which determines the presence of a target protein, such as a coronavirus spike protein, in the presence of a heterogeneous population of proteins and other biologics. Thus, under designated conditions, an antibody binds preferentially to a particular target protein, peptide or polysaccharide (such as an antigen present on the surface of a pathogen, for example a coronavirus spike protein and does not bind in a significant amount to other proteins present in the sample or subject. With regard to a spike protein, the epitope may be present on the spike protein of more than one type of coronavirus, such that the antibody binds to the spike protein on more than one types of virus, but does not bind to other proteins, such as proteins from other viruses or other proteins (non-spike) of coronavirus. Specific binding can be determined by standard methods. See Harlow & Lane, Antibodies, A Laboratory Manual, 2nd ed., Cold Spring Harbor Publications, New York (2013), for a description of immunoassay formats and conditions that can be used to determine specific immunoreactivity.

With reference to an antibody-antigen complex, specific binding of the antigen and antibody has a K_{D} of less than about 10⁻⁷ Molar, such as less than about 10⁻⁸ Molar, 10⁹, or even less than about 10⁻¹⁰ Molar. K_{D} refers to the dissociation constant for a given interaction, such as a polypeptide ligand interaction or an antibody antigen interaction. For example, for the bimolecular interaction of an antibody or antigen binding fragment and an antigen it is the concentration of the individual components of the bimolecular interaction divided by the concentration of the complex.

An antibody that specifically binds to an epitope on a coronavirus spike protein, such as the RBD domain, can bind molecules/agents including this domain, including viruses, substrate to which the spike protein is attached, or the protein in a biological specimen. It is, of course, recognized that a certain degree of non-specific interaction may occur between an antibody and a non-target. Typically, specific binding results in a much stronger association between the antibody and a spike protein than between the antibody other different coronavirus proteins (such as the E, M or N protein) or from non-coronavirus proteins. Specific binding typically results in greater than 2-fold, such as greater than 5-fold, greater than 10-fold, or greater than 100-fold increase in amount of bound antibody (per unit time) to a protein including the epitope or cell or tissue expressing the target epitope as compared to a protein or cell or tissue lacking this epitope. Specific binding to a protein under such conditions requires an antibody that is selected for its specificity for a particular protein. A variety of immunoassay formats are appropriate for selecting antibodies or other ligands specifically immunoreactive with a particular protein. For example, solid-phase ELISA immunoassays are routinely used to select monoclonal antibodies specifically immunoreactive with a protein.

**Subject:** Living multi-cellular vertebrate organisms, a category that includes human and non-human mammals, such as non-human primates, pigs, camels, bats, sheep, cows, dogs, cats, rodents, and the like. In an example, a subject is a human. In a particular example, the subject is a human. In an additional example, a subject is selected that is in need of inhibiting a SARS-CoV-2 infection. For example, the subject is either uninfected and at risk of the SARS-CoV-2 infection or is infected and in need of treatment.

**Transformed:** A transformed cell is a cell into which a nucleic acid molecule has been introduced by molecular biology techniques. As used herein, the term transformed and the like (e.g., transformation, transfection, transduction, etc.) encompasses all techniques by which a nucleic acid molecule might be introduced into such a cell, including transduction with viral vectors, transformation with plasmid vectors, and introduction of DNA by electroporation, lipofection, and particle gun acceleration.

**Vector:** An entity containing a nucleic acid molecule (such as a DNA or RNA molecule) bearing a promoter(s) that is operationally linked to the coding sequence of a protein of interest and can express the coding sequence. Non-limiting examples include a naked or packaged (lipid and/or protein) DNA, a naked or packaged RNA, a subcomponent of a virus or bacterium or other microorganism that may be replication-incompetent, or a virus or bacterium or other microorganism that may be replication-competent. A vector is sometimes referred to as a construct. Recombinant DNA vectors are vectors having recombinant DNA. A vector can include nucleic acid sequences that permit it to replicate in a host cell, such as an origin of replication. A vector can also include one or more selectable marker genes and other genetic elements. Viral vectors are recombinant nucleic acid vectors having at least some nucleic acid sequences derived from one or more viruses. In some aspects, a viral vector comprises a nucleic acid molecule encoding a disclosed antibody or antigen binding fragment that specifically binds to a coronavirus spike protein and neutralizes the coronavirus. In some aspects, the viral vector can be an adeno-associated virus (AAV) vector.

**Under conditions sufficient for:** A phrase that is used to describe any environment that permits a desired activity.

### II. Description of Several Embodiments

Monoclonal antibodies with activity against SARS-CoV-2, and SARS-CoV-related viruses from bats, pangolins, etc - collectively known as sarbecoviruses-are of value in pandemic preparedness efforts. These zoonotic coronaviruses can use multiple ACE2 orthologues for host cell entry and exhibit a similar binding affinity as SARS-CoV-2. Therefore, these viruses may cause cross-species transmission and lead to human infection. Although many monoclonal antibodies have been identified and shown protection efficacy for SARS-CoV-2, most of them exhibit no or weak cross-neutralizing activity against zoonotic coronaviruses. Hence, there is need to develop broad-spectrum monoclonal antibodies or antiviral agents against sarbecoviruses to combat future threat of zoonotic coronaviruses.

Monoclonal antibodies are also of use that neutralize Omicron variants. Disclosed herein are 23 monoclonal antibodies that have a high neutralization titers against the current dominant variant lineage XBB and its relatives. All 23 target the receptor-binding domain (RBD) of the spike protein. These antibodies have a unique neutralization breadth and potency. All are more broadly cross-reactive than, and most are more potent, than Ly-CoV1404 (bebtelovimab), the previous leading clinical monoclonal antibody; neutralize all current variants; and also neutralize multiple related sarbecoviruses.

Isolated monoclonal antibodies and antigen binding fragments that specifically bind a coronavirus spike protein are provided. The monoclonal antibodies and antigen binding fragments specifically bind to a coronavirus spike protein and neutralize SARS-CoV-2. In some aspects, the antibody specifically binds the spike protein of XBB.1, XBB1.5 and XBB1.16, as well as at least one SARS-CoV-2 related virus from bats or pangolins. The antibodies and antigen binding fragments can be fully human.

The antibodies and antigen binding fragments can neutralize SARS-CoV-2. In some aspects the disclosed antibodies can inhibit a SARS-CoV-2 infection *in vivo,* and can be administered prior to, or after, an infection with SARS-CoV-2.

Multispecific antibodies, such as bispecific antibodies, including the variable domains of these antibodies are also provided. In addition, disclosed herein are compositions comprising the antibodies and antigen binding fragments and a pharmaceutically acceptable carrier. Nucleic acids encoding the antibodies, antigen binding fragments, variable domains, and expression vectors (such as adeno-associated virus (AAV) viral vectors) comprising these nucleic acids are also provided. The antibodies, antigen binding fragments, nucleic acid molecules, host cells, and compositions can be used for research, diagnostic, treatment and prophylactic purposes. For example, the disclosed antibodies and antigen binding fragments can be used to diagnose a subject with a SARS-CoV-2 infection or can be administered to inhibit a SARS-CoV-2 infection in a subject.

### A. Monoclonal Antibodies that Specifically Bind a Coronavirus Spike protein and Antigen Binding Fragments Thereof

The discussion of monoclonal antibodies below refers to isolated monoclonal antibodies that include heavy and/or light chain variable domains (or antigen binding fragments thereof) comprising a CDR1, CDR2, and/or CDR3 with reference to the IMGT numbering scheme (unless the context indicates otherwise). Various CDR numbering schemes (such as the Kabat, Chothia or IMGT numbering schemes) can be used to determine CDR positions. The amino acid sequence and the CDRs of the heavy and light chain of the disclosed monoclonal antibody according to the IMGT numbering scheme are provided in the listing of sequences, but these are exemplary only. The disclosed monoclonal antibodies specifically bind the Spike protein of SARS-CoV-2.

### 1. Exemplary Monoclonal Antibodies

In some aspects, a monoclonal antibody is provided that comprises the heavy and light chain CDRs of any one of the antibodies described herein. In other aspects, a monoclonal antibody is provided that comprises the heavy and light chain variable regions of any one of the antibodies described herein. Antigen binding fragments of these monoclonal antibodies are also provided. In some aspects, this antibody, or an antigen binding fragment thereof, neutralizes SARS-CoV-2 BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and/or XEC, RaTG13, Pangolin GD, Pangolin GX-P2V, WIV1 and/or SHC-014.

In aspects, disclosed is A18-448.1, and antigen binding fragment thereof. In more aspects, this antibody or an antigen binding fragment thereof, neutralizes BA.1, BA.2, BA.4/5, EG.5.1, BA.2.86, JN.1, KP.2, KP.3, LB.1, KP.2.3, KP.3.1.1 and XEC. In other aspects, this antibody, or an antigen binding fragment thereof, neutralizes SARS-CoV related sarbecoviruses from humans, bats, and/or civet cats.

In other aspects, disclosed is F768-104_B2, and antigen binding fragments thereof. In some aspects, this antibody, or an antigen binding fragment thereof, neutralizes an animal-derived SARS-CoV-2 related virus. In some aspects, this antibody, or an antigen binding fragment thereof, specifically binds a spike protein from BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and XEC.

In further aspects, disclosed are Group 3 antibodies, and antigen biding fragments thereof. These antibodies include A80-102_G5, A80-338-19.5, A80-338-19.6, A80-338-19.8, and A80-338-19.9. In some aspects, these antibodies and antigen binding fragments have neutralizing activity against SARS-CoV-2 variants and/or SARS-CoV-related sarbecoviruses. In more aspects, these antibodies and antigen binding fragments have neutralizing activity against omicron BA.1, BA.2, BA.5, EG.5.1, BA.2.86, JN.1, KP.2, KP.3, LB.1, KP.2.3, KP.3.1.1 and XEC. In further aspects, these antibody and antigen binding fragment have neutralizing activity against SARS-CoV-2 related viruses from animals, including Pangolin GX variants, as well as SARS-CoV related sarbecoviruses from humans, bats, and civet cats.

In more aspects, disclosed are Group 4 antibodies, and antigen biding fragments thereof. These antibodies include E184-105_F3, A63-652-32.1, F768-104_G7, E184-105_H1, F768-104_D10, and A63-652-1.4. In some aspects, these antibodies and antigen binding fragments have neutralizing activity against Omicron lineage pseudotyped viruses. In more aspects, the antibodies and antigen binding fragments have neutralizing activity against BA.1, BA.2, BA.4/5, BA.2.75.2, BQ1.1, XBB, EG.5.1 and BA.2.86. In more aspects, these antibodies and antigen binding fragments neutralize SARS-CoV-2 related coronaviruses from bats and pangolins. F768-104_D10 and F768-104_G7 also neutralize the D614G strain bearing the F456L mutation. A63-652-1.4 has neutralizing activity against JN.1, KP.2, KP.3, LB.1, KP.2.3, KP.3.1.1 and XEC.

In other aspects, disclosed are Group 5 antibodies, and antigen biding fragments thereof. These antibodies include E184-105_E5, E184-105_H9, E184-105_H2, and A80-102+103_G1. In some aspects, these antibodies and antigen binding fragment have neutralizing activity against SARS-CoV-2 Omicron lineage pseudotyped viruses. In more aspects, these antibody and antigen binding fragment have neutralizing activity against BA.1, BA.2, BA.4/5, BA.2.75.2, BQ1.1, XBB, EG.5.1 and BA.2.86. In further aspects, these antibodies and antigen binding fragment neutralizes SARS-CoV-2 related coronaviruses from bats and pangolins. E184-105_E5 and A80-102+103_G1 also neutralize D614G strain bearing a variety of mutations known to reduce activity of other Class I antibodies, including the F456L mutation.

In further aspects, disclosed are Group 6 antibodies, and antigen biding fragments thereof. These antibodies include A80-103_E2, A80-103_B6, A18-618-452.1, A80-102+103_C1, A43-d321.10.4, and F768-100-pt2_A05. In some aspects, these antibodies and antigen binding fragments have neutralizing activity against SARS-CoV-2 Omicron lineage pseudotyped viruses. In more aspects, these antibodies and antigen binding fragments have neutralizing activity against BA.1, BA.2, BA.4/5, BA.2.75.2, BQ1.1, and XBB. In other aspects, these antibody and antigen binding fragments have neutralizing activity against BA EG.5.1, FL.1.5.1, BA.2.86, JN.1, KP.2, KP.3, LB.1, KP.2.3, KP.3.1.1 and XEC. In further aspects, these antibodies and antigen binding fragments neutralize SARS-CoV-2-related coronaviruses from animals, as well as SARS-CoV.

Table A provides the antibody names, sequences contained in the V_{H}, HCDR1, HCDR2, HCDR3, V_{L}, LCDR1, LCDR2, and LCDR3, and some position information, for the antibodies disclosed herein.

**Table A. IMGT CDRs of Antibodies and SEQ ID NOs**

| | | | |
|---|---|---|---|
| A18-618-448.1 V_{H} | | | |
| QVQLVQSGAEVKKPGSSVKVSCKVSGGTFSDSAVSWVRQAPGQGLEWMGRIIPIFSTIEYAQ NFQGRVTITADESTTTVYMELSYLGLEDTAVYYCARDRPIAVEYTGNWFDPWGQGTLVTV SS (SEQ ID NO: 1) | | | |

| V_{H} | SEQ ID NO: 1 positions | CDR protein sequence | CDR SEQ ID NO |
|---|---|---|---|
| HCDR1 | 26-33 | GGTFSDSA | 2 |
| HCDR2 | 51-58 | IIPIFSTI | 3 |
| HCDR3 | 97-113 | ARDRPIAVEYTGNWFDP | 4 |
| A18-618-448.1 V_{L} | | | |
| DVQMTQSPSSLSASVGDRVTITCRASQTIVGYLNWYQQQPGRAPKLLIYAASNLQSGVPPRF SGSGSGTDFTLTISDLQPEDFATYYCQQSYLTPPNTFGQGTKLEIK (SEQ ID NO: 5) | | | |

| V_{L} | SEQ ID NO: 5 positions | CDR Sequence | CDR SEQ ID NO |
|---|---|---|---|
| LCDR1 | 27-32 | QTIVGY | 6 |
| LCDR2 | 50-52 | AAS | 7 |
| LCDR3 | 89-98 | QQSYLTPPNT | 8 |
| F768-104_B2 V_{H} | | | |
| QVHLVQSGAEVKKPGASVKVSCKASGYTFTDYFIHWVRQAPGQGLEWMGWINPDDGDTN YAQKFHGWVAMTRDTSINTAYMELSRLKSNDTAVYYCARGTFSLRGFDWFDPWGQGTLV TVSS (SEQ ID NO: 9) | | | |

| V_{H} | SEQ ID NO: 9 positions | CDR protein sequence | CDR SEQ ID NO |
|---|---|---|---|
| HCDR1 | 26-33 | GYTFTDYF | 10 |
| HCDR2 | 51-58 | INPDDGDT | 11 |
| HCDR3 | 97-111 | ARGTFSLRGFDWFDP | 12 |
| F768-104_B2 V_{L} | | | |
| EIVLTQSPATLSVSPGERATLSCRASQSVSSLLAWYQRKPGQPPRLLIYGASTRATGIPARFSG SGSGTEFTLTISSLQSEDFAVYYCQQYNDWWTFGQGTKVEIK (SEQ ID NO: 13) | | | |

| V_{L} | SEQ ID NO: 13 positions | CDR Sequence | CDR SEQ ID NO |
|---|---|---|---|
| LCDR1 | 27-32 | QSVSSL | 14 |
| LCDR2 | 50-52 | GAS | 15 |
| LCDR3 | 89-96 | QQYNDWWT | 16 |
| E184-105_F3 V_{H} | | | |
| EVQLVESGGGLIQPGGSLRLSCAASEIIVSRNYMNWVRQAPGKGLEWVSVLYAGGSKFYAD SVKGRFTISRDDSNNTLYLQMSSLRVEDTAIYYCARGWDYYGMDVWGQGTTVTVAS (SEQ ID NO: 17) | | | |

| V_{H} | SEQ ID NO: 17 positions | CDR protein sequence | CDR SEQ ID NO |
|---|---|---|---|
| HCDR1 | 26-33 | EIIVSRNY | 18 |
| HCDR2 | 51-57 | LYAGGSK | 19 |
| HCDR3 | 96-106 | ARGWDYYGMDV | 20 |
| E184-105_F3 V_{L} | | | |
| DIQLTQSPSFLSASVGDRVTITCRASQGINRYLAWYQQKPGKAPKLLIYGASTLQSGVPSRFS GSGSGTEFTLTISSLQAEDFATYFCQHLNDYPVFGPGTKVNIK (SEQ ID NO: 21) | | | |

| V_{L} | SEQ ID NO: 21 positions | CDR Sequence | CDR SEQ ID NO |
|---|---|---|---|
| LCDR1 | 27-32 | QGINRY | 22 |
| LCDR2 | 50-52 | GAS | 23 |
| LCDR3 | 89-96 | QHLNDYPV | 24 |
| A63-652-32.1 V_{H} | | | |
| EVQLVESGGGLVQPGGSLRLSCAASGVIVSSNYMNWVRQAPGKGLEWVSVIYSGGSTFYA DSVRGRFTISRHDSQNTLFLQMNSLRPEDTAVYYCARDTVVYGMDVWGQGTTVIVSS (SEQ ID NO: 25) | | | |

| V_{H} | SEQ ID NO: 25 positions | CDR protein sequence | CDR SEQ ID NO |
|---|---|---|---|
| HCDR1 | 26-33 | GVIVSSNY | 26 |
| HCDR2 | 51-57 | IYSGGST | 27 |
| HCDR3 | 96-106 | ARDTVVYGMDV | 28 |
| A63-652-32.1 V_{L} | | | |
| DIQLTQSPSFLSASVGDRVTLTCRASQGITADLAWYQQKPGKAPKLLIYAASTLQSGVPSRFS GSGSGTEFTLTVGSLQPEDFATYYCQHHDTYSIAFGQGTRLEIK (SEQ ID NO: 29) | | | |

| V_{L} | SEQ ID NO: 29 positions | CDR protein sequence | CDR SEQ ID NO |
|---|---|---|---|
| LCDR1 | 27-32 | QGITAD | 30 |
| LCDR2 | 50-52 | AAS | 31 |
| LCDR3 | 89-97 | QHHDTYSIA | 32 |
| F768-104_G7 V_{H} | | | |
| EVQLVETGGGLVQPGGSLRLSCAASGVIVSRNYMSWVRQAPGKGLEWVSLIFAGGSTFYSD SVKGRFTISRDDSRNTVYLQMNSLRAEDTAVYYCARDLDVRGGLDVWGQGTTVTVSS (SEQ ID NO: 33) | | | |

| V_{H} | SEQ ID NO: 33 positions | CDR protein sequence | CDR SEQ ID NO |
|---|---|---|---|
| HCDR1 | 26-33 | GVIVSRNY | 34 |
| HCDR2 | 51-57 | IFAGGST | 35 |
| HCDR3 | 96-107 | ARDLDVRGGLDV | 36 |
| F768-104_G7 V_{L} | | | |
| DVQLTQFPSSLSASVGDRVTITCRASQDIPNYINWYQQKPGKAPRLLIYAASTLQSGVPSRFS GSGSGTDFTLTISSLQPEDFGTYYCQQSDTRTFGPGTKLEIK (SEQ ID NO: 37) | | | |

| V_{L} | SEQ ID NO: 37 positions | CDR Sequence | CDR SEQ ID NO |
|---|---|---|---|
| LCDR1 | 27-32 | QDIPNY | 38 |
| LCDR2 | 50-52 | AAS | 39 |
| LCDR3 | 89-95 | QQSDTRT | 40 |
| E184-105_H1 V_{H} | | | |
| EVQLVESGGGLVQPGGSLRVSCAASGLTVSSNYMTWVRQAPGKGLEWVSVIYPGGSAFYA DSVKGRFSITRDNSKNTLYLQMNSLRAEDTAVYYCARDRPSLGVGGGLALRDWGQGTLV TVSS (SEQ ID NO: 41) | | | |

| V_{H} | SEQ ID NO: 41 positions | CDR Sequence | CDR SEQ ID NO |
|---|---|---|---|
| HCDR1 | 26-33 | GLTVSSNY | 42 |
| HCDR2 | 51-57 | IYPGGSA | 43 |
| HCDR3 | 96-113 | ARDRPSLGVGGGGLALRD | 44 |
| E184-105_H1 V_{L} | | | |
| DIQMTQSPPSLSASVGDRVTITCQASQDINKFLNWFQQKPGKAPKLLIYDASNVEPGVPSRFS GSGSGTDFTFTISSLQPEDIATYYCQQYDNLPPGFGGGTKVEIR (SEQ ID NO: 45) | | | |

| V_{L} | SEQ ID NO: 45 positions | CDR protein sequence | CDR SEQ ID NO |
|---|---|---|---|
| LCDR1 | 27-32 | QDINKF | 46 |
| LCDR2 | 50-52 | DAS | 47 |
| LCDR3 | 89-97 | QQYDNLPPG | 48 |
| F768-104_D10 V_{H} | | | |
| EVQLVESGGGLVQPGGSLRLSCAASGIVVSANYMTWVRQAPGKGLEGVSLLFAGGSTFYA DSVKGRFTISRDNSKNTVFLQMNNLRAEDTAVYYCARNLVYYGMDVWGQGTTVTVST (SEQ ID NO: 49) | | | |

| V_{H} | SEQ ID NO: 49 positions | CDR Sequence | CDR SEQ ID NO |
|---|---|---|---|
| HCDR1 | 26-33 | GIVVSANY | 50 |
| HCDR2 | 51-57 | LFAGGST | 51 |
| HCDR3 | 96-106 | ARNLVYYGMDV | 52 |
| F768-104_D10 V_{L} | | | |
| DIQLTQSPSFLSASVGDRVTITCRASQGISDSLAWYQQKPGTAPKLLIYGASTLQSGVPSRFS GSGSATEFTLTISSLQPEDFATYYCQQLNSDSYTFGQGTKLEIK (SEQ ID NO: 53) | | | |

| V_{H} | SEQ ID NO: 53 positions | CDR protein sequence | CDR SEQ ID NO |
|---|---|---|---|
| LCDR1 | 27-32 | QGISDS | 54 |
| LCDR2 | 50-52 | GAS | 55 |
| LCDR3 | 89-97 | QQLNSDSYT | 56 |
| A63-652-1.4 V_{H} | | | |
| EEQLVVSGGGLVQPGGSLRLSCTASEITVIRNYMNWVRQAPGKGLEWVSVIYPGGSTFYAD SVQGRFTISRDNSKNTLYLQMNSLRAEDTAVYYCSRPIVGGRRGMDVWGQGTTVTVSS (SEQ ID NO: 57) | | | |

| V_{H} | SEQ ID NO: 57 positions | CDR Sequence | CDR SEQ ID NO |
|---|---|---|---|
| HCDR1 | 26-33 | EITVIRNY | 58 |
| HCDR2 | 51-57 | IYPGGST | 59 |
| HCDR3 | 96-108 | SRPIVGGRRGMDV | 60 |
| A63-652-1.4 V_{L} | | | |
| DIQMTQSPSSLSASVGDRVTITCQASQDINNFLNWYQQKPGKAPKLLIYDASNLETGVPSRFS GSGSGTDFTFTIISLQPEDIATYYCQHYDNPPYTFGQGTKLEIK (SEQ ID NO: 61) | | | |

| V_{L} | SEQ ID NO: 61 positions | CDR protein sequence | CDR SEQ ID NO |
|---|---|---|---|
| LCDR1 | 27-32 | QDINNF | 62 |
| LCDR2 | 50-52 | DAS | 63 |
| LCDR3 | 89-97 | QHYDNPPYT | 64 |
| E184-105_E5 V_{H} | | | |
| QLQLQESGPGLVKPSETLSLTCTVSGGSISRTSYYWGWIRQTPGKGLEWIGAIFYSGSTHYNP SLKSRVTISVDTSKDQFSLKLSSVTAADTAIYYCTRQMGLIDYWGQGTLVTVSS (SEQ ID NO: 65) | | | |

| V_{H} | SEQ ID NO: 65 positions | CDR Sequence | CDR SEQ ID NO |
|---|---|---|---|
| HCDR1 | 26-35 | GGSISRTSYY | 66 |
| HCDR2 | 53-59 | IFYSGST | 67 |
| HCDR3 | 98-106 | TRQMGLIDY | 68 |
| E184-105_E5 V_{L} | | | |
| DIQMTQSPFSLSASVGDRVTITCRASQGIGNSLAWYQQRPGKAPKLLLYAASTLESGVPSRF GGSGSGTDFTLTISSLQPEDFATYYCQQYYSTPPFTFGPGTKVDFK (SEQ ID NO: 69) | | | |

| V_{L} | SEQ ID NO: 69 positions | CDR protein sequence | CDR SEQ ID NO |
|---|---|---|---|
| LCDR1 | 27-32 | QGIGNS | 70 |
| LCDR2 | 50-52 | AAS | 71 |
| LCDR3 | 89-98 | QQYYSTPPFT | 72 |
| E184-105_H9 V_{H} | | | |
| QLQLQESGPGLVKPSETLSLTCTVSGDSISRSSYYWGWVRQPPGKGLEWIGNIYYSGTTYFN PSLQSRVTISVDTSKNQFSLKLSSVTAADTAVYYCARQMGFVDYWGQGTLVTVSS (SEQ ID NO: 73) | | | |

| V_{H} | SEQ ID NO: 73 positions | CDR protein sequence | CDR SEQ ID NO |
|---|---|---|---|
| HCDR1 | 26-35 | GDSISRSSYY | 74 |
| HCDR2 | 53-59 | IYYSGTT | 75 |
| HCDR3 | 98-106 | ARQMGFVDY | 76 |
| E184-105_H9 V_{L} | | | |
| DIQMTQSPSSLSASVGDRVTITCRASQGISNSLAWYQQKPGRAPKFLLYAASTLESGVPSRFS GSGSETDFTLTISSLQPEDFATYYCQQYYSPPPFTFGPGTKVDIK (SEQ ID NO: 77) | | | |

| V_{L} | SEQ ID NO:77 positions | CDR Sequence | CDR SEQ ID NO |
|---|---|---|---|
| LCDR1 | 27-32 | QGISNS | 78 |
| LCDR2 | 50-52 | AAS | 79 |
| LCDR3 | 89-98 | QQYYSPPPFT | 80 |
| | | | |
| E184-105_H2 V_{H} | | | |
| QLQLQESGPGLVKPSETLSLTCSVSGGSISSSSYYWGWIRQPPGKGLEWIGNMFYSGSTYYN PSLKSRVTISVDTSQNQVSLKLSSVTASDTAVYYCTRQMGFVDYWGQGTLVTVSS (SEQ ID NO: 81) | | | |

| V_{H} | SEQ ID NO: 81 positions | CDR protein sequence | CDR SEQ ID NO |
|---|---|---|---|
| | 26-35 | GGSISSSSYY | 82 |
| | 53-59 | MFYSGST | 83 |
| | 98-106 | TRQMGFVDY | 84 |
| E184-105_H2 V_{L} | | | |
| DIQLTQSPSSLSASVGDRVTITCRASEGISNSLAWYQHKPGKAPRFLLYAASTLESGVPSRFS GSGSGTDFTLTISGLQPEDFATYYCQQYYSSPPFTFGPGTTVDIR (SEQ ID NO: 85) | | | |

| V_{L} | SEQ ID NO:85 positions | CDR Sequence | CDR SEQ ID NO |
|---|---|---|---|
| | 27-32 | EGISNS | 86 |
| | 50-52 | AAS | 87 |
| | 89-98 | QQYYSSPPFT | 88 |
| A80-102+103_G1 V_{H} | | | |
| EVQLVQSGAEVKKPGESLKISCKGSGYSFSNYWIGWVRQMPGKGLEWMGIVYPYDSDTRY SPSFQGQVTISADKSLTTAYLQWSSVKASDTAIYYCARHGHYYGSGSYDGPDYWGQGTLV TVSS (SEQ ID NO: 89) | | | |

| V_{H} | SEQ ID NO: 89 positions | CDR protein sequence | CDR SEQ ID NO |
|---|---|---|---|
| | 26-33 | GYSFSNYW | 90 |
| | 51-58 | VYPYDSDT | 91 |
| | 97-113 | ARHGHYYGSGSYDGPDY | 92 |
| A80-102+103_G1 V_{L} | | | |
| QSVLTQPPSASGPPGQRVTISCSGSSSNIGGNTVNWYQQLPGTAPKLLIYNNNQRPSGVPDRF SGSKSGTSASLTISGLQSEDEADYYCAAWDDSLNGQVFGGGTKLTVL (SEQ ID NO: 93) | | | |

| V_{L} | SEQ ID NO: 93 positions | CDR Sequence | CDR SEQ ID NO |
|---|---|---|---|
| | 26-33 | SSNIGGNT | 94 |
| | 51-53 | NNN | 95 |
| | 90-100 | AAWDDSLNGQV | 96 |
| A80-103_E2 V_{H} | | | |
| EVQLVESGGNLVQPGGSLRLSCAASGFTFSSYWMTWVRRAPGKGLEWVANIKEDGSEKYY VDSVKGRFTISRDNAKNSLYLHMNSLRGEDTAVYYCARDPHTNGWYREKYYNGMDVWG QGTTVTVSS (SEQ ID NO: 97) | | | |

| V_{H} | SEQ ID NO: 97 positions | CDR protein sequence | CDR SEQ ID NO |
|---|---|---|---|
| | | GFTFSSYW | 98 |
| | | IKEDGSEK | 99 |
| | | ARDPHTNGWYREKYYNGM DV | 100 |
| A80-103_E2 V_{L} | | | |
| SYVLTQPPSMSVAAGKTAKITCGGNNIGSKSVHWYQQKPGQAPVLVIYSDSDRPSGIPERFS GSNSGNTATLTISRVEVEAGDEADYYCQVWDSSSDHWVFGGGTKLTVL (SEQ ID NO: 101) | | | |

| V_{L} | SEQ ID NO: 101 positions | CDR Sequence | CDR SEQ ID NO |
|---|---|---|---|
| | | NIGSKS | 102 |
| | | SDS | 103 |
| | | QVWDSSSDHWV | 104 |
| A80-103_B6 V_{H} | | | |
| EVQLVESGGNLVQPGRSLRLSCAASGFTFDDNAMHWVRQAPGKGLEWVSSINWSGGDIAY ADSVKGRFTISRDNAKNSLYLQMNSLRADDTALYYCAKGTVGEILFPLDYWGQGTLVTVSS (SEQ ID NO: 105) | | | |

| V_{H} | SEQ ID NO: 105 positions | CDR protein sequence | CDR SEQ ID NO |
|---|---|---|---|
| | | GFTFDDNA | 106 |
| | | INWSGGDI | 107 |
| | | AKGTVGEILFPLDY | 108 |
| A80-103_B6 V_{L} | | | |
| QSVLTQPPSASGTPGQRVTISCSGSSSNVGTYTVNWYQQLPGTAPKLLVYSYNHRPSGVPDR FSGSKSGTSASLAISGLQSEDEADYYCAAWDDSLNGWVFGGGTKLSVL (SEQ ID NO: 109) | | | |

| V_{L} | SEQ ID NO: 109 positions | CDR Sequence | CDR SEQ ID NO |
|---|---|---|---|
| | | SSNVGTYT | 110 |
| | | SYN | 111 |
| | | AAWDDSLNGWV | 112 |
| A18-618-452.1 V_{H} | | | |
| QGQLVESGGGVVQPGRSLRLSCAASGFTFSSYAMHWVRQTPGKGPEWVALISDDGRMKM YEDSVKGRFIISRDNPKNTLHLQLNTLRAEDTAVYYCTREDSRGLYWYFDLWGRGTLVTVS S (SEQ ID NO: 113) | | | |

| V_{H} | SEQ ID NO: 113 positions | CDR protein sequence | CDR SEQ ID NO |
|---|---|---|---|
| | | GFTFSSYA | 114 |
| | | ISDDGRMK | 115 |
| | | TREDSRGLYWYFDL | 116 |
| A18-618-452.1 V_{L} | | | |
| DIQMTQSPSSLSASVGDKITITCRASQTIGIYLNWYQQKPGKGPKLLVYAASNLHTGVPSRFS GSGSGTDFTLTISSLQPEDFALYYCQQSYITPQGTFGQGTKVEIK (SEQ ID NO: 117) | | | |

| V_{L} | SEQ ID NO: 117 positions | CDR Sequence | CDR SEQ ID NO |
|---|---|---|---|
| | | QTIGIY | 118 |
| | | AAS | 119 |
| | | QQSYITPQGT | 120 |
| A80-102+103_C1 V_{H} | | | |
| QEHLVESGGGVVQPGRSLRLSCAVSGFTFSRYAMHWVRQAPGKGLEWVAVISDDGRTKYY GDSAEGRFTISRDNSKNTLFLQMNSLRADDTAVFYCARDRWHRVTPAFYYFDLWGQGTLV TVSS (SEQ ID NO: 121) | | | |

| V_{H} | SEQ ID NO: 121 positions | CDR protein sequence | CDR SEQ ID NO |
|---|---|---|---|
| | | GFTFSRYA | 122 |
| | | ISDDGRTK | 123 |
| | | ARDRWHRVTPAFYYFDL | 124 |
| A80-102+103_C1 V_{L} | | | |
| EVVMTQSPATLSVSPGERATLSCRAGQSVNSNLAWYQHKPGQAPRLLIYDASTRATGIPARF SGSGSGTDFTLTISSLQSDDFALYYCQQYDNWYTFGQGTKLEIK (SEQ ID NO: 125) | | | |

| V_{L} | SEQ ID NO: 125 positions | CDR Sequence | CDR SEQ ID NO |
|---|---|---|---|
| | | QSVNSN | 126 |
| | | DAS | 127 |
| | | QQYDNWYT | 128 |
| A43-d321.10.4 V_{H} | | | |
| QVQLVESGGGVVQPGKSLRLSCAASGFTFRNYGMVWVRQAPGKGLEWVADIVYDGSDKY YADSVKGRFTISRDNAKNTLYLQMNSLRTEDTAVYFCAKRGGPYCSAGPCYGGYFDYWGQ GSLVTVSS (SEQ ID NO: 129) | | | |

| V_{H} | SEQ ID NO: 129 positions | CDR protein sequence | CDR SEQ ID NO |
|---|---|---|---|
| | | GFTFRNYG | 130 |
| | | IVYDGSDK | 131 |
| | | AKRGGPYCSAGPCYGGYFD Y | 132 |
| A43-d321.10.4 V_{L} | | | |
| DMQMTQSPSSLSASVGDRVTITCQASQDISNYLNWYQQKPGKAPKLLIYDASNLETGVPSRF SGSGSGTDFTFTIGSLQPEDFATYYCQQYGDLPVTFGGGTKVEIK (SEQ ID NO: 133) | | | |

| V_{L} | SEQ ID NO: 133 positions | CDR Sequence | CDR SEQ ID NO |
|---|---|---|---|
| | | QDISNY | 134 |
| | | DAS | 135 |
| | | QQYGDLPVT | 136 |
| F768-100-pt2_A05 V_{H} | | | |
| EAQLVESGGGLVQPGRSLRLSCTASGFTFGDSAMSWFRQAPGKGLEWVGFIRSKAWGGTT DYAASVKGRFTISRDDSKSIAYLQMNSLKTEDTAVYYCTRDRRCSGLSCYLLDFLGQGTLV TVSS (SEQ ID NO: 137) | | | |

| V_{H} | SEQ ID NO: 137 positions | CDR protein sequence | CDR SEQ ID NO |
|---|---|---|---|
| | | GFTFGDSA | 138 |
| | | IRSKAWGGTT | 139 |
| | | TRDRRCSGLSCYLLDF | 140 |
| F768-100-pt2_A05 V_{L} | | | |
| DIVMTQSPDSLAVSLGERATINCKSSQSVLDSSNNRNYLAWYHQKPGQPPKLLIYWASTRES GVPDRFSGSGSGTDFTLTISSLQAEDVAVYYCQQYYSTPQTFGQGTKLEII (SEQ ID NO: 141) | | | |

| V_{L} | SEQ ID NO: 141 positions | CDR Sequence | CDR SEQ ID NO |
|---|---|---|---|
| | | QSVLDSSNNRNY | 142 |
| | | WAS | 143 |
| | | QQYYSTPQT | 144 |
| A80-102_G5 V_{H} | | | |
| QVQLVQSGAEVKKPGASVKVSCKASGYSFTTNYAMHWVRQAPGQRLEWMGWINVGNDN TKYSQKFQGRVTITRDTSASTVNMELSSLRSEDTAVYYCARSTPFYDRRGFYFLGYFQHWG QGTLVTVSS (SEQ ID NO: 145) | | | |

| V_{H} | SEQ ID NO: 145 positions | CDR protein sequence | CDR SEQ ID NO |
|---|---|---|---|
| | 26-34 | GYSFTTNYA | 146 |
| | 52-59 | INVGNDNT | 147 |
| | 98-117 | ARSTPFYDRRGFYFLGYFQH | 148 |
| A80-102_G5 V_{L} | | | |
| DIQMTQSPSSLSASVGDRVTITCQASQDISNYLNWYQQKPGKAPKVLISDASNLETGVPSRFS GSGSGTDFIFTISSLQLEDVATYYCQQHDNVPLTFGGGTKVEIK (SEQ ID NO: 149) | | | |

| V_{L} | SEQ ID NO: 149 positions | CDR Sequence | CDR SEQ ID NO |
|---|---|---|---|
| | 27-32 | QDISNY | 150 |
| | 50-52 | DAS | 151 |
| | 89-97 | QQHDNVPLTF | 152 |
| A18-338-19.5 V_{H} | | | |
| QVQLVQSGAEVKKPGASVKVSCKASGYIFTSFAIHWVRQAPGHRPEWMGWINPGNGNTQY SQKFQGRVTITRDTSASTANMELSSLGSEDTAVYFCARSTPYYDRTGSYWLGYFQHWGQGT LVSVSS (SEQ ID NO: 153) | | | |

| V_{H} | SEQ ID NO: 153 positions | CDR protein sequence | CDR SEQ ID NO |
|---|---|---|---|
| | 26-34 | GYIFTSFAI | 154 |
| | 51-58 | INPGNGNT | 155 |
| | 97-116 | ARSTPYYDRTGSYWLGYFQH | 156 |
| A18-338-19.5 V_{L} | | | |
| DVQMTQSPSSLSASVGDRVTITCQASQDVTNYLNWYQQKPGQAPKVLISDASDLETGVPSR FSGSGSGTDFTFTISSLQPEDIATYYCQQYGNLPLTFGGGTKVEIK (SEQ ID NO: 157) | | | |

| V_{L} | SEQ ID NO: 157 positions | CDR Sequence | CDR SEQ ID NO |
|---|---|---|---|
| | 27-32 | QDVTNY | 158 |
| | 50-52 | DAS | 159 |
| | 89-97 | QQYGNLPLTF | 160 |
| A18-338-19.6 V_{H} | | | |
| QVQLVQSGAEVKKPGASVKVSCKASGYIFTSFALHWVRQAPGQRPEWMGWINAGNGDTE YSQKFQGRVTITRDTSATTATMELSSLRSEDTAVYFCARSTPYYDRRGYYWLNYFQHWGQ GTLVTVSS (SEQ ID NO: 161) | | | |

| V_{H} | SEQ ID NO: 161 positions | CDR protein sequence | CDR SEQ ID NO |
|---|---|---|---|
| | 26-34 | GYIFTSFAL | 162 |
| | 51-58 | INAGNGDT | 163 |
| | 97-116 | ARSTPYYDRRGYYWLNYFQ H | 164 |
| A18-338-19.6 V_{L} | | | |
| DIQMTQSPSSLSASVGDRVTITCQASQDISNHLNWYQQKPGKAPKVLISDASDLETGVPSRFS GSGSGTDFTFIINSLQPEDFATYYCQQYDNLPLTFGGGTKVETK (SEQ ID NO: 165) | | | |

| V_{L} | SEQ ID NO: 165 positions | CDR Sequence | CDR SEQ ID NO |
|---|---|---|---|
| | 27-32 | QDISNH | 166 |
| | 50-52 | DAS | 167 |
| | 89-97 | QQYDNLPLTF | 168 |
| A18-338-19.8 V_{H} | | | |
| QVQLVQSGAEVKKPGASVKVSCKSSGYTFTSHNFGIHWVRLAPGQRLEWMGWINTGNGNT KYSQKSQGRVTITRDTSASTAYMELSNLNSEDTAVYYCARSTPYYDRSGHYCLGYFQHWG QGTLVSVSS (SEQ ID NO: 169) | | | |

| V_{H} | SEQ ID NO: 169 positions | CDR protein sequence | CDR SEQ ID NO |
|---|---|---|---|
| | 26-35 | GYTFTSHNFG | 170 |
| | 53-60 | INTGNGNT | 171 |
| | 99-118 | ARSTPYYDRSGHYCLGYFQH | 172 |
| A18-338-19.8 V_{L} | | | |
| DIQMTQSPSSLSASVGDRVTITCQASQDINNYLNWYQQKPGEAPKVVIFDATDLETGVPSRF SGSGSGTDFTFTISSLQPEDIATYYCQQDGNLPLTFGGGTKLEIK (SEQ ID NO: 173) | | | |

| V_{L} | SEQ ID NO: 173 positions | CDR Sequence | CDR SEQ ID NO |
|---|---|---|---|
| | 27-32 | QDINNY | 174 |
| | 50-52 | DAT | 175 |
| | 89-97 | QQDGNLPLTF | 176 |
| A18-338-19.9 V_{H} | | | |
| QVQLVQSGAEVKKPGASVKVSCKASGYIFTSFAIHWVRQAPGQRLEWMGWINAGNGNTEY SQKFQGRVTITRDTSASTANMYLSSLRSEDTAVYYCARSTPYYDRTGSYWLGYFQHWGQG TLVSVSS (SEQ ID NO: 177) | | | |

| V_{H} | SEQ ID NO: 177 positions | CDR protein sequence | CDR SEQ ID NO |
|---|---|---|---|
| | 26-34 | GYIFTSFAI | 178 |
| | 51-58 | INAGNGNT | 179 |
| | 97-116 | ARSTPYYDRTGSYWLGYFQH | 180 |
| A18-338-19.9 V_{L} | | | |
| DIQMTQSPSSLSASVGDRVTITCQASQDVSNFLNWYQQKPGEAPKVLISDASSLETGVPSRFS GSGSGTDFTFTISSLQPEDIATYYCQQYGNLPLTFGGGTKVEIK (SEQ ID NO: 181) | | | |

| V_{L} | SEQ ID NO: 181 positions | CDR Sequence | CDR SEQ ID NO |
|---|---|---|---|
| | 27-32 | QDVSNF | 182 |
| | 50-52 | DAS | 183 |
| | 89-97 | QQYGNLPLTF | 184 |

### a. Monoclonal antibody A18-618-448.1

In some aspects, the antibody or antigen binding fragment is based on or derived from the A18-618-448.1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-618-448.1 antibody, and specifically binds to a coronavirus spike protein, and neutralizes a coronavirus. The coronavirus can be SARS-CoV-2. In some aspects, the antibody or antigen binding fragment neutralizes BA.1, BA.2, and BA.4/5, EG.5.1, BA.2.86 JN.1, KP.2, KP.3, LB.1, KP.2.3, KP.3.1.1, and XEC. In other aspects, the antibody or antigen binding fragment neutralizes SARS-CoV related sarbecoviruses from humans, bats, and/or civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 1, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 5, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 1 and 5, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 2, 3, and 4, respectively, and/or a V_{L} comprising a LCDR1, a LCDR2, and a LCDR3 as set forth as SEQ ID NOs: 6, 7 (AAS), and 8, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 2, 3, and 4, respectively, a V_{L} comprising a LCDR1, a LCDR2, and a LCDR3 as set forth as SEQ ID NOs: 6, 7 (AAS), and 8 respectively, wherein the V_{H} comprises an amino acid sequence at least 90% identical to SEQ ID NO: 1, such as 95%, 96%, 97%, 98% or 99% identical to SEQ ID NO: 1, and wherein the V_{L} comprises an amino acid sequence at least 90% identical to SEQ ID NO: 5, such as 95%, 96%, 97%, 98% or 99% identical to SEQ ID NO: 5, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 1, and specifically binds to a coronavirus spike protein, and neutralizes a coronavirus. In more aspects, the antibody or antigen binding fragment comprises a V_{L} comprising the amino acid sequence set forth as SEQ ID NO: 5, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 1 and 5, respectively, and specifically binds to a coronavirus spike protein, and neutralizes a coronavirus. The coronavirus can be SARS-CoV-2.

In some aspects, the disclosed antibodies inhibit viral entry and/or replication.

### b. Monoclonal antibody F768-104_B2

In some aspects, the antibody or antigen binding fragment is based on or derived from the F768-104_B2 antibody, and specifically binds to a coronavirus spike protein, and neutralizes a coronavirus. In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the F768-104_B2 antibody, and specifically binds to a coronavirus spike protein, and neutralizes a coronavirus. The coronavirus can be SARS-CoV-2.

In some aspects, the antibody or antigen binding fragment neutralizes an animal-derived SARS-CoV-2 related virus. In some aspects, the antibody or antigen binding fragment specifically binds a spike protein from BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1, and XEC.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 9, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 13, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 9 and 13, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 10, 11, and 12, respectively, and/or a V_{L} comprising a LCDR1, a LCDR2, and a LCDR3 as set forth as SEQ ID NOs: 14, 15 (GAS), and 16, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 10, 11, and 12, respectively, a V_{L} comprising a LCDR1, a LCDR2, and a LCDR3 as set forth as SEQ ID NOs: 14, 15 (GAS), and 16, respectively, wherein the V_{H} comprises an amino acid sequence at least 90% identical to SEQ ID NO: 9, such as 95%, 96%, 97%, 98% or 99% identical to SEQ ID NO: 9, and wherein the V_{L} comprises an amino acid sequence at least 90% identical to SEQ ID NO: 13, such as 95%, 96%, 97%, 98% or 99% identical to SEQ ID NO: 13, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 9, and specifically binds to a coronavirus spike protein, and neutralizes a coronavirus. In more aspects, the antibody or antigen binding fragment comprises a V_{L} comprising the amino acid sequence set forth as SEQ ID NO: 13, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 9 and 13, respectively, and specifically binds to a coronavirus spike protein, and neutralizes a coronavirus. The coronavirus can be SARS-CoV-2.

In some aspects, the disclosed antibodies inhibit viral entry and/or replication.

### c. Monoclonal antibody E184-105_F3

In some aspects, the antibody or antigen binding fragment is based on or derived from the E184-105_F3 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the E184-105_F3 antibody, and specifically binds to a coronavirus spike protein, and neutralizes a coronavirus. The coronavirus can be SARS-CoV-2.

In some aspects, the antibody or antigen binding fragment has neutralizing activity against Omicron lineage pseudotyped viruses. In more aspects, the antibody or antigen binding fragment has neutralizing activity against BA.1, BA.2, BA.4/5, BA.2.75.2, BQ1.1, XBB, EG.5.1 BA.2.86, JN.1, KP.2, KP.3, LB.1, KP.2.3. In more aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 17, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 21, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 17 and 21, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 18, 19, and 20, respectively, and/or a V_{L} comprising a LCDR1, a LCDR2, and a LCDR3 as set forth as SEQ ID NOs: 22, 23 (GAS), and 24, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 18, 19, and 20, respectively, a V_{L} comprising a LCDR1, a LCDR2, and a LCDR3 as set forth as SEQ ID NOs: 22, 23 (GAS), and 24, respectively, wherein the V_{H} comprises an amino acid sequence at least 90% identical to SEQ ID NO: 17, such as 95%, 96%, 97%, 98% or 99% identical to SEQ ID NO: 17, and wherein the V_{L} comprises an amino acid sequence at least 90% identical to SEQ ID NO: 21, such as 95%, 96%, 97%, 98% or 99% identical to SEQ ID NO: 21, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 17, and specifically binds to a coronavirus spike protein, and neutralizes a coronavirus. In more aspects, the antibody or antigen binding fragment comprises a V_{L} comprising the amino acid sequence set forth as SEQ ID NO: 21, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 17 and 21, respectively, and specifically binds to a coronavirus spike protein, and neutralizes a coronavirus. The coronavirus can be SARS-CoV-2.

In some aspects, the disclosed antibodies inhibit viral entry and/or replication.

### d. Monoclonal antibody A63-652-32.1

In some aspects, the antibody or antigen binding fragment is based on or derived from the A63-652-32.1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A63-652-32.1 antibody, and specifically binds to a coronavirus spike protein, and neutralizes a coronavirus. The coronavirus can be SARS-CoV-2.

In some aspects, the antibody or antigen binding fragment has neutralizing activity against Omicron lineage pseudotyped viruses. In more aspects, the antibody or antigen binding fragment has neutralizing activity against BA.1, BA.2, BA.4/5, BA.2.75.2, BQ1.1, XBB, EG.5.1 and BA.2.86. In more aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 25, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 29, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 25 and 29, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 26, 27, and 28, respectively, and/or a V_{L} comprising a LCDR1, a LCDR2, and a LCDR3 as set forth as SEQ ID NOs: 30, 31 (AAS), and 32, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 26, 27, and 28, respectively, a V_{L} comprising a LCDR1, a LCDR2, and a LCDR3 as set forth as SEQ ID NOs: 30, 31 (AAS), and 32, respectively, wherein the V_{H} comprises an amino acid sequence at least 90% identical to SEQ ID NO: 25, such as 95%, 96%, 97%, 98% or 99% identical to SEQ ID NO: 25, and wherein the V_{L} comprises an amino acid sequence at least 90% identical to SEQ ID NO: 29, such as 95%, 96%, 97%, 98% or 99% identical to SEQ ID NO: 29, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 25, and specifically binds to a coronavirus spike protein, and neutralizes a coronavirus. In more aspects, the antibody or antigen binding fragment comprises a V_{L} comprising the amino acid sequence set forth as SEQ ID NO: 29, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 25 and 29, respectively, and specifically binds to a coronavirus spike protein, and neutralizes a coronavirus. The coronavirus can be SARS-CoV-2.

In some aspects, the disclosed antibodies inhibit viral entry and/or replication.

### e. Monoclonal antibody F768-104_G7

In some aspects, the antibody or antigen binding fragment is based on or derived from the F768-104_G7 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the F768-104_G7 antibody, and specifically binds to a coronavirus spike protein, and neutralizes a coronavirus. The coronavirus can be SARS-CoV-2.

In some aspects, the antibody or antigen binding fragment has neutralizing activity against Omicron lineage pseudotyped viruses. In more aspects, the antibody or antigen binding fragment has neutralizing activity against BA.1, BA.2, BA.4/5, BA.2.75.2, BQ1.1, XBB, EG.5.1 and BA.2.86. In more aspects, the antibody or antigen binding fragment neutralizes SARS-CoV-2 related coronaviruses from bats and pangolins. In further aspects, the monoclonal antibody or antigen binding fragment neutralizes the D614G strain bearing the F456L mutation.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 33, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 37, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 33 and 37, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 34, 35, and 36, respectively, and/or a V_{L} comprising a LCDR1, a LCDR2, and a LCDR3 as set forth as SEQ ID NOs: 38, 39 (AAS), and 40, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 34, 35, and 36, respectively, a V_{L} comprising a LCDR1, a LCDR2, and a LCDR3 as set forth as SEQ ID NOs: 38, 39 (AAS), and 40, respectively, wherein the V_{H} comprises an amino acid sequence at least 90% identical to SEQ ID NO: 33, such as 95%, 96%, 97%, 98% or 99% identical to SEQ ID NO: 33, and wherein the V_{L} comprises an amino acid sequence at least 90% identical to SEQ ID NO: 37, such as 95%, 96%, 97%, 98% or 99% identical to SEQ ID NO: 37, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 33, and specifically binds to a coronavirus spike protein, and neutralizes a coronavirus. In more aspects, the antibody or antigen binding fragment comprises a V_{L} comprising the amino acid sequence set forth as SEQ ID NO: 37, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 33 and 37, respectively, and specifically binds to a coronavirus spike protein, and neutralizes a coronavirus. The coronavirus can be SARS-CoV-2.

In some aspects, the disclosed antibodies inhibit viral entry and/or replication.

### f. Monoclonal antibody E184-105_H1

In some aspects, the antibody or antigen binding fragment is based on or derived from the E184-105_H1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the E184-105_H1 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, XBB, EG.5.1, BA.2.86 and JN.1. In further aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 41, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 45, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 41 and 45, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 42, 43, and 44, respectively, and/or a V_{L} comprising a LCDR1, a LCDR2, and a LCDR3 as set forth as SEQ ID NOs: 46, 47 (DAS), and 48, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 42, 43, and 44, respectively, a V_{L} comprising a LCDR1, a LCDR2, and a LCDR3 as set forth as SEQ ID NOs: 46, 47 (DAS), and 48, respectively, wherein the V_{H} comprises an amino acid sequence at least 90% identical to SEQ ID NO: 41, such as 95%, 96%, 97%, 98% or 99% identical to SEQ ID NO: 41, and wherein the V_{L} comprises an amino acid sequence at least 90% identical to SEQ ID NO: 45, such as 95%, 96%, 97%, 98% or 99% identical to SEQ ID NO: 45, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 41, and specifically binds to a coronavirus spike protein, and neutralizes a coronavirus. In more aspects, the antibody or antigen binding fragment comprises a V_{L} comprising the amino acid sequence set forth as SEQ ID NO: 45, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 41 and 45, respectively, and specifically binds to a coronavirus spike protein, and neutralizes a coronavirus. The coronavirus can be SARS-CoV-2.

In some aspects, the disclosed antibodies inhibit viral entry and/or replication.

### g. Monoclonal antibody F768-104_D10

In some aspects, the antibody or antigen binding fragment is based on or derived from the F768-104_D10 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the F768-104_D10 antibody, and specifically binds to a coronavirus spike protein, and neutralizes a coronavirus. The coronavirus can be SARS-CoV-2.

In some aspects, the antibody or antigen binding fragment has neutralizing activity against Omicron lineage pseudotyped viruses. In more aspects, the antibody or antigen binding fragment has neutralizing activity against BA.1, BA.2, BA.4/5, BA.2.75.2, BQ1.1, XBB, EG.5.1, BA.2.86 and JN.1. In more aspects, the antibody or antigen binding fragment neutralizes SARS-CoV-2 related coronaviruses from bats and pangolins. In further aspects, the monoclonal antibody or antigen binding fragment neutralizes the D614G strain bearing the F456L mutation.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 49, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 53, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 49 and 53, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 50, 51, and 52, respectively, and/or a V_{L} comprising a LCDR1, a LCDR2, and a LCDR3 as set forth as SEQ ID NOs: 54, 55 (GAS), and 56, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 50, 51, and 52, respectively, a V_{L} comprising a LCDR1, a LCDR2, and a LCDR3 as set forth as SEQ ID NOs: 54, 55 (GAS), and 56, respectively, wherein the V_{H} comprises an amino acid sequence at least 90% identical to SEQ ID NO: 49, such as 95%, 96%, 97%, 98% or 99% identical to SEQ ID NO: 49, and wherein the V_{L} comprises an amino acid sequence at least 90% identical to SEQ ID NO: 53, such as 95%, 96%, 97%, 98% or 99% identical to SEQ ID NO: 53, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 49, and specifically binds to a coronavirus spike protein, and neutralizes a coronavirus. In more aspects, the antibody or antigen binding fragment comprises a V_{L} comprising the amino acid sequence set forth as SEQ ID NO: 53, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 49 and 53, respectively, and specifically binds to a coronavirus spike protein, and neutralizes a coronavirus. The coronavirus can be SARS-CoV-2.

In some aspects, the disclosed antibodies inhibit viral entry and/or replication.

### h. Monoclonal antibody A63-652-1.4

In some aspects, the antibody or antigen binding fragment is based on or derived from the A63-652-1.4 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A63-652-1.4 antibody, and specifically binds to a coronavirus spike protein, and neutralizes a coronavirus. The coronavirus can be SARS-CoV-2.

In some aspects, the antibody or antigen binding fragment has neutralizing activity against Omicron lineage pseudotyped viruses. In more aspects, the antibody or antigen binding fragment has neutralizing activity against BA.1, BA.2, BA.4/5, BA.2.75.2, BQ1.1, XBB, EG.5.1,BA.2.86, JN.1, KP.2, KP.3, LB.1, KP.2.3, KP.3.1.1 and XEC. In more aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 57, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 61, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 57 and 61, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 58, 59, and 60, respectively, and/or a V_{L} comprising a LCDR1, a LCDR2, and a LCDR3 as set forth as SEQ ID NOs: 62, 63 (DAS), and 64, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 58, 59, and 60, respectively, a V_{L} comprising a LCDR1, a LCDR2, and a LCDR3 as set forth as SEQ ID NOs: 62, 63 (DAS), and 64, respectively, wherein the V_{H} comprises an amino acid sequence at least 90% identical to SEQ ID NO: 57, such as 95%, 96%, 97%, 98% or 99% identical to SEQ ID NO: 57, and wherein the V_{L} comprises an amino acid sequence at least 90% identical to SEQ ID NO: 61, such as 95%, 96%, 97%, 98% or 99% identical to SEQ ID NO: 61, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 57, and specifically binds to a coronavirus spike protein, and neutralizes a coronavirus. In more aspects, the antibody or antigen binding fragment comprises a V_{L} comprising the amino acid sequence set forth as SEQ ID NO: 61, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 57 and 61, respectively, and specifically binds to a coronavirus spike protein, and neutralizes a coronavirus. The coronavirus can be SARS-CoV-2.

In some aspects, the disclosed antibodies inhibit viral entry and/or replication.

### i. Monoclonal antibody E184-105_E5

In some aspects, the antibody or antigen binding fragment is based on or derived from the E184-105_E5 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the E184-105_E5 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, XBB, EG.5.1,BA.2.86 and JN.1. In further aspects, the antibody or antigen binding fragment neutralizes SARS-CoV-2 related coronaviruses from bats and pangolins. In further aspects, the monoclonal antibody or antigen binding fragment neutralizes the D614G strain bearing the F456L mutation.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 65, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 69, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 65 and 69, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 66, 67, and 68, respectively, and/or a V_{L} comprising a LCDR1, a LCDR2, and a LCDR3 as set forth as SEQ ID NOs: 70, 71 (AAS), and 72, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 66, 67, and 68, respectively, a V_{L} comprising a LCDR1, a LCDR2, and a LCDR3 as set forth as SEQ ID NOs: 70, 71 (AAS), and 72, respectively, wherein the V_{H} comprises an amino acid sequence at least 90% identical to SEQ ID NO: 65, such as 95%, 96%, 97%, 98% or 99% identical to SEQ ID NO: 65, and wherein the V_{L} comprises an amino acid sequence at least 90% identical to SEQ ID NO: 69, such as 95%, 96%, 97%, 98% or 99% identical to SEQ ID NO: 69, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 65, and specifically binds to a coronavirus spike protein, and neutralizes a coronavirus. In more aspects, the antibody or antigen binding fragment comprises a V_{L} comprising the amino acid sequence set forth as SEQ ID NO: 69, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 65 and 69, respectively, and specifically binds to a coronavirus spike protein, and neutralizes a coronavirus. The coronavirus can be SARS-CoV-2.

In some aspects, the disclosed antibodies inhibit viral entry and/or replication.

### j. Monoclonal antibody E184-105_H9

In some aspects, the antibody or antigen binding fragment is based on or derived from the E184-105_H9 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the E184-105_H9 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, XBB, EG.5.1, BA.2.86, and JN.1. In further aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 73, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 77, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 73 and 77, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 74, 75, and 76, respectively, and/or a V_{L} comprising a LCDR1, a LCDR2, and a LCDR3 as set forth as SEQ ID NOs: 78, 79 (AAS), and 80, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 74, 75, and 76, respectively, a V_{L} comprising a LCDR1, a LCDR2, and a LCDR3 as set forth as SEQ ID NOs: 78, 79 (AAS), and 80, respectively, wherein the V_{H} comprises an amino acid sequence at least 90% identical to SEQ ID NO: 73, such as 95%, 96%, 97%, 98% or 99% identical to SEQ ID NO: 73, and wherein the V_{L} comprises an amino acid sequence at least 90% identical to SEQ ID NO: 77, such as 95%, 96%, 97%, 98% or 99% identical to SEQ ID NO: 77, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 73 and specifically binds to a coronavirus spike protein, and neutralizes a coronavirus. In more aspects, the antibody or antigen binding fragment comprises a V_{L} comprising the amino acid sequence set forth as SEQ ID NO: 77, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 73 and 77, respectively, and specifically binds to a coronavirus spike protein, and neutralizes a coronavirus. The coronavirus can be SARS-CoV-2.

In some aspects, the disclosed antibodies inhibit viral entry and/or replication.

### k. Monoclonal antibody E184-105_H2

In some aspects, the antibody or antigen binding fragment is based on or derived from the E184-105_H2 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the E184-105_H2 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, XBB, EG.5.1 and BA.2.86. In further aspects, the antibody or antigen binding fragment neutralizes SARS-CoV-2 related coronaviruses from bats and pangolins.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 81, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 85, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 81 and 85, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 82, 83, and 84, respectively, and/or a V_{L} comprising a LCDR1, a LCDR2, and a LCDR3 as set forth as SEQ ID NOs: 86, 87 (AAS), and 88, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 82, 83, and 84, respectively, a V_{L} comprising a LCDR1, a LCDR2, and a LCDR3 as set forth as SEQ ID NOs: 86, 87 (AAS), and 88, respectively, wherein the V_{H} comprises an amino acid sequence at least 90% identical to SEQ ID NO: 81, such as 95%, 96%, 97%, 98% or 99% identical to SEQ ID NO: 81, and wherein the V_{L} comprises an amino acid sequence at least 90% identical to SEQ ID NO: 85, such as 95%, 96%, 97%, 98% or 99% identical to SEQ ID NO: 85, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 81, and specifically binds to a coronavirus spike protein, and neutralizes a coronavirus. In more aspects, the antibody or antigen binding fragment comprises a V_{L} comprising the amino acid sequence set forth as SEQ ID NO: 85, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 81 and 85, respectively, and specifically binds to a coronavirus spike protein, and neutralizes a coronavirus. The coronavirus can be SARS-CoV-2.

In some aspects, the disclosed antibodies inhibit viral entry and/or replication.

### l. Monoclonal antibody A80-102+103_G1

In some aspects, the antibody or antigen binding fragment is based on or derived from the A80-102+103_G1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A80-102+103_G1 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, XBB, EG.5.1 and BA.2.86. In further aspects, the antibody or antigen binding fragment neutralizes SARS-CoV-2 related coronaviruses from bats and pangolins. In further aspects, the monoclonal antibody or antigen binding fragment neutralizes the D614G strain bearing the F456L mutation.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 89, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 93, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 89 and 93, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 90, 91, and 92, respectively, and/or a V_{L} comprising a LCDR1, a LCDR2, and a LCDR3 as set forth as SEQ ID NOs: 94, 95 (NNN), and 96, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 90, 91, and 92, respectively, a V_{L} comprising a LCDR1, a LCDR2, and a LCDR3 as set forth as SEQ ID NOs: 94, 95 (NNN), and 96, respectively, wherein the V_{H} comprises an amino acid sequence at least 90% identical to SEQ ID NO: 89, such as 95%, 96%, 97%, 98% or 99% identical to SEQ ID NO: 89, and wherein the V_{L} comprises an amino acid sequence at least 90% identical to SEQ ID NO: 93, such as 95%, 96%, 97%, 98% or 99% identical to SEQ ID NO: 93, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 89, and specifically binds to a coronavirus spike protein, and neutralizes a coronavirus. In more aspects, the antibody or antigen binding fragment comprises a V_{L} comprising the amino acid sequence set forth as SEQ ID NO: 93, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 89 and 93, respectively, and specifically binds to a coronavirus spike protein, and neutralizes a coronavirus. The coronavirus can be SARS-CoV-2.

In some aspects, the disclosed antibodies inhibit viral entry and/or replication.

### m. Monoclonal antibody A80-103_E2

In some aspects, the antibody or antigen binding fragment is based on or derived from the A80-103_E2 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A80-103_E2 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, BA.2.86, JN.1, KP.2, KP.3, LB.1, KP.2.3, KP.3.1.1 and XEC. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 97, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 101, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 97 and 101, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 98, 99, and 100, respectively, and/or a V_{L} comprising a LCDR1, a LCDR2, and a LCDR3 as set forth as SEQ ID NOs: 102, 103 (SDS), and 104, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 98, 99, and 100, respectively, a V_{L} comprising a LCDR1, a LCDR2, and a LCDR3 as set forth as SEQ ID NOs: 102, 103 (SDS), and 104, respectively, wherein the V_{H} comprises an amino acid sequence at least 90% identical to SEQ ID NO: 97, such as 95%, 96%, 97%, 98% or 99% identical to SEQ ID NO: 97, and wherein the V_{L} comprises an amino acid sequence at least 90% identical to SEQ ID NO: 101, such as 95%, 96%, 97%, 98% or 99% identical to SEQ ID NO: 101, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 97, and specifically binds to a coronavirus spike protein, and neutralizes a coronavirus. In more aspects, the antibody or antigen binding fragment comprises a V_{L} comprising the amino acid sequence set forth as SEQ ID NO: 101, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 97 and 101, respectively, and specifically binds to a coronavirus spike protein, and neutralizes a coronavirus. The coronavirus can be SARS-CoV-2.

In some aspects, the disclosed antibodies inhibit viral entry and/or replication.

### n. Monoclonal antibody A80-103_B6

In some aspects, the antibody or antigen binding fragment is based on or derived from the A80-103_B6 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A80-103_B6 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, BA.2.86, JN.1, KP.2, KP.3, LB.1, KP.2.3, KP.3.1.1 and XEC. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 105, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 109, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 105 and 109, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 106, 107, and 108, respectively, and/or a V_{L} comprising a LCDR1, a LCDR2, and a LCDR3 as set forth as SEQ ID NOs: 110, 111 (SYN), and 112, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 106, 107, and 108, respectively, a V_{L} comprising a LCDR1, a LCDR2, and a LCDR3 as set forth as SEQ ID NOs: 110, 111 (SYN), and 112, respectively, wherein the V_{H} comprises an amino acid sequence at least 90% identical to SEQ ID NO: 105, such as 95%, 96%, 97%, 98% or 99% identical to SEQ ID NO: 105, and wherein the V_{L} comprises an amino acid sequence at least 90% identical to SEQ ID NO: 109, such as 95%, 96%, 97%, 98% or 99% identical to SEQ ID NO: 109, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 105, and specifically binds to a coronavirus spike protein, and neutralizes a coronavirus. In more aspects, the antibody or antigen binding fragment comprises a V_{L} comprising the amino acid sequence set forth as SEQ ID NO: 109, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 105 and 109, respectively, and specifically binds to a coronavirus spike protein, and neutralizes a coronavirus. The coronavirus can be SARS-CoV-2.

In some aspects, the disclosed antibodies inhibit viral entry and/or replication.

### o. Monoclonal antibody A18-618-452.1

In some aspects, the antibody or antigen binding fragment is based on or derived from the A18-618-452.1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-618-452.1 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, BA.2.86, JN.1, KP.2, KP.3, LB.1, KP.2.3, KP.3.1.1 and XEC. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 113, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 117, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 113 and 117, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 114, 115, and 116, respectively, and/or a V_{L} comprising a LCDR1, a LCDR2, and a LCDR3 as set forth as SEQ ID NOs: 118, 119 (AAS), and 120, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 114, 115, and 116, respectively, a V_{L} comprising a LCDR1, a LCDR2, and a LCDR3 as set forth as SEQ ID NOs: 118, 119 (AAS), and 120, respectively, wherein the V_{H} comprises an amino acid sequence at least 90% identical to SEQ ID NO: 113, such as 95%, 96%, 97%, 98% or 99% identical to SEQ ID NO: 113, and wherein the V_{L} comprises an amino acid sequence at least 90% identical to SEQ ID NO: 117, such as 95%, 96%, 97%, 98% or 99% identical to SEQ ID NO: 117, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 113, and specifically binds to a coronavirus spike protein, and neutralizes a coronavirus. In more aspects, the antibody or antigen binding fragment comprises a V_{L} comprising the amino acid sequence set forth as SEQ ID NO: 117, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 113 and 117, respectively, and specifically binds to a coronavirus spike protein, and neutralizes a coronavirus. The coronavirus can be SARS-CoV-2.

In some aspects, the disclosed antibodies inhibit viral entry and/or replication.

### p. Monoclonal antibody A80-102+103_C1

In some aspects, the antibody or antigen binding fragment is based on or derived from the A80-102+103_C1 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A80-102+103_C1 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, BA.2.86, IN.1, KP.2, KP.3, LB.1, KP.2.3, KP.3.1.1 and XEC. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 121, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 125, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 121 and 125, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 122, 123, and 124, respectively, and/or a V_{L} comprising a LCDR1, a LCDR2, and a LCDR3 as set forth as SEQ ID NOs: 126, 127 (DAS), and 128, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 122, 123, and 124, respectively, a V_{L} comprising a LCDR1, a LCDR2, and a LCDR3 as set forth as SEQ ID NOs: 126, 127 (DAS), and 128, respectively, wherein the V_{H} comprises an amino acid sequence at least 90% identical to SEQ ID NO: 121, such as 95%, 96%, 97%, 98% or 99% identical to SEQ ID NO: 121, and wherein the V_{L} comprises an amino acid sequence at least 90% identical to SEQ ID NO: 125, such as 95%, 96%, 97%, 98% or 99% identical to SEQ ID NO: 125, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 121, and specifically binds to a coronavirus spike protein, and neutralizes a coronavirus. In more aspects, the antibody or antigen binding fragment comprises a V_{L} comprising the amino acid sequence set forth as SEQ ID NO: 125, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 121 and 125, respectively, and specifically binds to a coronavirus spike protein, and neutralizes a coronavirus. The coronavirus can be SARS-CoV-2.

In some aspects, the disclosed antibodies inhibit viral entry and/or replication.

### q. Monoclonal antibody A43-d321.10.4

In some aspects, the antibody or antigen binding fragment is based on or derived from the A43-d321.10.4 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A43-d321.10.4 antibody, and specifically binds to a coronavirus spike protein, and neutralizes a coronavirus.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, BA.2.86, JN.1, KP.2, KP.3, LB.1, KP.2.3, KP.3.1.1 and XEC. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 129, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 133, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 129 and 133, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 130, 131, and 132, respectively, and/or a V_{L} comprising a LCDR1, a LCDR2, and a LCDR3 as set forth as SEQ ID NOs: 134, 135 (DAS), and 136, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 130, 131, and 132, respectively, a V_{L} comprising a LCDR1, a LCDR2, and a LCDR3 as set forth as SEQ ID NOs: 134, 135 (DAS), and 136, respectively, wherein the V_{H} comprises an amino acid sequence at least 90% identical to SEQ ID NO: 129, such as 95%, 96%, 97%, 98% or 99% identical to SEQ ID NO: 129, and wherein the V_{L} comprises an amino acid sequence at least 90% identical to SEQ ID NO: 133, such as 95%, 96%, 97%, 98% or 99% identical to SEQ ID NO: 133, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 129, and specifically binds to a coronavirus spike protein, and neutralizes a coronavirus. In more aspects, the antibody or antigen binding fragment comprises a V_{L} comprising the amino acid sequence set forth as SEQ ID NO: 133, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 129 and 133, respectively, and specifically binds to a coronavirus spike protein, and neutralizes a coronavirus. The coronavirus can be SARS-CoV-2.

In some aspects, the disclosed antibodies inhibit viral entry and/or replication.

### r. Monoclonal antibody F768-100-pt2_A05

In some aspects, the antibody or antigen binding fragment is based on or derived from the F768-100-pt2_A05 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the F768-100-pt2_A05 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 Omicron lineage pseudotyped viruses. In more aspects, the antibody or antigen binding fragment has neutralizing activity against BA.1, BA.2, BA.4/5, BA.2.75.2, BQ1.1, and XBB. In other aspects, the antibody or antigen binding fragment has neutralizing activity against BA EG.5.1, FL.1.5.1, and BA.2.86. In further aspects, the antibody or antigen binding fragment neutralizes SARS-CoV-2-related coronaviruses from animals, as well as SARS-CoV.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 137, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 141, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 137 and 141, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 138, 139, and 140, respectively, and/or a V_{L} comprising a LCDR1, a LCDR2, and a LCDR3 as set forth as SEQ ID NOs: 142, 143 (WAS), and 144, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 138, 139, and 140, respectively, a V_{L} comprising a LCDR1, a LCDR2, and a LCDR3 as set forth as SEQ ID NOs: 142, 143 (WAS), and 144, respectively, wherein the V_{H} comprises an amino acid sequence at least 90% identical to SEQ ID NO: 137, such as 95%, 96%, 97%, 98% or 99% identical to SEQ ID NO: 137, and wherein the V_{L} comprises an amino acid sequence at least 90% identical to SEQ ID NO: 141, such as 95%, 96%, 97%, 98% or 99% identical to SEQ ID NO: 141, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 137, and specifically binds to a coronavirus spike protein, and neutralizes a coronavirus. In more aspects, the antibody or antigen binding fragment comprises a V_{L} comprising the amino acid sequence set forth as SEQ ID NO: 141, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 137 and 141, respectively, and specifically binds to a coronavirus spike protein, and neutralizes a coronavirus. The coronavirus can be SARS-CoV-2.

In some aspects, the disclosed antibodies inhibit viral entry and/or replication.

### s. Monoclonal A80-102_G5

In some aspects, the antibody or antigen binding fragment is based on or derived from the A80-102_G5 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A80-102_G5 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 variants and/or SARS-CoV-related sarbecoviruses. In more aspects, the antibody or antigen binding fragment has neutralizing activity against omicron BA.1, BA.2, BA.5, EG.5.1,BA.2.86, JN.1, KP.2, KP.3, LB.1, KP.2.3, KP.3.1.1, and XEC. In further aspects, the antibody or antigen binding fragment has neutralizing activity against SARS-CoV-2 related viruses from animals, including Pangolin GX variants, as well as SARS-CoV related sarbecoviruses from humans, bats, and civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 145, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 149, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 145 and 149, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 146, 147, and 148, respectively, and/or a V_{L} comprising a LCDR1, a LCDR2, and a LCDR3 as set forth as SEQ ID NOs: 150, 151 (DAS), and 152, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 146, 147, and 148, respectively, a V_{L} comprising a LCDR1, a LCDR2, and a LCDR3 as set forth as SEQ ID NOs: 150, 151 (DAS), and 152, respectively, wherein the V_{H} comprises an amino acid sequence at least 90% identical to SEQ ID NO: 145, such as 95%, 96%, 97%, 98% or 99% identical to SEQ ID NO: 145, and wherein the V_{L} comprises an amino acid sequence at least 90% identical to SEQ ID NO: 149, such as 95%, 96%, 97%, 98% or 99% identical to SEQ ID NO: 149, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 145, and specifically binds to a coronavirus spike protein, and neutralizes a coronavirus. In more aspects, the antibody or antigen binding fragment comprises a V_{L} comprising the amino acid sequence set forth as SEQ ID NO: 149, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 145 and 149, respectively, and specifically binds to a coronavirus spike protein, and neutralizes a coronavirus. The coronavirus can be SARS-CoV-2.

In some aspects, the disclosed antibodies inhibit viral entry and/or replication.

### t. Monoclonal antibody A18-338-19.5

In some aspects, the antibody or antigen binding fragment is based on or derived from the A18-338-19.5 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-338-19.5 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 variants and/or SARS-CoV-related sarbecoviruses. In more aspects, the antibody or antigen binding fragment has neutralizing activity against omicron BA.1, BA.2, BA.5, EG.5.1 and BA.2.86. In further aspects, the antibody or antigen binding fragment has neutralizing activity against SARS-CoV-2 related viruses from animals, including Pangolin GX variants, as well as SARS-CoV related sarbecoviruses from humans, bats, and civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 153, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 157, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 153 and 157, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 154, 155, and 156, respectively, and/or a V_{L} comprising a LCDR1, a LCDR2, and a LCDR3 as set forth as SEQ ID NOs: 158, 159 (DAS), and 160, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 154, 155, and 156, respectively, a V_{L} comprising a LCDR1, a LCDR2, and a LCDR3 as set forth as SEQ ID NOs: 158, 159 (DAS), and 160, respectively, wherein the V_{H} comprises an amino acid sequence at least 90% identical to SEQ ID NO: 153, such as 95%, 96%, 97%, 98% or 99% identical to SEQ ID NO: 153, and wherein the V_{L} comprises an amino acid sequence at least 90% identical to SEQ ID NO: 157, such as 95%, 96%, 97%, 98% or 99% identical to SEQ ID NO: 157, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 153, and specifically binds to a coronavirus spike protein, and neutralizes a coronavirus. In more aspects, the antibody or antigen binding fragment comprises a V_{L} comprising the amino acid sequence set forth as SEQ ID NO: 157, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 153 and 157, respectively, and specifically binds to a coronavirus spike protein, and neutralizes a coronavirus. The coronavirus can be SARS-CoV-2.

In some aspects, the disclosed antibodies inhibit viral entry and/or replication.

### u. Monoclonal antibody A18-338-19.6

In some aspects, the antibody or antigen binding fragment is based on or derived from the A18-338-19.6 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-338-19.6 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 variants and/or SARS-CoV-related sarbecoviruses. In more aspects, the antibody or antigen binding fragment has neutralizing activity against omicron BA.1, BA.2, BA.5, EG.5.1 and BA.2.86. In further aspects, the antibody or antigen binding fragment has neutralizing activity against SARS-CoV-2 related viruses from animals, including Pangolin GX variants, as well as SARS-CoV related sarbecoviruses from humans, bats, and civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 161, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 165, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 161 and 165, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 162, 163, and 164, respectively, and/or a V_{L} comprising a LCDR1, a LCDR2, and a LCDR3 as set forth as SEQ ID NOs: 166, 167 (DAS), and 168, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 162, 163, and 164, respectively, a V_{L} comprising a LCDR1, a LCDR2, and a LCDR3 as set forth as SEQ ID NOs: 166, 167 (DAS), and 168, respectively, wherein the V_{H} comprises an amino acid sequence at least 90% identical to SEQ ID NO: 161, such as 95%, 96%, 97%, 98% or 99% identical to SEQ ID NO: 161, and wherein the V_{L} comprises an amino acid sequence at least 90% identical to SEQ ID NO: 165, such as 95%, 96%, 97%, 98% or 99% identical to SEQ ID NO: 165, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 161, and specifically binds to a coronavirus spike protein, and neutralizes a coronavirus. In more aspects, the antibody or antigen binding fragment comprises a V_{L} comprising the amino acid sequence set forth as SEQ ID NO: 165, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 161 and 165, respectively, and specifically binds to a coronavirus spike protein, and neutralizes a coronavirus. The coronavirus can be SARS-CoV-2.

In some aspects, the disclosed antibodies inhibit viral entry and/or replication.

### v. Monoclonal antibody A18-338-19.8

In some aspects, the antibody or antigen binding fragment is based on or derived from the A18-338-19.8 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-338-19.8 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 variants and/or SARS-CoV-related sarbecoviruses. In more aspects, the antibody or antigen binding fragment has neutralizing activity against omicron BA.1, BA.2, BA.5, EG.5.1,BA.2.86, JN.1, KP.2, KP.3, LB.1, KP.2.3, KP.3.1.1, and XEC. In further aspects, the antibody or antigen binding fragment has neutralizing activity against SARS-CoV-2 related viruses from animals, including Pangolin GX variants, as well as SARS-CoV related sarbecoviruses from humans, bats, and civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 169, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 173, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 169 and 173, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 170, 171, and 172, respectively, and/or a V_{L} comprising a LCDR1, a LCDR2, and a LCDR3 as set forth as SEQ ID NOs: 174, 175 (DAT), and 176, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 170, 171, and 172, respectively, a V_{L} comprising a LCDR1, a LCDR2, and a LCDR3 as set forth as SEQ ID NOs: 174, 175 (DAT), and 176, respectively, wherein the V_{H} comprises an amino acid sequence at least 90% identical to SEQ ID NO: 169, such as 95%, 96%, 97%, 98% or 99% identical to SEQ ID NO: 169, and wherein the V_{L} comprises an amino acid sequence at least 90% identical to SEQ ID NO: 173, such as 95%, 96%, 97%, 98% or 99% identical to SEQ ID NO: 173, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 169, and specifically binds to a coronavirus spike protein, and neutralizes a coronavirus. In more aspects, the antibody or antigen binding fragment comprises a V_{L} comprising the amino acid sequence set forth as SEQ ID NO: 173, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 169 and 173, respectively, and specifically binds to a coronavirus spike protein, and neutralizes a coronavirus. The coronavirus can be SARS-CoV-2.

In some aspects, the disclosed antibodies inhibit viral entry and/or replication.

### w. Monoclonal antibody A18-338-19.9

In some aspects, the antibody or antigen binding fragment is based on or derived from the A18-338-19.9 antibody, and specifically binds to a coronavirus spike protein, and neutralizes a coronavirus.

In some examples, the antibody or antigen binding fragment comprises a V_{H} and a V_{L} comprising the HCDR1, the HCDR2, the HCDR3, the LCDR1, the LCDR2, and the LCDR3, respectively (for example, according to IMGT, Kabat or Chothia), of the A18-338-19.9 antibody, and specifically binds to a coronavirus spike protein, and neutralizes a coronavirus. The coronavirus can be SARS-CoV-2.

In some aspects, the antibody or antigen binding fragment has neutralizing activity against SARS-CoV-2 variants and/or SARS-CoV-related sarbecoviruses. In more aspects, the antibody or antigen binding fragment has neutralizing activity against omicron BA.1, BA.2, BA.5, EG.5.1 and BA.2.86. In further aspects, the antibody or antigen binding fragment has neutralizing activity against SARS-CoV-2 related viruses from animals, including Pangolin GX variants, as well as SARS-CoV related sarbecoviruses from humans, bats, and civet cats.

In some aspects, the antibody or antigen binding fragment comprises a V_{H} comprising an amino acid sequence at least 90% (such as at least 95%, at least 96%, at least 97%, at least 98%, or at least 99%) identical to the amino acid sequence set forth as SEQ ID NO: 177, and specifically binds to a coronavirus spike protein, and neutralizes a coronavirus. In more aspects, the antibody or antigen binding fragment comprises a V_{L} comprising an amino acid sequence at least 90% (such as at least 95%, at least 96%, at least 97%, at least 98%, or at least 99%) identical to the amino acid sequence set forth as SEQ ID NO: 181, and specifically binds to a coronavirus spike protein, and neutralizes a coronavirus. In additional aspects, the antibody or antigen binding fragment comprises a V_{H} and a V_{L} independently comprising amino acid sequences at least 90% (such as at least 95%, at least 96%, at least 97%, at least 98%, or at least 99%) identical to the amino acid sequences set forth as SEQ ID NOs: 177 and 181, respectively, and specifically binds to a coronavirus spike protein and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 178, 179, and 180, respectively, and/or a V_{L} comprising a LCDR1, a LCDR2, and a LCDR3 as set forth as SEQ ID NOs: 182, 183 (DAS), and 184, respectively, and specifically binds to a coronavirus spike protein, and neutralizes a coronavirus. The coronavirus can be SARS-CoV-2.

In some aspects, the antibody or antigen binding fragment comprises a V_{H} comprising a HCDR1, a HCDR2, and a HCDR3 as set forth as SEQ ID NOs: 178, 179, and 180, respectively, a V_{L} comprising a LCDR1, a LCDR2, and a LCDR3 as set forth as SEQ ID NOs: 182, 183 (DAS), and 184, respectively, wherein the V_{H} comprises an amino acid sequence at least 90% identical to SEQ ID NO: 177, such as 95%, 96%, 97%, 98% or 99% identical to SEQ ID NO: 177, and wherein the V_{L} comprises an amino acid sequence at least 90% identical to SEQ ID NO: 181, such as 95%, 96%, 97%, 98% or 99% identical to SEQ ID NO: 181, and the antibody or antigen binding fragment specifically binds to a coronavirus spike protein, and neutralizes a coronavirus. In this aspect, variations due to sequence identify fall outside the CDRs. The coronavirus can be SARS-CoV-2.

In some aspects, the antibody or antigen binding fragment comprises a V_{H} comprising the amino acid sequence set forth as SEQ ID NO: 177, and specifically binds to a coronavirus spike protein, and neutralizes a coronavirus. In more aspects, the antibody or antigen binding fragment comprises a V_{L} comprising the amino acid sequence set forth as SEQ ID NO: 181, and specifically binds to a coronavirus spike protein, and neutralizes a coronavirus. In some aspects, the antibody or antigen binding fragment comprises a V_{H} and a V_{L} comprising the amino acid sequences set forth as SEQ ID NOs: 177 and 181, respectively, and specifically binds to a coronavirus spike protein, and neutralizes a coronavirus. The coronavirus can be SARS-CoV-2.

In some aspects, the disclosed antibodies inhibit viral entry and/or replication.

### 2. Additional Description of Antibodies and Antigen Binding Fragments

An antibody or antigen binding fragment of the antibodies disclosed herein can be a human antibody or fragment thereof. Chimeric antibodies are also provided. The antibody or antigen binding fragment can include any suitable framework region, such as (but not limited to) a human framework region from another source, or an optimized framework region. Alternatively, a heterologous framework region, such as, but not limited to a mouse or monkey framework region, can be included in the heavy or light chain of the antibodies.

The antibody can be of any isotype. The antibody can be, for example, an IgA, IgM or an IgG antibody, such as IgG₁, IgG₂, IgG₃, or IgG₄. The class of an antibody that specifically binds to a coronavirus spike protein can be switched with another. In one aspect, a nucleic acid molecule encoding V_{L} or V_{H} is isolated such that it does not include any nucleic acid sequences encoding the constant region of the light or heavy chain, respectively. A nucleic acid moleculeB8 encoding V_{L} or V_{H} is then operatively linked to a nucleic acid sequence encoding a C_{L} or C_{H} from a different class of immunoglobulin molecule. This can be achieved, for example, using a vector or nucleic acid molecule that comprises a C_{L} or C_{H} chain. For example, an antibody that specifically binds the spike protein, that was originally IgG may be class switched to an IgA. Class switching can be used to convert one IgG subclass to another, such as from IgG₁ to IgG₂ IgG₃, or IgG₄.

In some examples, the disclosed antibodies are oligomers of antibodies, such as dimers, trimers, tetramers, pentamers, hexamers, septamers, octomers and so on.

The antibody or antigen binding fragment can be derivatized or linked to another molecule (such as another peptide or protein). In general, the antibody or antigen binding fragment is derivatized such that the binding to the spike protein is not affected adversely by the derivatization or labeling. For example, the antibody or antigen binding fragment can be functionally linked (by chemical coupling, genetic fusion, noncovalent association or otherwise) to one or more other molecular entities, such as another antibody (for example, a bi-specific antibody or a diabody), a detectable marker, an effector molecule, or a protein or peptide that can mediate association of the antibody or antibody portion with another molecule (such as a streptavidin core region or a polyhistidine tag).

### (a) Binding affinity

In several aspects, the antibody or antigen binding fragment specifically binds the coronavirus spike protein with an affinity (e.g., measured by K_{D}) of no more than 1.0 x 10⁻⁸ M, no more than 5.0 x 10⁻⁸ M, no more than 1.0 x 10⁻⁹ M, no more than 5.0 x 10⁻⁹ M, no more than 1.0 x 10⁻¹⁰ M, no more than 5.0 x 10⁻¹⁰ M, or no more than 1.0 x 10⁻¹¹ M. K_{D} can be measured, for example, by a radiolabeled antigen binding assay (RIA) performed with the Fab version of an antibody of interest and its antigen. In one assay, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, *e.g.,* Chen et al., J. Mol. Biol. 293(4):865-881, 1999). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23° C.). In a non-adsorbent plate (NUNC^{™} Catalog #269620), 100 µM or 26 pM [¹²⁵I]-antigen are mixed with serial dilutions of a Fab of interest (e.g., consistent with assessment of the anti-VEGF antibody, Fab-12, in Presta et al., Cancer Res. 57(20):4593-4599, 1997). The Fab of interest is then incubated overnight; however, the incubation may continue for a longer period (*e.g.,* about 65 hours) to ensure that equilibrium is reached. Thereafter, the mixtures are transferred to the capture plate for incubation at room temperature (*e.g*., for one hour). The solution is then removed and the plate washed eight times with 0.1% polysorbate 20 (TWEEN-20^{®}) in PBS. When the plates have dried, 150 µl/well of scintillant (MICROSCINT^{™}-20; PerkinElmer) is added, and the plates are counted on a TOPCOUNT^{™} gamma counter (PerkinElmer) for ten minutes. Concentrations of each Fab that give less than or equal to 20% of maximal binding are chosen for use in competitive binding assays.

In another assay, K_{D} can be measured using surface plasmon resonance assays using a BIACOREO-2000 or a BIACORE^{®}-3000 (BIAcore, Inc., Piscataway, N.J.) at 25° C with immobilized antigen CM5 chips at ~10 response units (RU). Briefly, carboxymethylated dextran biosensor chips (CM5, BIACORE^{®}, Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25° C at a flow rate of approximately 25 l/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (K_{D}) is calculated as the ratio k_{off}/kₒₙ. See, *e.g.,* Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO^{™} spectrophotometer (ThermoSpectronic) with a stirred cuvette. Affinity can also be measured by high throughput SPR using the Carterra LSA.

### (b) Multispecific antibodies

In some aspects, a multi-specific antibody, or a bi-specific antibody, such as a dual variable domain antibody (DVD-IG^{™}) is provided that comprises an antibody or antigen binding fragment that specifically binds a coronavirus spike protein, as provided herein. Multi-specific antibodies formats that can be produced using the presently disclosed antibody and antigen binding fragments are disclosed, for example, in Misasi et al., doi.org/10.1101/2022.07.29.502029, biorxiv.org/content/10.1101/2022.07.29.502029v3, November 21, 2022, which is incorporated herein by reference.

The multispecific antibody can be, for example, a bispecific, or trispecific antibody. In some aspects, the multi-valent antibody is a monospecific antibody (for example, trivalent but one specificity). The mutltispecific antibody can include A18-448.1, or an antigen binding fragment thereof. The multispecific antibody can include F768-104_B2, or an antigen binding fragment thereof. The multispecific antibody can include a Group 3 antibody, or an antigen binding fragment thereof. The multispcific antibody can include a Group 4 antibody, or an antigen binding fragment thereof. The multispecific antibody can include a Group 5 antibody, or an antigen binding fragment thereof. The multispecific antibody can include a Group 6 antibody, or an antigen binding fragment thereof.

The multispecific antibody, such as the bispecific antibody, can include the A18-618-448.1 antibody, or an antigen binding fragment thereof, and a Group 4 antibody or an antigen binding fragment thereof. The Group 4 antibody can be, for example, the F768-104_G7 antibody. The multispecific antibody, such as the bispecific antibody, can include the F768-104_B2 antibody, or an antigen binding fragment thereof, combined with a Group 3 antibody or antigen binding fragment thereof. The Group 3 antibody can be, for example, A80-102_G5. The multispecific antibody, such as the bispecific antibody, can include the F768-104_B2 antibody, or an antigen binding fragment thereof, and the A18-448.1 antibody, or an antigen binding fragment thereof.

Other antibodies that specifically bind SARS-CoV-2 can be included in the multispecific antibody, such as the bispecific antibody. In some aspects, the multispecific antibody, such as the bispecific antibody, can include F768-104_B2 or an antigen binding fragment thereof and F769-E12, or an antigen binding fragment thereof, disclosed in U.S. Provisional Application No. 63/433,719, incorporated herein by reference. In further aspects, the multispecific antibody, such as the bispecific antibody, can include A18-448.1 or an antigen binding fragment thereof and F769-E12, F770-G11, or F770-E8, or an antigen binding fragments thereof, disclosed in U.S. Application No. 63/433,719, incorporated herein by reference.

In some aspects, the antibody can be a bispecific antibody. Any suitable method can be used to design and produce a bispecific antibody, such as crosslinking two or more antibodies, antigen binding fragments (such as scFvs) of the same type or of different types. Exemplary methods of making multispecific antibodies, such as bispecific antibodies, include those described in PCT Pub. No. WO2013/163427, which is incorporated by reference herein in its entirety. Non-limiting examples of suitable crosslinkers include those that are heterobifunctional, having two distinctly reactive groups separated by an appropriate spacer (such as m-maleimidobenzoyl-N-hydroxysuccinimide ester) or homobifunctional (such as disuccinimidyl suberate).

The multispecific antibody may have any suitable format that allows for binding to the coronavirus spike protein by the antibody or antigen binding fragment as provided herein. Bispecific single chain antibodies can be encoded by a single nucleic acid molecule. Non-limiting examples of bispecific single chain antibodies, as well as methods of constructing such antibodies are provided in U.S. Pat. Nos. 8,076,459, 8,017,748, 8,007,796, 7,919,089, 7,820,166, 7,635,472, 7,575,923, 7,435,549, 7,332,168, 7,323,440, 7,235,641, 7,229,760, 7,112,324, 6,723,538. Additional examples of bispecific single chain antibodies can be found in PCT application No. WO 99/54440; Mack et al., J. Immunol., 158(8):3965-3970, 1997; Mack et al., Proc. Natl. Acad. Sci. U.S.A., 92(15):7021-7025, 1995; Kufer et al., Cancer Immunol. Immunother., 45(3-4):193-197, 1997; Löffler et al., Blood, 95(6):2098-2103, 2000; and Brühl et al., J. Immunol., 166(4):2420-2426, 2001. Production of bispecific Fab-scFv ("bibody") molecules are described, for example, in Schoonjans et al. (J. Immunol., 165(12):7050-7057, 2000) and Willems et al. (J. Chromatogr. B Analyt. Technol. Biomed Life Sci. 786(1-2):161-176, 2003). For bibodies, a scFv molecule can be fused to one of the V_{L} -CL (L) or V_{H} -CH1 chains, *e.g.,* to produce a bibody one scFv is fused to the C-term of a Fab chain.

The bispecific tetravalent immunoglobulin known as the dual variable domain immunoglobulin or DVD-immunoglobulin molecule is disclosed in Wu et al., MAbs. 2009;1:339-47, doi: 10.4161/mabs.1.4.8755, incorporated herein by reference. See also Nat Biotechnol. 2007 Nov;25(11):1290-7. doi: 10.1038/nbt1345. Epub 2007 Oct 14., also incorporated herein by reference. A DVD-immunoglobulin molecule includes two heavy chains and two light chains. Unlike IgG, however, both heavy and light chains of a DVD-immunoglobulin molecule contain an additional variable domain (VD) connected via a linker sequence at the N-termini of the V_{H} and V_{L} of an existing monoclonal antibody (mAb). Thus, when the heavy and the light chains combine, the resulting DVD-immunoglobulin molecule contains four antigen recognition sites, see Jakob et al., Mabs 5: 358-363, 2013, incorporated herein by reference, see Fig. 1 for schematic and space-filling diagrams. A DVD-immunoglobulin molecule functions to bind two different antigens on each DFab simultaneously.

The outermost or N-terminal variable domain is termed VD1 and the innermost variable domain is termed VD2; the VD2 is proximal to the C-terminal CH1 or CL. As disclosed in Jakob et al., *supra*, DVD-immunoglobulin molecules can be manufactured and purified to homogeneity in large quantities, have pharmacological properties similar to those of a conventional IgG₁, and show in vivo efficacy. Any of the disclosed monoclonal antibodies can be included in a DVD-immunoglobulin format.

### (c) Antigen Binding Fragments

Antigen binding fragments are encompassed by the present disclosure, such as Fab, F(ab')₂, and Fv which include a heavy chain and V_{L} and specifically bind a coronavirus spike protein. These antibody fragments retain the ability to selectively bind with the antigen and are "antigen-binding" fragments. Non-limiting examples of such fragments include:
(1) Fab, the fragment which contains a monovalent antigen-binding fragment of an antibody molecule, can be produced by digestion of whole antibody with the enzyme papain to yield an intact light chain and a portion of one heavy chain;
(2) Fab', the fragment of an antibody molecule can be obtained by treating whole antibody with pepsin, followed by reduction, to yield an intact light chain and a portion of the heavy chain;
(3) (Fab')₂, the fragment of the antibody that can be obtained by treating whole antibody with the enzyme pepsin without subsequent reduction; F(ab')₂ is a dimer of two Fab' fragments held together by two disulfide bonds;
(4) Fv, a genetically engineered fragment containing the V_{L} and V_{L} expressed as two chains; and
(5) Single chain antibody (such as scFv), defined as a genetically engineered molecule containing the V_{H} and the V_{L} linked by a suitable polypeptide linker as a genetically fused single chain molecule (see, *e.g.,* Ahmad et al., Clin. Dev. Immunol., 2012, doi:10.1155/2012/980250; Marbry and Snavely, IDrugs, 13(8):543-549, 2010). The intramolecular orientation of the V_{H} -domain and the V_{L}-domain in a scFv, is not decisive for the provided antibodies (*e.g*., for the provided multispecific antibodies). Thus, scFvs with both possible arrangements (V_{H}-domain-linker domain-V_{L}-domain; V_{L}-domain-linker domain-V_{H}-domain) may be used.
(6) A dimer of a single chain antibody (scFV₂), defined as a dimer of a scFV. This has also been termed a "miniantibody."

Any suitable method of producing the above-discussed antigen binding fragments may be used. Non-limiting examples are provided in Harlow and Lane, Antibodies: A Laboratory Manual, 2nd, Cold Spring Harbor Laboratory, New York, 2013.

Antigen binding fragments can be prepared by proteolytic hydrolysis of the antibody or by expression in a host cell (such as an *E. coli* cell) of DNA encoding the fragment. Antigen binding fragments can also be obtained by pepsin or papain digestion of whole antibodies by conventional methods. For example, antigen binding fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments.

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

### (d) Variants

In some aspects, amino acid sequence variants of the antibodies and multispecific antibodies, such as bispecific antibodies) provided herein are provided. For example, it may be desirable to improve the binding affinity and/or other biological properties of the antibody or bispecific antibody. Amino acid sequence variants of an antibody may be prepared by introducing appropriate modifications into the nucleotide sequence encoding the antibody V_{H} domain and/or V_{L} domain, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g*., antigen-binding.

In some aspects, variants having one or more amino acid substitutions are provided. Sites of interest for substitutional mutagenesis include the CDRs and the framework regions. Amino acid substitutions may be introduced into an antibody of interest and the products screened for a desired activity, *e.g*., retained/improved antigen binding, decreased immunogenicity, or improved ADCC or CDC.

The variants typically retain amino acid residues necessary for correct folding and stabilizing between the V_{H} and the V_{L} regions, and will retain the charge characteristics of the residues in order to preserve the low pI and low toxicity of the molecules. Amino acid substitutions can be made in the V_{H} and the V_{L} regions to increase yield.

In some aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 1. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 5.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 9. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 13.

In further aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 17. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 21.

In yet other aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 29. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 29.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 33. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 37.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 41. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 45.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 49. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 53.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 57. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 61.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 65. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 69.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 73. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 77.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 81. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 85.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 89. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 93.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 97. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 101.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 105. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 109.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 113. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 117.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 121. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 125.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 129. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 133.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 137. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 141.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 145. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 149.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 153. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 157.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 161. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 165.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 169. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 173.

In more aspects, the heavy chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 177. In some aspects, the light chain of the antibody comprises up to 10 (such as up to 1, up to 2, up to 3, up to 4, up to 5, up to 6, up to 7, up to 8, or up to 9) amino acid substitutions (such as conservative amino acid substitutions) compared to the amino acid sequence set forth as one of SEQ ID NO: 181.

In some aspects, the antibody or antigen binding fragment can include up to 10 (such as up to 1, up to 2, up to 3, up to 4, up to 5, up to 6, up to 7, up to 8, or up to 9) amino acid substitutions (such as conservative amino acid substitutions) in the framework regions of the heavy chain of the antibody/multispecific antibody, or the light chain of the antibody/multipecific antibody, or the heavy and light chains of the antibody/bispecific antibody, compared to known framework regions, or compared to the framework regions of the antibody, and maintain the specific binding activity for the epitope of the spike protein. In these aspects, the substitutions are not in the CDRs.

In some aspects, substitutions, insertions, or deletions may occur within one or more CDRs so long as such alterations do not substantially reduce the ability of the antibody to bind antigen. For example, conservative alterations (e.g., conservative substitutions as provided herein) that do not substantially reduce binding affinity may be made in CDRs. In some aspects of the variant V_{H} and V_{L} sequences provided above, each CDR either is unaltered, or contains no more than one, two or three amino acid substitutions. In some aspects of the variant V_{H} and V_{L} sequences provided above, only the framework residues are modified so the CDRs are unchanged.

To increase binding affinity of the antibody, the V_{L} and V_{H} segments can be randomly mutated, such as within HCDR3 region or the LCDR3 region, in a process analogous to the *in vivo* somatic mutation process responsible for affinity maturation of antibodies during a natural immune response. Thus *in vitro* affinity maturation can be accomplished by amplifying V_{H} and V_{L} regions using PCR primers complementary to the HCDR3 or LCDR3, respectively. In this process, the primers have been "spiked" with a random mixture of the four nucleotide bases at certain positions such that the resultant PCR products encode V_{H} and V_{L} segments into which random mutations have been introduced into the V_{H} and/or V_{L} CDR3 regions. These randomly mutated V_{H} and V_{L} segments can be tested to determine the binding affinity for the spike protein. In particular examples, the V_{H} amino acid sequence is one of SEQ ID NOs:1, 9, 17, 25, 33, 41, 49, 57, 65, 73, 81, 89, 97, 105, 113, 121, 129, 137, 145, 153, 161, 169, or 177. In other examples, the V_{L} amino acid sequence is one of SEQ ID NOs: 5, 13, 21, 29, 37, 45, 53, 61, 69, 77, 85, 93, 101, 109, 117, 125, 133, 141, 149, 157, 165, 173, or 181, respectively.

In some aspects, an antibody disclosed herein, an antigen binding fragment, or bispecific antibody is altered to increase or decrease the extent to which the antibody or antigen binding fragment is glycosylated. Addition or deletion of glycosylation sites may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH₂ domain of the Fc region. See, *e.g*., Wright et al. Trends Biotechnol. 15(1):26-32, 1997. The oligosaccharide may include various carbohydrates, *e.g.,* mannose, N-acetyl glucosamine (GlcNAc), galactose, and sialic acid, as well as a fucose attached to a GlcNAc in the "stem" of the biantennary oligosaccharide structure. In some aspects, modifications of the oligosaccharide in an antibody may be made in order to create antibody variants with certain improved properties.

In one aspect, variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g. complex, hybrid and high mannose structures) as measured by MALDI-TOF mass spectrometry, as described in WO 2008/077546, for example. Asn297 refers to the asparagine residue located at about position 297 in the Fc region; however, Asn297 may also be located about ±3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO 2002/031140; Okazaki et al., J. Mol. Biol., 336(5):1239-1249, 2004; Yamane-Ohnuki et al., Biotechnol. Bioeng. 87(5):614-622, 2004. Examples of cell lines capable of producing defucosylated antibodies include Lec 13 CHO cells deficient in protein fucosylation (Ripka et al., Arch. Biochem. Biophys. 249(2):533-545, 1986; US Pat. Appl. No. US 2003/0157108 and WO 2004/056312, especially at Example 11), and knockout cell lines, such as alpha-1,6-fucosyltransferase gene, FUT8, knockout CHO cells (see, *e.g.,* Yamane-Ohnuki et al., Biotechnol. Bioeng., 87(5): 614-622, 2004; Kanda et al., Biotechnol. Bioeng., 94(4):680-688, 2006; and WO2003/085107).

Antibody variants are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody variants are described, *e.g.,* in WO 2003/011878 (Jean-Mairet et al*.*); U.S. Pat. No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g.,* in WO 1997/30087; WO 1998/58964; and WO 1999/22764.

In several aspects, the constant region of the antibody or bispecific antibody comprises one or more amino acid substitutions to optimize *in vivo* half-life of the antibody. The serum half-life of IgG Abs is regulated by the neonatal Fc receptor (FcRn). Thus, in several aspects, the antibody comprises an amino acid substitution that increases binding to the FcRn. Non-limiting examples of such substitutions include substitutions at IgG constant regions T250Q and M428L (see, *e.g.,* Hinton et al., J Immunol., 176(1):346-356, 2006); M428L and N434S (the "LS" mutation, see, *e.g.,* Zalevsky, et al., Nature Biotechnol., 28(2):157-159, 2010); N434A (see, *e.g.,* Petkova et al., Int. Immunol., 18(12):1759-1769, 2006); T307A, E380A, and N434A (see, *e.g.,* Petkova et al., Int. Immunol., 18(12):1759-1769, 2006); and M252Y, S254T, and T256E (see, *e.g.,* Dall'Acqua et al., J. Biol. Chem., 281(33):23514-23524, 2006). The disclosed antibodies and antigen binding fragments can be linked to or comprise an Fc polypeptide including any of the substitutions listed above, for example, the Fc polypeptide can include the M428L and N434S substitutions.

In some aspects, an antibody or multispecific (such as bispecific) antibody provided herein may be further modified to contain additional nonproteinaceous moieties. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in an application under defined conditions, etc.

### B. Conjugates

The antibodies, antigen binding fragments, and multispecific (such as bispecific) antibodies that specifically bind to a coronavirus spike protein, as disclosed herein, can be conjugated to an agent, such as an effector molecule or detectable marker. Both covalent and noncovalent attachment means may be used. Various effector molecules and detectable markers can be used, including (but not limited to) toxins and radioactive agents such as ¹²⁵I, ³²P, ¹⁴C, ³H and ³⁵S and other labels, target moieties, enzymes and ligands, etc. The choice of a particular effector molecule or detectable marker depends on the particular target molecule or cell, and the desired biological effect.

The procedure for attaching a detectable marker to an antibody, antigen binding fragment, or bispecific antibody. varies according to the chemical structure of the effector. Polypeptides typically contain a variety of functional groups, such as carboxyl (-COOH), free amine (-NH₂) or sulfhydryl (-SH) groups, which are available for reaction with a suitable functional group on a polypeptide to result in the binding of the effector molecule or detectable marker. Alternatively, the antibody, antigen binding fragment, or bispecific antibody, is derivatized to expose or attach additional reactive functional groups. The derivatization may involve attachment of any suitable linker molecule. The linker is capable of forming covalent bonds to both the antibody or antigen binding fragment and to the effector molecule or detectable marker. Suitable linkers include, but are not limited to, straight or branched-chain carbon linkers, heterocyclic carbon linkers, or peptide linkers. Where the antibody, antigen binding fragment, or bispecific antibody, and the effector molecule or detectable marker are polypeptides, the linkers may be joined to the constituent amino acids through their side chains (such as through a disulfide linkage to cysteine) or the alpha carbon, or through the amino, and/or carboxyl groups of the terminal amino acids.

In view of the large number of methods that have been reported for attaching a variety of radiodiagnostic compounds, radiotherapeutic compounds, labels (such as enzymes or fluorescent molecules), toxins, and other agents to antibodies, a suitable method for attaching a given agent to an antibody or antigen binding fragment or bispecific antibody can be determined.

The antibody, antigen binding fragment or multispecific (such as bispecific) antibody can be conjugated with a detectable marker; for example, a detectable marker capable of detection by ELISA, spectrophotometry, flow cytometry, microscopy or diagnostic imaging techniques (such as CT, computed axial tomography (CAT), MRI, magnetic resonance tomography (MTR), ultrasound, fiberoptic examination, and laparoscopic examination). Specific, non-limiting examples of detectable markers include fluorophores, chemiluminescent agents, enzymatic linkages, radioactive isotopes and heavy metals or compounds (for example super paramagnetic iron oxide nanocrystals for detection by MRI). For example, useful detectable markers include fluorescent compounds, including fluorescein, fluorescein isothiocyanate, rhodamine, 5-dimethylamine-1-napthalenesulfonyl chloride, phycoerythrin, lanthanide phosphors and the like. Bioluminescent markers are also of use, such as luciferase, green fluorescent protein (GFP), and yellow fluorescent protein (YFP). An antibody, antigen binding fragment, or multispecific (such as bispecific) antibody, can also be conjugated with enzymes that are useful for detection, such as horseradish peroxidase, β- galactosidase, luciferase, alkaline phosphatase, glucose oxidase and the like. When an antibody or antigen binding fragment is conjugated with a detectable enzyme, it can be detected by adding additional reagents that the enzyme uses to produce a reaction product that can be discerned. For example, when the agent horseradish peroxidase is present, the addition of hydrogen peroxide and diaminobenzidine leads to a colored reaction product, which is visually detectable. An antibody, antigen binding fragment, or multispecific (such as bispecific) antibody, may also be conjugated with biotin, and detected through indirect measurement of avidin or streptavidin binding. It should be noted that the avidin itself can be conjugated with an enzyme or a fluorescent label.

The antibody, antigen binding fragment or bispecific antibody, can be conjugated with a paramagnetic agent, such as gadolinium. Paramagnetic agents such as superparamagnetic iron oxide are also of use as labels. Antibodies can also be conjugated with lanthanides (such as europium and dysprosium), and manganese. An antibody, antigen binding fragment, or multispecific (such as bispecific) antibody, may also be labeled with a predetermined polypeptide epitope recognized by a secondary reporter (such as leucine zipper pair sequences, binding sites for secondary antibodies, metal binding domains, epitope tags).

The antibody, antigen binding fragment or multispecific (such as bispecific) antibody, can also be conjugated with a radiolabeled amino acid, for example, for diagnostic purposes. For instance, the radiolabel may be used to detect a coronavirus by radiography, emission spectra, or other diagnostic techniques. Examples of labels for polypeptides include, but are not limited to, the following radioisotopes: ³H, ¹⁴C, ³⁵S, ⁹⁰Y, ^{99m}Tc, ¹¹¹In ¹²⁵I, ¹³¹I. The radiolabels may be detected, for example, using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label.

The average number of detectable marker moieties per antibody, antigen binding fragment, or bispecific antibody in a conjugate can range, for example, from 1 to 20 moieties per antibody or antigen binding fragment. In some aspects, the average number of effector molecules or detectable marker moieties per antibody or antigen binding fragment in a conjugate range from about 1 to about 2, from about 1 to about 3, about 1 to about 8; from about 2 to about 6; from about 3 to about 5; or from about 3 to about 4. The loading (for example, effector molecule per antibody ratio) of a conjugate may be controlled in different ways, for example, by: (i) limiting the molar excess of effector molecule-linker intermediate or linker reagent relative to antibody, (ii) limiting the conjugation reaction time or temperature, (iii) partial or limiting reducing conditions for cysteine thiol modification, (iv) engineering by recombinant techniques the amino acid sequence of the antibody such that the number and position of cysteine residues is modified for control of the number or position of linker-effector molecule attachments.

### C. Polynucleotides and Expression

Nucleic acid molecules (for example, cDNA or RNA molecules) encoding the amino acid sequences of antibodies, antigen binding fragments, bispecific antibodies, and conjugates that specifically bind to a coronavirus spike protein, as disclosed herein, are provided. Nucleic acids encoding these molecules can readily be produced using the amino acid sequences provided herein (such as the CDR sequences and V_{H} and V_{L} sequences), sequences available in the art (such as framework or constant region sequences), and the genetic code. In several aspects, nucleic acid molecules can encode the V_{H}, the V_{L}, or both the V_{H} and V_{L} (for example in a bicistronic expression vector) of a disclosed antibody or antigen binding fragment. In some aspects, the nucleic acid molecules encode an scFv. In several aspects, the nucleic acid molecules can be expressed in a host cell (such as a mammalian cell) to produce a disclosed antibody or antigen binding fragment. Nucleic acid molecules encoding an scFv are provided.

The genetic code can be used to construct a variety of functionally equivalent nucleic acid sequences, such as nucleic acids which differ in their sequence but which encode the same antibody sequence, or encode a conjugate or fusion protein including the V_{L} and/or V_{H} nucleic acid sequence.

In a non-limiting example, an isolated nucleic acid molecule encodes the V_{H} of a disclosed antibody. In another non-limiting example, the nucleic acid molecule encodes the V_{L} of a disclosed antibody. In further non-limiting examples, the nucleic acid molecule can encode a bi-specific antibody, such as in DVD-immunoglobulin format.

Nucleic acid molecules encoding the antibodies, antigen binding fragments, multispecific (such as bispecific) antibodies, and conjugates that specifically bind to a coronavirus spike protein can be prepared by any suitable method including, for example, cloning of appropriate sequences or by direct chemical synthesis by standard methods. Chemical synthesis produces a single stranded oligonucleotide. This can be converted into double stranded DNA by hybridization with a complementary sequence or by polymerization with a DNA polymerase using the single strand as a template.

Exemplary nucleic acids can be prepared by cloning techniques. Examples of appropriate cloning and sequencing techniques can be found, for example, in Green and Sambrook (Molecular Cloning: A Laboratory Manual, 4th ed., New York: Cold Spring Harbor Laboratory Press, 2012) and Ausubel et al. (Eds.) (Current Protocols in Molecular Biology, New York: John Wiley and Sons, including supplements).

Nucleic acids can also be prepared by amplification methods. Amplification methods include the polymerase chain reaction (PCR), the ligase chain reaction (LCR), the transcription-based amplification system (TAS), and the self-sustained sequence replication system (3SR).

The nucleic acid molecules can be expressed in a recombinantly engineered cell such as bacteria, plant, yeast, insect and mammalian cells. The antibodies, antigen binding fragments, and conjugates can be expressed as individual proteins including the V_{H} and/or V_{L} (linked to an effector molecule or detectable marker as needed), or can be expressed as a fusion protein. Any suitable method of expressing and purifying antibodies and antigen binding fragments may be used; non-limiting examples are provided in Al-Rubeai (Ed.), Antibody Expression and Production, Dordrecht; New York: Springer, 2011). An immunoadhesin can also be expressed. Thus, in some examples, nucleic acids encoding a V_{H} and V_{L}, and immunoadhesin are provided. The nucleic acid sequences can optionally encode a leader sequence.

To create a scFv the V_{H}- and V_{L}-encoding DNA fragments can be operatively linked to another fragment encoding a flexible linker, *e.g*., encoding the amino acid sequence (Gly₄-Ser)₃, such that the V_{H} and V_{L} sequences can be expressed as a contiguous single-chain protein, with the V_{L} and V_{H} domains joined by the flexible linker (see, *e.g.,* Bird et al., Science, 242(4877):423-426, 1988; Huston et al., Proc. Natl. Acad. Sci. U.S.A., 85(16):5879-5883, 1988; McCafferty et al., Nature, 348:552-554, 1990; Kontermann and Dübel (Eds.), Antibody Engineering, Vols. 1-2, 2nd ed., Springer-Verlag, 2010; Greenfield (Ed.), Antibodies: A Laboratory Manual, 2nd ed. New York: Cold Spring Harbor Laboratory Press, 2014). Optionally, a cleavage site can be included in a linker, such as a furin cleavage site.

The single chain antibody may be monovalent, if only a single V_{H} and V_{L} are used, bivalent, if two V_{H} and V_{L} are used, or polyvalent, if more than two V_{H} and V_{L} are used. Bispecific or polyvalent antibodies may be generated that bind specifically to a coronavirus spike protein and another antigen. The encoded V_{H} and V_{L} optionally can include a furin cleavage site between the V_{H} and V_{L} domains. Linkers can also be encoded, such as when the nucleic acid molecule encodes a bi-specific antibody in DVD-IG^{™} format.

One or more DNA sequences encoding the antibodies, antigen binding fragments, bispecific antibodies, or conjugates can be expressed *in vitro* by DNA transfer into a suitable host cell. The cell may be prokaryotic or eukaryotic. Numerous expression systems available for expression of proteins including *E. coli,* other bacterial hosts, yeast, and various higher eukaryotic cells such as the COS, CHO, HeLa and myeloma cell lines, can be used to express the disclosed antibodies and antigen binding fragments. Methods of stable transfer, meaning that the foreign DNA is continuously maintained in the host may be used. Hybridomas expressing the antibodies of interest are also encompassed by this disclosure.

The expression of nucleic acids encoding the antibodies, antigen binding fragments, and multispecific antibodies (such as bispecific antibodies) described herein can be achieved by operably linking the DNA or cDNA to a promoter (which is either constitutive or inducible), followed by incorporation into an expression cassette. The promoter can be any promoter of interest, including a cytomegalovirus promoter. Optionally, an enhancer, such as a cytomegalovirus enhancer, is included in the construct. The cassettes can be suitable for replication and integration in either prokaryotes or eukaryotes. Typical expression cassettes contain specific sequences useful for regulation of the expression of the DNA encoding the protein. For example, the expression cassettes can include appropriate promoters, enhancers, transcription and translation terminators, initiation sequences, a start codon (*i.e.,* ATG) in front of a protein-encoding gene, splicing signals for introns, sequences for the maintenance of the correct reading frame of that gene to permit proper translation of mRNA, and stop codons. The vector can encode a selectable marker, such as a marker encoding drug resistance (for example, ampicillin or tetracycline resistance).

To obtain high level expression of a cloned gene, it is desirable to construct expression cassettes which contain, for example, a strong promoter to direct transcription, a ribosome binding site for translational initiation (e.g., internal ribosomal binding sequences), and a transcription/translation terminator. For *E. coli,* this can include a promoter such as the T7, trp, lac, or lamda promoters, a ribosome binding site, and preferably a transcription termination signal. For eukaryotic cells, the control sequences can include a promoter and/or an enhancer derived from, for example, an immunoglobulin gene, HTLV, SV40 or cytomegalovirus, and a polyadenylation sequence, and can further include splice donor and/or acceptor sequences (for example, CMV and/or HTLV splice acceptor and donor sequences). The cassettes can be transferred into the chosen host cell by any suitable method such as transformation or electroporation for *E. coli* and calcium phosphate treatment, electroporation or lipofection for mammalian cells. Cells transformed by the cassettes can be selected by resistance to antibiotics conferred by genes contained in the cassettes, such as the amp, gpt, neo and hyg genes.

Modifications can be made to a nucleic acid encoding a polypeptide described herein without diminishing its biological activity. Some modifications can be made to facilitate the cloning, expression, or incorporation of the targeting molecule into a fusion protein. Such modifications include, for example, termination codons, sequences to create conveniently located restriction sites, and sequences to add a methionine at the amino terminus to provide an initiation site, or additional amino acids (such as poly His) to aid in purification steps.

Once expressed, the antibodies, antigen binding fragments, multispecific (such as bispecific) antibodies, and conjugates can be purified according to standard procedures in the art, including ammonium sulfate precipitation, affinity columns, column chromatography, and the like (see, generally, Simpson et al. (Eds.), Basic methods in Protein Purification and Analysis: A Laboratory Manual, New York: Cold Spring Harbor Laboratory Press, 2009). The antibodies, antigen binding fragment, and conjugates need not be 100% pure. Once purified, partially or to homogeneity as desired, if to be used prophylatically, the polypeptides should be substantially free of endotoxin.

Methods for expression of antibodies, antigen binding fragments, bispecific antibodies, and conjugates, and/or refolding to an appropriate active form, from mammalian cells, and bacteria such as *E*. *coli* have been described and are applicable to the antibodies disclosed herein. See, *e.g.,* Greenfield (Ed.), Antibodies: A Laboratory Manual, 2nd ed. New York: Cold Spring Harbor Laboratory Press, 2014, Simpson et al. (Eds.), Basic methods in Protein Purification and Analysis: A Laboratory Manual, New York: Cold Spring Harbor Laboratory Press, 2009, and Ward et al., Nature 341(6242):544-546, 1989.

### D. Methods and Compositions

### 1. Inhibiting a coronavirus infection

Methods are disclosed herein for the inhibition of a coronavirus infection in a subject, such as a SARS-CoV-2 infection. Methods are also disclosed herein for the inhibition of a coronavirus infection, such as a SARS-CoV-2 BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and/or XEC, RaTG13, Pangolin GD, Pangolin GX-P2V, WIV1 and/or SHC-014. The SARS-CoV-2 can be BA.1, BA.2, BA.4/5, BA.2.75.2, XBB.1.5, EG.5.1, FL.1.5.1, BA.2.86 IN.1, KP.2, KP.3, LB.1, KP.2.3, KP.3.1.1 or XEC. The methods include administering to the subject an effective amount (that is, an amount effective to inhibit the infection in the subject) of a disclosed antibody, antigen binding fragment, or bispecific antibody, or a nucleic acid encoding such an antibody, antigen binding fragment, or bispecific antibody, to a subject at risk of a coronavirus infection or having the coronavirus infection. The methods can be used pre-exposure or post-exposure. The subject can be immunocompromised. The subject can be elderly, such as greater than about 65, about 70, about 75, about 80, about 85 or about 90 years of age. In some aspects, the antibody or antigen binding fragment can be used in the form of a bi-specific antibody. The antigen binding fragment can be an scFv.

The infection does not need to be completely eliminated or inhibited for the method to be effective. For example, the method can decrease the infection by a desired amount, for example by at least 10%, at least 20%, at least 50%, at least 60%, at least 70%, at least 80%, at least 90%, at least 95%, at least 98%, or even at least 100% (elimination or prevention of detectable coronavirus infection) as compared to the coronavirus infection in the absence of the treatment. In some aspects, the subject can also be treated with an effective amount of an additional agent, such as an anti-viral agent.

In some aspects, administration of an effective amount of a disclosed antibody, antigen binding fragment, bispecific antibody, or nucleic acid molecule, inhibits the establishment of an infection and/or subsequent disease progression in a subject, which can encompass any statistically significant reduction in activity (for example, growth or invasion) or symptoms of the coronavirus infection in the subject.

Methods are disclosed herein for the inhibition of a coronavirus, such as SARS-CoV-2 replication in a subject. The coronavirus can be SARS-CoV-2 BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and/or XEC, RaTG13, Pangolin GD, Pangolin GX-P2V, WIV1 and/or SHC-014. The coronavirus can be SARS-CoV-1, or a SARS-CoV-1 related virus from non-human animals, such as civet cats or pangolins. The methods include administering to the subject an effective amount (that is, an amount effective to inhibit replication in the subject) of a disclosed antibody, antigen binding fragment, bispecific antibody, or a nucleic acid encoding such an antibody, antigen binding fragment, or bispecific antibody, to a subject at risk of a coronavirus infection or having a coronavirus infection. The methods can be used pre-exposure or post-exposure.

Methods are disclosed for treating a SARS-CoV-2 infection in a subject. Methods are also disclosed for preventing a SARS-CoV-2 infection in a subject. These methods include administering one or more of the disclosed antibodies, antigen binding fragments, bispecific antibodies, or nucleic acid molecule encoding such molecules, or a composition including such molecules, as disclosed herein.

Antibodies, antigen binding fragments thereof, and bispecific antibodies can be administered by intravenous infusion. Doses of the antibody, antigen binding fragment, or bispecific antibody vary, but generally range between about 0.5 mg/kg to about 50 mg/kg, such as a dose of about 1 mg/kg, about 5 mg/kg, about 10 mg/kg, about 20 mg/kg, about 30 mg/kg, about 40 mg/kg, or about 50 mg/kg. In some aspects, the dose of the antibody, antigen binding fragment or bispecific antibody can be from about 0.5 mg/kg to about 5 mg/kg, such as a dose of about 1 mg/kg, about 2 mg/kg, about 3 mg/kg, about 4 mg/kg or about 5 mg/kg. The antibody, antigen binding fragment, or bispecific antibody is administered according to a dosing schedule determined by a medical practitioner. In some examples, the antibody, antigen binding fragment or bispecific antibody is administered weekly, every two weeks, every three weeks or every four weeks.

In some aspects, the method of inhibiting the infection in a subject further comprises administration of one or more additional agents to the subject. Additional agents of interest include, but are not limited to, anti-viral agents such as hydroxychloroquine, arbidol, remdesivir, favipiravir, baricitinib, lopinavir/ritonavir, Zinc ions, and interferon beta-1b, or their combinations.

In some aspects, the method comprises administration of a first antibody that specifically binds to a SARS-CoV-2 spike protein as disclosed herein and a second antibody that also specifically binds to a SARS-CoV-2 protein, such as a different epitope of the coronavirus protein Thus, 2, 3, 4, 5, 6, 7, 8, 9, 10, 11, 12, 13, 14, 15, 16, 17, 18, 19 or 20 of the disclosed antibodies, or antigen binding fragments thereof, can be administered to the subject

The method can include administering at least 2, 3, 4, or 5 of the disclosed monoclonal antibodies or antigen binding fragments. The method can include administration of an effective amount of A18-448.1, or an antigen binding fragment thereof. The method can include administration of an effective amount of F768-104_B2, or an antigen binding fragment thereof. The method can include administration of an effective amount of a Group 3 antibody, or an antigen binding fragment thereof. The method can include administration of an effective amount of a Group 4 antibody, or an antigen binding fragment thereof. The method can include administration of an effective amount of a Group 5 antibody, or an antigen binding fragment thereof. The method can include administration of an effective amount of can include a Group 6 antibody, or an antigen binding fragment thereof.

A combination of use includes, but is not limited to, A18-618-448.1, or an antigen binding fragment thereof, with a Group 4 antibody or an antigen binding fragment thereof. The Group 4 antibody can be, for example, F768-104_G7. Another combination of use is F768-104_B2, or an antigen binding fragment thereof, combined with a Group 3 antibody or antigen binding fragment thereof. The Group 3 antibody can be, for example, A80-102_G5. A further combination of use is F768-104_B2, or an antigen binding fragment thereof, combined with A18-448.1, or an antigen binding fragment thereof.

Other antibodies that specifically bind SARS-CoV-2 can be used with the presently disclosed antibodies. In some aspects, F768-104_B2 or an antigen binding fragment thereof can be used in combination with antibody F769-E12, or an antigen binding fragment thereof, disclosed in U.S. Provisional Application No. 63/433,719, incorporated herein by reference. In addition, A18-448.1 or an antigen binding fragment thereof can be used in combination with F769-E12, F770-G11, or F770-E8,or an antigen binding fragment thereof, disclosed in # U.S. Application No. 63/433,719, incorporated herein by reference.

In some aspects, a Class I and a Class II, and Class I and a Class III, a Class II and a Class III, or a Class I, Class II, and a Class III antibody are administered to the subject in combination. More than one Class I, Class II, or Class III antibody can be utilized. In some aspects, a Class I and a Class II, and Class I and a Class III, a Class II and a Class III, or a Class I, Class II, and a Class III antibody are administered to the subject in combination. More than one Class I, Class II, or Class III antibody can be utilized.

The disclosed methods can include the administration of one or more multispecific antibodies. Combinations of these multispecific antibodies, and combinations of bispecific antibodies, such as one, two, three, four, or five of these multispecific antibodies, such as bispecific antibodies, can be administered to the subject. Nucleic acid molecules are also of use in these aspects.

In some aspects, a subject is administered DNA or RNA encoding a disclosed antibody, antigen binding fragment, or multispecific antibody (such as a bispecific antibody), to provide *in vivo* antibody production, for example using the cellular machinery of the subject. Any suitable method of nucleic acid administration may be used; non-limiting examples are provided in U.S. Patent No. 5,643,578, U.S. Patent No. 5,593,972 and U.S. Patent No. 5,817,637. U.S. Patent No. 5,880,103 describes several methods of delivery of nucleic acids encoding proteins to an organism. One approach to administration of nucleic acids is direct administration with plasmid DNA, such as with a mammalian expression plasmid. The nucleotide sequence encoding the disclosed antibody, antigen binding fragments thereof, or multispecific antibody (such as a bispecific antibody), can be placed under the control of a promoter to increase expression. The methods include liposomal delivery of the nucleic acids. Such methods can be applied to the production of an antibody, or antigen binding fragments thereof. In some aspects, a disclosed antibody or antigen binding fragment is expressed in a subject using the pVRC8400 vector (described in Barouch et al., J. Virol., 79(14), 8828-8834, 2005, which is incorporated by reference herein).

In several aspects, a subject (such as a human subject at risk of a coronavirus infection or having a coronavirus infection) can be administered an effective amount of an AAV viral vector that comprises one or more nucleic acid molecules encoding a disclosed antibody, antigen binding fragment, or multispecific (such as bispecific) antibody. The AAV viral vector is designed for expression of the nucleic acid molecules encoding a disclosed antibody, antigen binding fragment, or bispecific antibody, and administration of the effective amount of the AAV viral vector to the subject leads to expression of an effective amount of the antibody, antigen binding fragment, or bispecific antibody in the subject. Non-limiting examples of AAV viral vectors that can be used to express a disclosed antibody, antigen binding fragment, or bispecific antibody in a subject include those provided in Johnson et al., Nat. Med., 15(8):901-906, 2009 and Gardner et al., Nature, 519(7541):87-91, 2015, each of which is incorporated by reference herein in its entirety.

In one aspect, a nucleic acid encoding a disclosed antibody, antigen binding fragment, or multispecific antibody (such as a bispecific antibody), is introduced directly into tissue. For example, the nucleic acid can be loaded onto gold microspheres by standard methods and introduced into the skin by a device such as Bio-Rad's HELIOS^{™} Gene Gun. The nucleic acids can be "naked," consisting of plasmids under control of a strong promoter.

Typically, the DNA is injected into muscle, although it can also be injected directly into other sites. Dosages for injection are usually around 0.5 µg/kg to about 50 mg/kg, and typically are about 0.005 mg/kg to about 5 mg/kg (see, *e.g.,* U.S. Patent No. 5,589,466).

Single or multiple administrations of a composition including a disclosed antibody, antigen binding fragment, or multispecific antibody (such as a bispecific antibody), conjugate, or nucleic acid molecule encoding such molecules, can be administered depending on the dosage and frequency as required and tolerated by the patient. The dosage can be administered once, but may be applied periodically until either a desired result is achieved or until side effects warrant discontinuation of therapy. Generally, the dose is sufficient to inhibit a coronavirus infection without producing unacceptable toxicity to the patient.

Data obtained from cell culture assays and animal studies can be used to formulate a range of dosage for use in humans. The dosage normally lies within a range of circulating concentrations that include the ED₅₀, with little or minimal toxicity. The dosage can vary within this range depending upon the dosage form employed and the route of administration utilized. The effective dose can be determined from cell culture assays and animal studies.

The SARS-CoV-2 -specific antibody, antigen binding fragment, multispecific antibody (such as a bispecific antibody), or nucleic acid molecule encoding such molecules, or a composition including such molecules, can be administered to subjects in various ways, including local and systemic administration, such as, *e.g.,* by injection subcutaneously, intravenously, intra-arterially, intraperitoneally, intramuscularly, intradermally, or intrathecally. In an aspect, the antibody, antigen binding fragment, multispecific antibody (such as a bispecific antibody), or nucleic acid molecule encoding such molecules, or a composition including such molecules, is administered by a single subcutaneous, intravenous, intra-arterial, intraperitoneal, intramuscular, intradermal or intrathecal injection once a day. The antibody, antigen binding fragment, multispecific antibody (such as a bispecific antibody), conjugate, or nucleic acid molecule encoding such molecules, or a composition including such molecules, can also be administered by direct injection at or near the site of disease. A further method of administration is by osmotic pump (*e.g.,* an Alzet pump) or mini-pump (*e.g*., an Alzet mini-osmotic pump), which allows for controlled, continuous and/or slow-release delivery of the antibody, antigen binding fragment, conjugate, or nucleic acid molecule encoding such molecules, or a composition including such molecules, over a pre-determined period. The osmotic pump or mini-pump can be implanted subcutaneously, or near a target site.

### 2. Compositions

Compositions are provided that include one or more of the coronavirus spike protein-specific antibody, antigen binding fragment, multispecific antibody (such as a bispecific antibody), conjugate, or nucleic acid molecule encoding such molecules, that are disclosed herein in a pharmaceutically acceptable carrier. In some aspects, the composition comprises two, three, four or more antibodies, antigen binding fragments, or bispecific antibodies, that specifically bind a coronavirus spike protein. The compositions are useful, for example, for example, for the inhibition or detection of a coronavirus infection, such as, but not limited to, a SARS-CoV-2 infection. The coronavirus can be SARS-CoV-2 BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and/or XEC, RaTG13, Pangolin GD, Pangolin GX-P2V, WIV1 and/or SHC-014. The coronavirus can be SARS-CoV-1, or a SARS-CoV-1 related virus from non-human animals, such as civet cats or pangolins. In some aspects, the compositions includes one or more of the A18-448.1, F768-104_B2, A80-102_G5, A80-338-19.5, A80-338-19.6, A80-338-19.8, A80-338-19.9, E184-105_F3, A63-652-32.1, F768-104_G7, E184-105_H1, F768-104_D10, A63-652-1.4, E184-105_E5, E184-105_H9, E184-105_H2, A80-102+103_G1, A80-103_E2, A80-103_B6, A18-618-452.1, A80-102+103_C1, A43-d321.10.4, and F768-100-pt2_A05 antibodies disclosed herein, or an antigen binding fragment thereof, or a multispecific antibody thereof. In some aspects, the composition comprises two, three, four or more antibodies that specifically bind a coronavirus spike protein. The compositions are useful, for example, for example, for the inhibition or detection of a coronavirus infection, such as a SARS-CoV-2 infection.

The compositions can be prepared in unit dosage forms, such as in a kit, for administration to a subject. The amount and timing of administration are at the discretion of the administering physician to achieve the desired purposes. The antibody, antigen binding fragment, bispecific antibody, conjugate, or nucleic acid molecule encoding such molecules can be formulated for systemic or local administration. In one example, the, antigen binding fragment, bispecific antibody, conjugate, or nucleic acid molecule encoding such molecules, is formulated for parenteral administration, such as intravenous administration.

In some aspects, the antibody, antigen binding fragment, bispecific antibody, or conjugate thereof, in the composition is at least 70% (such as at least 75%, at least 80%, at least 85%, at least 90%, at least 95%, at least 96%, at least 97%, at least 98% or at least 99%) pure. In some aspects, the composition contains less than 10% (such as less than 5%, less than 4%, less than 3%, less than 2%, less than 1%, less than 0.5%, or even less) of macromolecular contaminants, such as other mammalian (e.g., human) proteins.

The compositions for administration can include a solution of the antibody, antigen binding fragment, multispecific antibody (such as a bispecific antibody), conjugate, or nucleic acid molecule encoding such molecules, dissolved in a pharmaceutically acceptable carrier, such as an aqueous carrier. A variety of aqueous carriers can be used, for example, buffered saline and the like. These solutions are sterile and generally free of undesirable matter. These compositions may be sterilized by any suitable technique. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of antibody in these formulations can vary widely, and will be selected primarily based on fluid volumes, viscosities, body weight and the like in accordance with the particular mode of administration selected and the subject's needs.

A typical composition for intravenous administration comprises about 0.01 to about 30 mg/kg of antibody, antigen binding fragment, bispecific antibody, or conjugate per subject per day (or the corresponding dose of a conjugate including the antibody or antigen binding fragment). Any suitable method may be used for preparing administrable compositions; non-limiting examples are provided in such publications as Remington: The Science and Practice of Pharmacy, 22nd ed., London, UK: Pharmaceutical Press, 2013. In some aspects, the composition can be a liquid formulation including one or more antibodies, antigen binding fragments, or bispecific antibodies, in a concentration range from about 0.1 mg/ml to about 20 mg/ml, or from about 0.5 mg/ml to about 20 mg/ml, or from about 1 mg/ml to about 20 mg/ml, or from about 0.1 mg/ml to about 10 mg/ml, or from about 0.5 mg/ml to about 10 mg/ml, or from about 1 mg/ml to about 10 mg/ml.

Antibodies, an antigen binding fragment thereof, a multispecific antibody (such as a bispecific antibody), or a nucleic acid encoding such molecules, can be provided in lyophilized form and rehydrated with sterile water before administration, although they are also provided in sterile solutions of known concentration. A solution including the antibody, antigen binding fragment, bispecific antibody, or a nucleic acid encoding such molecules, can then be added to an infusion bag containing 0.9% sodium chloride, USP, and typically administered at a dosage of from 0.5 to 15 mg/kg of body weight. Considerable experience is available in the art in the administration of antibody drugs, which have been marketed in the U.S. since the approval of Rituximab in 1997. Antibodies, antigen binding fragments, conjugates, or a nucleic acid encoding such molecules, can be administered by slow infusion, rather than in an intravenous push or bolus. In one example, a higher loading dose is administered, with subsequent, maintenance doses being administered at a lower level. For example, an initial loading dose of 4 mg/kg may be infused over a period of some 90 minutes, followed by weekly maintenance doses for 4-8 weeks of 2 mg/kg infused over a 30-minute period if the previous dose was well tolerated.

Controlled-release parenteral formulations can be made as implants, oily injections, or as particulate systems. For a broad overview of protein delivery systems see, Banga, Therapeutic Peptides and Proteins: Formulation, Processing, and Delivery Systems, Lancaster, PA: Technomic Publishing Company, Inc., 1995. Particulate systems include microspheres, microparticles, microcapsules, nanocapsules, nanospheres, and nanoparticles. Microcapsules contain the active protein agent, such as a cytotoxin or a drug, as a central core. In microspheres, the active protein agent is dispersed throughout the particle. Particles, microspheres, and microcapsules smaller than about 1 µm are generally referred to as nanoparticles, nanospheres, and nanocapsules, respectively. Capillaries have a diameter of approximately 5 µm so that only nanoparticles are administered intravenously. Microparticles are typically around 100 µm in diameter and are administered subcutaneously or intramuscularly. See, for example, Kreuter, Colloidal Drug Delivery Systems, J. Kreuter (Ed.), New York, NY: Marcel Dekker, Inc., pp. 219-342, 1994; and Tice and Tabibi, Treatise on Controlled Drug Delivery: Fundamentals, Optimization, Applications, A. Kydonieus (Ed.), New York, NY: Marcel Dekker, Inc., pp. 315-339, 1992.

Polymers can be used for ion-controlled release of the compositions disclosed herein. Any suitable polymer may be used, such as a degradable or nondegradable polymeric matrix designed for use in controlled drug delivery. Alternatively, hydroxyapatite has been used as a microcarrier for controlled release of proteins. In yet another aspect, liposomes are used for controlled release as well as drug targeting of the lipid-capsulated drug.

### 2. Methods of detection and diagnosis

Methods are also provided for the detection of the presence of a coronavirus spike protein *in vitro* or *in vivo.* In one example, the presence of a coronavirus spike protein is detected in a biological sample from a subject and can be used to identify a subject with a coronavirus infection. The coronavirus can be SARS-CoV-2 BA.1, BA.2, BA.4/5, BA.2.75.2, XBB.1.5, EG.5.1, FL.1.5.1, BA.2.86, JN.1, KP.2, KP.3, LB.1, KP.2.3, KP.3.1.1 and/or XEC, RaTG13, Pangolin GD, Pangolin GX-P2V, WIV1 and/or SHC-014. The coronavirus can be SARS-CoV-1, or a SARS-CoV-1 related virus from non-human animals, such as civet cats or pangolins.

The sample can be any sample, including, but not limited to, tissue from biopsies, autopsies and pathology specimens. Biological samples also include sections of tissues, for example, frozen sections taken for histological purposes. Biological samples further include body fluids, such as blood, serum, plasma, sputum, spinal fluid or urine. The method of detection can include contacting a cell or sample, with an antibody, antigen binding fragment, or multispecific antibody (such as a bispecific antibody), that specifically binds to a SARS-CoV-2 spike protein, or conjugate thereof (*e.g.*, a conjugate including a detectable marker) under conditions sufficient to form an immune complex, and detecting the immune complex (*e.g.*, by detecting a detectable marker conjugated to the antibody or antigen binding fragment.

In one aspect, the antibody, antigen binding fragment or multispecific antibody (such as a bispecific antibody), is directly labeled with a detectable marker. In another aspect, the antibody (or antigen binding fragment or bispecific antibody) that binds the SARS-CoV-2 spike protein (the primary antibody) is unlabeled and a secondary antibody or other molecule that can bind the primary antibody is utilized for detection. The secondary antibody is chosen that is able to specifically bind the specific species and class of the first antibody. For example, if the first antibody is a human IgG, then the secondary antibody may be an anti-human-IgG. Other molecules that can bind to antibodies include, without limitation, Protein A and Protein G, both of which are available commercially. Suitable labels for the antibody, antigen binding fragment, bispecific antibody or secondary antibody are known and described above, and include various enzymes, prosthetic groups, fluorescent materials, luminescent materials, magnetic agents and radioactive materials.

In some aspects, the disclosed antibodies, antigen binding fragments thereof, or multispecific antibody (such as a bispecific antibody), are used to test vaccines. For example, to test if a vaccine composition including a coronavirus spike protein or fragment thereof assumes a conformation including the epitope of a disclosed antibody. Thus, provided herein is a method for testing a vaccine, wherein the method comprises contacting a sample containing the vaccine, such as a coronavirus spike protein immunogen, with a disclosed antibody, antigen binding fragment, or bispecific antibody, under conditions sufficient for formation of an immune complex, and detecting the immune complex, to detect the vaccine including the epitope of interest in the sample. In one example, the detection of the immune complex in the sample indicates that vaccine component, such as the immunogen assumes a conformation capable of binding the antibody or antigen binding fragment.

### Overview

Clause 1: An isolated monoclonal antibody or antigen binding fragment thereof, comprising a heavy chain variable (V_{H}) region and a light chain variable region (V_{L}) comprising a heavy chain complementarity determining region (HCDR)1, a HCDR2, and a HCDR3, and a light chain complementarity determining region (LCDR)1, a LCDR2, and a LCDR3 of the V_{H} and V_{L} set forth as any one of: a) SEQ ID NOs: 1 and 5, respectively (A18-618-448.1); b) SEQ ID NOs: 9 and 13, respectively (F768-104_B2); c) SEQ ID NOs: 17 and 21, respectively (E184-105_F3); d) SEQ ID NOs: 25 and 29, respectively (A63-652-32.1); e) SEQ ID NOs: 33 and 37, respectively (F768-104_G7); g) SEQ ID NOs: 41 and 45, respectively (E184-105_H1); f) SEQ ID NOs: 49 and 53, respectively (F768-104_D10); h) SEQ ID NOs: 57 and 61, respectively (A63-652-1.4); i) SEQ ID NOs: 65 and 69, respectively (E184-105_E5); j) SEQ ID NOs: 73 and 77, respectively (E184-105_H9); k) SEQ ID NOs: 81 and 85,respectively (E184-105_H2); 1) SEQ ID NOs: 89 and 93, respectively (A80-102+103_G1); m) SEQ ID NOs: 97 and 101, respectively (A80-103_E2); n) SEQ ID NOs: 105 and 109, respectively (A80-103_B6); o) SEQ ID NOs: 113 and 117, respectively (A18-618-452.1); p) SEQ ID NOs: 121 and 125, respectively (A80-102+103_C1); q) SEQ ID NOs: 129 and 133, respectively (A43-d321.10.4); r) SEQ ID NOs: 137 and 141, respectively (F768-100-pt2_A05); s) SEQ ID NOs: 145 and 149, respectively (A80-102_G5); t) SEQ ID NOs: 153 and 157, respectively (A18-338-19.5); u) SEQ ID NOs: 161 and 165, respectively (A18-338-19.6); v) SEQ ID NOs: 169 and 173, respectively (A18-338-19.8); or w) SEQ ID NOs: 177 and 181, respectively (A18-338-19.9), wherein the monoclonal antibody or antigen binding fragment specifically binds SARS CoV)-2.

Clause 2: The isolated monoclonal antibody or antigen binding fragment of clause 1, wherein the HCDR1, the HCDR2, the HCDR3, the LCDR1, the LCDR2, and the LCDR3 comprise the amino acids sequences set forth as any one of: a) SEQ ID NOs: 2, 3, 4, 6, 7 (AAS), and 8; b) SEQ ID NOs: 10, 11, 12, 14, 15 (GAS), and 16; c) SEQ ID NOs: 18, 19, 20, 22, 23 (GAS), and 24; d) SEQ ID NOs: 26, 27, 28, 30, 31 (AAS), and 32; e) SEQ ID NOs: 34, 35, 36, 38, 39 (AAS), and 40; f) SEQ ID NOs: 42, 43, 44, 46, 47 (DAS), and 48; g) SEQ ID NOs: 50, 51, 52, 54, 55 (GAS), and 56; h) SEQ ID NOs: 58, 59, 60, 62, 63 (DAS), and 64; i) SEQ ID NOs: 66, 67, 68, 70, 71 (AAS), and 72; j) SEQ ID NOs: 74, 75, 76, 78, 79 (AAS), and 80; k) SEQ ID NOs: 82, 83, 84, 86, 87 (AAS), and 88; 1) SEQ ID NOs: 90, 91, 92, 94, 95 (NNN), and 96; m) SEQ ID NOs: 98, 99, 100, 102, 103 (SDS), and 104; n) SEQ ID NOs: 106, 107, 108, 110, 111 (SYN), and 112; o) SEQ ID NOs: 114, 115, 116, 118, 119 (AAS), and 120; p) SEQ ID NOs: 122, 123, 124, 126, 127 (DAS), and 128; q) SEQ ID NOs: 130, 131, 132, 134, 135 (DAS), and 136; r) SEQ ID NOs: 138, 139, 140, 142, 143 (WAS), and 144; s) SEQ ID NOs: 146, 147, 148, 150, 151 (DAS), and 152; t) SEQ ID NOs: 154, 155, 156, 158, 159 (DAS), and 160; u) SEQ ID NOs: 162, 163, 164, 166, 167 (DAS), and 168; v) SEQ ID NOs: 170, 171, 172, 174, 175 (DAT), and 176; or w) SEQ ID NOs: 178, 179, 180, 182, 183 (DAS), and 184, respectively.

Clause 3: The isolated monoclonal antibody or antigen binding fragment of clause 1 or clause 2, wherein the V_{H} and the V_{L} comprise the amino acid sequences at least 90% identical to the amino acid sequences set forth as any one of: a) SEQ ID NOs: 1 and 5; b) SEQ ID NOs: 9 and 13; c) SEQ ID NOs: 17 and 21; d) SEQ ID NOs: 25 and 29; e) SEQ ID NOs: 33 and 37; f) SEQ ID NOs: 41 and 45; g) SEQ ID NOs: 49 and 53; h) SEQ ID NOs: 57 and 61; i) SEQ ID NOs: 65 and 69; j) SEQ ID NOs: 73 and 77; k) SEQ ID NOs: 81 and 85; l) SEQ ID NOs: 89 and 93; m) SEQ ID NOs: 97 and 101; n) SEQ ID NOs: 105 and 109; o) SEQ ID NOs: 113 and 117; p) SEQ ID NOs: 121 and 125; q) SEQ ID NOs: 129 and 133; r) SEQ ID NOs: 137 and 141; s) SEQ ID NOs: 145 and 149; t) SEQ ID NOs: 153 and 157; u) SEQ ID NOs: 161 and 165; v) SEQ ID NOs: 169 and 173; or w) SEQ ID NOs: 177 and 181, respectively.

Clause 4: The isolated monoclonal antibody or antigen binding fragment of any one of the prior clauses, comprising a human framework region.

Clause 5: The isolated monoclonal antibody or antigen binding fragment of any one of the prior clauses, wherein the V_{H} and the V_{L} comprise the amino acid sequences set forth as any one of: a) SEQ ID NOs: 1 and 5; b) SEQ ID NOs: 9 and 13; c) SEQ ID NOs: 17 and 21;d) SEQ ID NOs: 25 and 29; e) SEQ ID NOs: 33 and 37; f) SEQ ID NOs: 41 and 45; g) SEQ ID NOs: 49 and 53; h) SEQ ID NOs: 57 and 61; i) SEQ ID NOs: 65 and 69; j) SEQ ID NOs: 73 and 77; k) SEQ ID NOs: 81 and 85; 1) SEQ ID NOs: 89 and 93; m) SEQ ID NOs: 97 and 101; n) SEQ ID NOs: 105 and 109; o) SEQ ID NOs: 113 and 117; p) SEQ ID NOs: 121 and 125; q) SEQ ID NOs: 129 and 133; r) SEQ ID NOs: 137 and 141; s) SEQ ID NOs: 145 and 149; t) SEQ ID NOs: 153 and 157; u) SEQ ID NOs: 161 and 165; v) SEQ ID NOs: 169 and 173; or w) SEQ ID NOs: 177 and 181respectively.

Clause 6: The isolated monoclonal antibody of any one of the prior clauses, wherein the monoclonal antibody comprises a human constant domain.

Clause 7: The isolated monoclonal antibody of any one of the prior clauses, wherein the monoclonal antibody is a human antibody.

Clause 8: The isolated monoclonal antibody of any one of the prior clauses, wherein the monoclonal antibody is an IgG.

Clause 9: The isolated monoclonal antibody of any one of the prior clauses, comprising a recombinant constant domain comprising a modification that increases the half-life of the monoclonal antibody.

Clause 10: The isolated monoclonal antibody of clause 9, wherein the modification increases binding to the neonatal Fc receptor.

Clause 11: The isolated monoclonal antibody or antigen binding fragment of any one of clauses 1-10, wherein the monoclonal antibody neutralizes SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 and/or SHC-014.

Clause 12: The antigen binding fragment of any one of clauses 1-5 or 11.

Clause 13: The antigen binding fragment of clause 12, wherein the antigen binding fragment is a Fv, Fab, F(ab')₂, scFV or a scFV₂ fragment.

Clause 14: The isolated monoclonal antibody or antigen binding fragment of any one of clauses 1-13, conjugated to a detectable marker.

Clause 15: A multi-specific antibody comprising the monoclonal antibody or antigen binding fragment of any one of clauses 1-14.

Clause 16: An isolated nucleic acid molecule encoding the monoclonal antibody or antigen binding fragment of any one of clauses 1-15, or a V_{H} or V_{L} of the monoclonal antibody.

Clause 17: The nucleic acid molecule of clause 16, wherein the nucleic acid molecule is a cDNA sequence encoding the V_{H} and the V_{L}.

Clause 18: The nucleic acid molecule of clause 16 or clause 17, operably linked to a promoter.

Clause 19: A vector comprising the nucleic acid molecule of any one of clauses 16-18.

Clause 20: A host cell comprising the nucleic acid molecule of any one of clauses 16-20, or the vector of clause 19.

Clause 21: A pharmaceutical composition for use in inhibiting a coronavirus infection, comprising an effective amount of the monoclonal antibody or antigen binding fragment of any one of clauses 1-14, the multispecific antibody of clause 15, the nucleic acid molecule of any one of clauses 16-18, or the vector of clause 19; and a pharmaceutically acceptable carrier.

Clause 22: A method of producing an antibody or antigen binding fragment that specifically binds to a coronavirus spike protein, comprising: expressing one or more nucleic acid molecules encoding the monoclonal antibody, antigen binding fragment of any one of clauses 1-14 in a host cell; and purifying the antibody or antigen binding fragment.

Clause 23: A method of detecting the presence of a SARS CoV-2 in a biological sample from a subject, comprising: contacting the biological sample with an effective amount of the monoclonal antibody or antigen binding fragment of any one of clauses 1-14 under conditions sufficient to form an immune complex; and detecting the presence of the immune complex in the biological sample, wherein the presence of the immune complex in the biological sample indicates the presence of the SARS CoV-2 in the sample.

Clause 24: The method of clause 23, wherein detecting the detecting the presence of the immune complex in the biological sample indicates that the subject has a SARS-CoV-2 infection.

Clause 25: A method of inhibiting a SARS CoV-2 infection in a subject, comprising administering an effective amount of the monoclonal antibody or antigen binding fragment of any one of clauses 1-14, the multispecific antibody of clause 15, the nucleic acid molecule of any one of clauses 16-18, or the vector of clause 19, or the pharmaceutical composition of clause 21 to the subject, wherein the subject has or is at risk of a SARS CoV-2 infection.

Clause 26: The method of clause 25, wherein the coronavirus is SARS-CoV-2 D614G, BA.1, BA.2, BA.4/5, BA.2.75.2, BQ.1.1, XBB.1, XBB.1.5, XBB.1.16, CH.1.1, XBB.2.3.2, XBC.1, EG.5.1, XBB.1.16.6, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.

Clause 27: Use of the monoclonal antibody, antigen binding fragment, multispecific antibody, nucleic acid molecule, vector, or pharmaceutical composition of any one of clauses1-25 to inhibit a SARS CoV-2 infection in a subject or to detect the presence of a SARS CoV-2 in a biological sample.

Clause 28: The use of clause 27,wherein the SARS CoV-2 is SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.

### EXAMPLES

Disclosed are 23 monoclonal antibodies with clinical use for COVID-19 prevention and treatment. The antibodies are potently neutralizing and target the Spike glycoprotein of SARS-CoV-2. They are more potent than other monoclonal antibodies in current clinical testing, and more cross-reactive than the antibodies previously available under EUA. Notably, they neutralize SARS-CoV-2-related virus strains from animals, and many also neutralize SARS-CoV and related animal coronaviruses, suggesting utility against new variants that may emerge, and against coronaviruses with pandemic potential.

### Example 1

### Methods

***Binding and sorting of B-cells using flow cytometry:*** SARS CoV-2 vaccinated and/or convalescent subjects were selected for antibody isolation based on high serum neutralizing antibody titers against the omicron-lineage variants BA4/5, BQ.1.1, and XBB. Memory B cells from peripheral mononuclear blood cells were identified by their binding to fluorescently-labelled SARS CoV-2 Spike RBD protein or full-length soluble Spike, and sorted into single wells. For several subjects, SARS-CoV-2 spike-specific B cells were isolated using S-2P spike proteins derived from variants BQ.1.1 and XBB. In other cases, antibodies were selected with a higher likelihood of binding to class I and III RBD epitopes that could also neutralize Omicron lineage viruses. In those cases, B cells were single sorted using RBD proteins with specific mutations.

***Sequencing, cloning and expression:*** B cell heavy and light chain nucleotide sequences were determined using next-generation sequencing approaches. The heavy and light chain sequences were cloned into expression cassette/plasmid vectors following synthesis. Expression cassettes were used to express antibodies for initial screening; further characterization was performed with larger-scale preparations using plasmid vectors. Antibodies were purified using standard methods and techniques.

***Epitope Mapping:*** Global mapping to determine mAb binding properties and epitopes on S-2P or RBD was performed by ELISA-based, Octet/BLI, or SPR competition assays using mAbs that have known RBD epitopes, including antibodies of classes I, II, III, and IV. For mapping by competition ELISA, unlabeled competitor mAbs were added to the SARS-CoV-2 WA-1 S2P-coated plate. Following incubation for 30 min at room temperature, biotinylated mAbs were added, and OD readings were recorded, using biotinylated mAb alone as a binding control. Percent inhibition of binding was calculated as follows: 100 - (reading with biotin-mAb in the presence of competing mAb)/reading with biotin-mAb alone) x100.

***Neutralization:*** Neutralization of virus infection by mAbs was determined using pseudotyped lentivirus particles bearing coronavirus Spike protein. Infection caused by the viruses was determined by measuring the expression of a luciferase reporter gene that was encoded by the virus genome. SARS-CoV-2 variants in the test panel include pre-omicron variants alpha, beta, gamma, and delta; omicron variants BA.1, BA.2, BA.4/BA.5, BA.2.75.2, BQ.1.1, XBB.1, and XBB.1.5; and recent variants XBB.1.16, CH.1., XBB.2.3.2, XBC.1, EG.5.1, XBB.1.16.6, FL.1.5.1, BA.2.86, JN.1, KP.2, KP.3, LB.1, KP.2.3, KP.3.1.1 and XEC. SARS-CoV-2-related sarbecoviruses include RaTG13-d21aa from bats and several strains from pangolins. The standard SARS-CoV strain and its relative Frankfurt 1 were used, in addition to SARS-CoV-related viruses SHC014 from bats and Civet007 from Civet cats. Fine epitope mapping was conducted using neutralization assays with panels of Spike proteins bearing point mutations in D614G background.

***Synergy:*** Antibody 1 and antibody 2 were diluted separately and mixed in a matrix format. After incubating with pseudovirus (XBB.1.5 or EG.5.1) antibody-virus mixture was added to pre-plated 293-TMPRSS2-ACE2 cells in triplicate. Plates were harvested 72 h later and percent neutralization was calculated. A web-based software "SynergyFinder3.0" (http://synergyfinder.fimm.fi) was used to calculate average and maximum ZIP synergy scores. ZIP score <-10 indicates antagonism, -10-10 indicates additivity, and >10 indicates synergistic neutralization.

***Structure:*** The molecular structure of several antibodies in complex with RBD or S-2P was determined by cryo-electron microscopy (Cryo-EM).

### Example 2

### Antibodies

Antibody 1. A18-448.1 is a highly potent and cross-reactive neutralizing antibody with activity against SARS-CoV-2 variants and SARS-CoV-related sarbecoviruses. It neutralized all tested SARS-CoV-2 variants, including potent neutralization against pre-omicron SARS-CoV-2 variants, omicron BA.1, BA.2, and BA.4/5, EG.5.1 and BA.2.86 (IC50 0.001-0.01 µg/ml) and most recent JN.1 and its subvariants including KP.3.1.1 and XEC (IC50 0.0026-0.0095ug/ml; FIG. 7). It potently neutralized SARS-CoV related sarbecoviruses from human, bat, and civet cat derivation (IC50 0.001-0.01 µg/ml) and several animal-derived SARS-CoV-2 related viruses (Table 1; FIGS. 3A-3H). Mapping studies of antibody A18-448.1 indicate an epitope of class I/IV: in competition assays using ELISA, it competes known class I mAbs including CB6, class III mAbs including LY-CoV1404 and class IV mAbs including BD55-5514 for binding to S-2P, indicating that it belonged to class IV (Table 2; FIGS. 4A-4B). Structural data were consistent with a class IV epitope (Figure 1).

Compared to previous monoclonals, A18-448.1 was more broadly-cross reactive, and in most cases also more potent. In its epitope and neutralization profile, it was similar to antibody SA55, also called BD55-5514, which currently is in clinical trials in China. Notably, A18-448.1 was more potent than SA55. Both antibodies had an identical binding competition profile. Both were unaffected by common escape mutations including F456L; their activity was abrogated by spike mutations at positions 500 and 504 (Table 3; FIGS. 5A-5C), which are very rare in the spike sequence GIAID database. Genetically, both had heavy chain genes derived from IGHV1-69, however their CDRH1, CDRH2, and CDRH3 sequences were different; and while the A18-448.1 light chain gene was derived IGKV1D-39, SA55 is derived from IGVK1-33. A18-448.1 is different from SA55.

Antibody 2. F768-104_B2 was a highly potent and cross-reactive neutralizing antibody with activity against SARS-CoV-2 variants and SARS-CoV. It showed broad and potent (IC50<0.1 µg/mL) neutralization of SARS-CoV-2 Omicron lineage pseudotyped viruses including BA.1, BA.2, BA.4/5, BA.2.75.2, XBB.1.5, EG.5.1, FL.1.5.1, and BA.2.86, and also potently neutralize most recent variants including JN.1, KP.2, KP.3, LB.1, KP.2.3, KP.3.1.1 and XEC (IC50 0.051-0.591 µg/ml). It also neutralized SARS-CoV (IC50=0.125 µg/mL) and several animal-derived SARS-CoV-2 related viruses. This breadth and potency was equivalent to or better than the previous leading authorized therapeutic antibody, LY-CoV1404 (Table 1; FIGS. 3A-3H; FIG.7).

A competition assay for F768-104_B2 was consistent with its belonging to Class III, as it competes with known Class III mAbs including Ly-CoV1404 (bebtelovimab) (Table 2; FIGS. 4A-4B). It shows reduced, but not abrogated, neutralization to pseudovirus containing K444N on the D614G spike, and still neutralizes pseudoviruses with other substitutions on the key residues for class III antibodies, such as G446V/R, V445A/R and P449R (Table 3; FIGS. 5A-5C), indicating the uniqueness of this antibody. Cryo-EM structural determination of the epitope footprint on RBD indicates that, compared to other Class III antibodies, it makes contact with more highly-conserved residues (FIG. 2); this is consistent with its superior breadth of activity across variants.

### Example 3

### Additional Antibodies

### Group 3 Antibodies: A80-19 Lineage

Antibodies A80-102_G5, A80-338-19.5, A80-338-19.6, A80-338-19.8, and A80-338-19.9 (alternate names A80-19.3, A80-19.5, A80-19.6, A80-19.8, and A80-19.9, respectively) were highly related antibodies derived from the same donor. The antibodies had neutralizing activity against SARS-CoV-2 variants and SARS-CoV-related sarbecoviruses. They were inferred to be members of a clonal lineage, *ie,* derived from a common B cell precursor in the donor. Genetically, they were derived from heavy chain gene IGHV1-3*01 and kappa light chain IGKV1D-33*01 or IGKV1-33*01. At the amino acid level, their heavy chains are 83-95% identical, and their light chains are 88-95% identical. These antibodies were able to neutralize all tested SARS-CoV-2 variants, including potent neutralization against pre-omicron SARS-CoV-2 variants (IC50 <1 µg/ml), with modest potency against omicron BA.1, BA.2, BA.5, EG.5.1, BA.2.86 as well as most recent variants including JN.1, KP.2, KP.3, LB.1, KP.2.3, KP.3.1.1 and XEC (IC50 1-10 µg/ml). They potently neutralized SARS-CoV-2 related viruses from animals, including Pangolin GX variants, as well as SARS-CoV related sarbecoviruses from human, bat, and civet cat derivation (IC50 <0.05 µg/ml) (Table 1; FIGS. 3A-3H; FIG.7). Mapping studies indicated an epitope of class I/IV (Table 2; FIGS. 4A-4B). A80-102_G5 maintained neutralization potency against a panel of D614G-based pseudoviruses bearing a variety of point mutations that impact other class I and IV antibodies, including the F456L mutation, which is a mutation seen in the majority of variants circulating in the fall of 2023 (Table 3; FIGS. 5A-5C).

### Group 4 Antibodies: Class I, genetically similar

Antibodies E184-105_F3, A63-652-32.1, F768-104_G7, E184-105_H1, F768-104_D10, and A63-652-1.4 were classified as Class I and showed broad and potent neutralization of SARS-CoV-2 Omicron lineage pseudotyped viruses. Strains including BA.1, BA.2, BA.4/5, BA.2.75.2, BQ1.1, and XBB, as well as more recent variants including EG.5.1 and BA.2.86, were potently neutralized (IC50 0.0001 - 0.35 µg/mL). These antibodies also neutralized SARS-CoV-2 related coronaviruses from bats and pangolins (Table 1; FIGS. 3A-3H). F768-104_D10 and F768-104_G7 were able to potently neutralize D614G strain bearing the F456L mutation, which is a mutation seen in the majority of strains circulating in the fall of 2023 (Table 3; FIGS. 5A-5C). E184-105_F3 was able to neutralize JN.1 and sublineages including KP.2, KP.3, KP.2.3 and LB.1(Figure. 7). A63-652-1.4 also showed potent neutralization of most recent variants including KP.3.1.1 and XEC (Figure. 7). Competition profiles of these antibodies and/or their genetics was consistent with their binding to the Class I epitope within RBD (Table 2; FIGS. 4A-4B). Genetically they were derived from IGHV3-53 or IGVH3-66, paired with IGVK1-9 or IGVK1D-33, a profile often seen in Class I antibodies. Each of these antibodies shows breadth and potency that is not seen by LY-CoV1404 or other clinically used antibodies.

### Group 5 Antibodies: Class I, diverse gene derivations

Antibodies E184-105_E5, E184-105_H9, E184-105_H2, and A80-102+103_G1 were classified as Class I and showed broad and potent neutralization of SARS-CoV-2 Omicron lineage pseudotyped viruses, including BA.1, BA.2, BA.4/5, BA.2.75.2, BQ1.1, XBB, as well as more recent variants including EG.5.1 and BA.2.86 (IC50 0.003 - 0.487 µg/mL). These antibodies also neutralized SARS-CoV-2 related coronaviruses from bats and pangolins (Table 1; FIGS. 3A-3H). E184-105_E5, E184-105_H9 also neutralized JN.1 (Figure. 7). Competition profiles of these antibodies were consistent with their binding to the Class I epitope within RBD (Table 2; FIGS. 4A-4B). Unlike many other Class I antibodies, they were genetically derived from IGVH4-39 or IGVH5-51. Each of these antibodies showed breadth and potency that was not seen by LY-CoV1404 or other clinically used antibodies. E184-105_E5 and A80-102+103_G1 were able to potently neutralize D614G strain bearing a variety of mutations known to reduce activity of other Class I antibodies, including the F456L mutation, which is a mutation seen in the majority of Omicron variants circulating in the fall of 2023 (Table 3; FIGS. 5A-5C).

### Group 6 Antibodies: Diverse genetics and epitopes

**Antibodies A80-103_E2, A80-103_B6, A18-618-452.1, A80-102+103**_**C1, A43-d321.10.4, and F768-100-pt2_A05** showed broad and potent (IC50<0.1 µg/mL) neutralization of SARS-CoV-2 Omicron lineage pseudotyped viruses including BA.1, BA.2, BA.4/5, BA.2.75.2, BQ1.1, and XBB, with modest potency against EG.5.1, FL.1.5.1, and BA.2.86 as well as most recent variants including JN.1, KP.2, KP.3, LB.1, KP.2.3, KP.3.1.1 and XEC (IC50 1-10 µg/mL). They also potently neutralize SARS-CoV-2-related coronaviruses from animals, as well as SARS-CoV (IC50 0.02-2 µg/mL) (Table 1; FIGS. 3A-3H; FIG. 7). A80-103_B6, A80-102+103_C1, and A80-103_E2 maintained neutralization potency against a panel of D614G-based pseudoviruses bearing a variety of point mutations that impact other class I and IV antibodies, including the F456L mutation, which is a mutation seen in the majority of variants circulating in the fall of 2023 (Table 3; FIGS. 5A-5C).

This group of antibodies targets a variety of antigenic sites:
Mapping data for A80-103_E2 suggests Site V (Table 2; FIGS. 4A-4B).
A80-103_B6 competed with Class IV antibodies, but not Class I, suggesting a unique mode of binding (Table 2; FIGS. 4A-4B). Further mapping is performed by antibodies A43-d321.10.4, A80-102+103_C1, and A18-618-452.1.
Sequence information is provided below:
   **Ab1. A18-618-448.1**

The amino acid sequences for the heavy and light chains of the expressed version of **A18-618-448.1** can be found in Table 1 Variable heavy and light chain sequences were synthesized and cloned into immunoglobulin expression vectors containing human constant regions for the IgG1 heavy chain and kappa light chain. These vectors were used to express antibody that was purified.

**Table 1 A18-618-448.1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1.69*04** | **IGHD2-2*01 F** | **IGHJ5*02** | |
| CAGGTCCAGCTGGTGCAGTCTGGGGCTGAGGTGAAGAAGCCTGGGTCCTCGGTGAAGGTCTC CTGCAAGGTTTCTGGAGGCACCTTCAGTGATTCTGCTGTCAGCTGGGTGCGACAGGCCCCTG GACAAGGGCTTGAGTGGATGGGAAGGATCATCCCCATCTTTCTACAATAGAATATGCACAG AACTTCCAGGGCAGAGTCACCATAACCGCGGACGAATCCACGACCACAGTCTACATGGAATT GAGTTACCTGGGACTTGAGGACACGGCCGTATATTACTGTGCGAGAGACCGTCCTATAGCAG TAGAATATACGGGGAACTGGTTCGACCCCTGGGGCCAGGGAACCCTGGTCACCGTGTCCTCA G (SEQ ID NO: 185) | | | | |
| QVQLVQSGAEVKKPGSSVKVSCKVSGGTFSDSAVSWVRQAPGQGLEWMGRIIPIFSTIEYAQNF QGRVTITADESTTTVYMELSYLGLEDTAVYYCARDRPIAVEYTGNWFDPWGQGTLVTVSS (SEQ ID NO: 1) | | | | |
| **Light Chain** | **IGKV1D-39*01, IGKV1-39*01** | | **IGKJ2*01** | |
| GACGTCCAGATGACCCAGTCTCCATCCTCCCTGTCTGCATCTGTAGGAGACAGAGTCACCAT CACTTGCCGGGCAAGTCAGACTATTGTCGGCTATTTAAATTGGTATCAGCAACAACCAGGGA GAGCCCCTAAGCTCCTGATCTATGCTGCATCCAATTTGCAAAGTGGAGTCCCTCCTAGGTTCA GTGGCAGTGGATCTGGGACAGATTTCACTCTCACCATCAGCGATCTGCAACCTGAAGATTTT GCCACTTACTACTGTCAACAGAGTTACCTTACCCCTCCGAACACTTTTGGCCAGGGGACCAA GCTGGAGATCAAGC (SEQ ID NO: 186) | | | | |
| DVQMTQSPSSLSASVGDRVTITCRASQTIVGYLNWYQQQPGRAPKLLIYAASNLQSGVPPRFSGS GSGTDFTLTISDLQPEDFATYYCQQSYLTPPNTFGQGTKLEIK (SEQ ID NO: 5) | | | | |

### Ab2. F768-104_B2

The amino acid sequences for the heavy and light chains of the expressed version of **F768-104_B2** can be found in Table 2 Variable heavy and light chain sequences were synthesized and cloned into immunoglobulin expression vectors containing human constant regions for the IgG1 heavy chain and kappa light chain. These vectors were used to express antibody that was purified.

**Table 2 F768-104_B2 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-2*04** | **IGHD3-16*01 F** | **IGHJ5*02** | |
| CAGGTGCACCTGGTGCAGTCTGGGGCTGAGGTGAAGAAGCCTGGGGCCTCAGTGAAGGTCT CCTGCAAGGCTTCGGATACACCTTCACTGACTACTTTATTCACTGGGTGCGACAGGCCCCG GGACAAGGGCTTGAGTGGATGGGGTGGATCAACCCTGACGATGGCGACACAAACTATGCAC AGAAGTTTCATGGCTGGGTCGCCATGACCAGGGACACGTCCATCAACACAGCCTACATGGAG TTGAGTAGGCTGAAATCTAATGACACGGCCGTGTATTACTGTGCGAGAGGGACCTTTTCTCT ACGTGGATTTGACTGGTTCGACCCCTGGGGCCAGGGAACCCTGGTCACCGTCTCCTCAG (SEQ ID NO: 187) | | | | |
| QVHLVQSGAEVKKPGASVKVSCKASGYTFTDYFIHWVRQAPGQGLEWMGWINPDDGDTNYA QKFHGWVAMTRDTSINTAYMELSRLKSNDTAVYYCARGTFSLRGFDWFDPWGQGTLVTVSS (SEQ ID NO: 9) | | | | |
| **Light Chain** | **IGKV3-15*01** | | **IGKJ1*01** | |
| GAAATAGTGCTGACGCAGTCTCCAGCCACCCTGTCTGTGTCTCCAGGGGAAAGAGCCACCCT CTCCTGCAGGGCCAGTCAGAGTGTTAGCAGCCTCTTAGCCTGGTACCAGCGGAAACCTGGCC AGCCTCCCAGGCTCCTCATCTATGGTGCATCCACCAGGGCCACTGGTATCCCAGCCAGGTTC AGTGGCAGTGGGTCTGGGACAGAGTTCACTCTCACCATCAGCAGCCTGCAGTCTGAAGATTT TGCAGTTTATTACTGTCAGCAGTATAATGACTGGTGGACGTTCGGCCAAGGGACCAAGGTGG AAATCAAGC (SEQ ID NO: 188) | | | | |
| EIVLTQSPATLSVSPGERATLSCRASQSVSSLLAWYQRKPGQPPRLLIYGASTRATGIPARFSGSGS GTEFTLTISSLQSEDFAVYYCQQYNDWWTFGQGTKVEIK (SEQ ID NO: 13) | | | | |

### Ab3. E184-105_F3

The amino acid sequences for the heavy and light chains of the expressed version of E184-105_F3 can be found in Table 3. Variable heavy and light chain sequences were synthesized and cloned into immunoglobulin expression vectors containing human constant regions for the IgG1 heavy chain and kappa light chain. These vectors were used to express antibody that was purified

**Table 3 E184-105_F3 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-53*01 F** | **IGHD5-24*01 ORF** | **IGHJ6*02** | |
| GAAGTGCAGCTGGTGGAGTCTGGAGGAGGCTTGATCCAGCCGGGCGGGTCCCTGCGACTCTC CTGCGCAGCCTCCGAAATCATCGTCAGTAGGAACTATATGAACTGGGTCCGCCAGGCTCCAG GGAAGGGGCTGGAGTGGGTCTCGGTTCTTTATGCCGGTGGTAGTAAATTCTATGCAGACTCT GTGAAGGGTCGATTCACCATCTCCAGAGACGATTCCAACAACACGCTGTATCTTCAAATGAG CAGCCTGAGAGTCGAGGACACGGCCATCTATTATTGTGCGAGAGGATGGGACTACTATGGTA TGGACGTCTGGGGCCAAGGGACCACGGTCACCGTCGCCTCAG (SEQ ID NO: 189) | | | | |
| EVQLVESGGGLIQPGGSLRLSCAASEIIVSRNYMNWVRQAPGKGLEWVSVLYAGGSKFYADSV KGRFTISRDDSNNTLYLQMSSLRVEDTAIYYCARGWDYYGMDVWGQGTTVTVAS (SEQ ID NO: 17) | | | | |
| **Light Chain** | **IGKV1-9*01** | | **IGKJ3*01** | |
| GACATCCAGTTGACCCAGTCTCCATCCTTCCTGTCTGCGTCTGTAGGAGACAGAGTCACCATC ACTTGCCGGGCCAGTCAGGGCATTAACAGGTATTTAGCCTGGTATCAGCAAAAACCAGGGA AAGCCCCTAAGCTCCTGATCTATGGTGCATCCACTTTGCAAAGTGGGGTCCCGTCAAGGTTC AGCGGCAGTGGATCTGGCACAGAATTCACCCTCACAATCAGCAGCCTGCAGGCTGAAGATTT TGCTACTTATTTCTGTCAACACCTTAATGATTACCCCGTTTTCGGCCCTGGGACCAAAGTGAA TATCAAAC (SEQ ID NO: 190) | | | | |
| DIQLTQSPSFLSASVGDRVTITCRASQGINRYLAWYQQKPGKAPKLLIYGASTLQSGVPSRFSGSG SGTEFTLTISSLQAEDFATYFCQHLNDYPVFGPGTKVNIK (SEQ ID NO: 21) | | | | |

### Ab4. A63-652-32.1

The amino acid sequences for the heavy and light chains of the expressed version of A63-652-32.1 can be found in Table 4. Variable heavy and light chain sequences were synthesized and cloned into immunoglobulin expression vectors containing human constant regions for the IgG1 heavy chain and kappa light chain. These vectors were used to express antibody that was purified.

**Table 4. A63-652-32.1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-53*04** | **IGHD4-23*01 ORF** | **IGHJ6*02** | |
| GAGGTGCAACTGGTGGAGTCTGGAGGAGGCTTGGTCCAGCCGGGGGGGTCCCTGAGACTCT CCTGTGCAGCCTCTGGGTCATCGTCAGCAGCAATTACATGAACTGGGTCCGCCAGGCTCCA GGGAAGGGGCTGGAGTGGGTCTCAGTTATTTATAGCGGTGGCAGCACATTCTACGCAGACTC CGTGAGGGGCCGATTCACCATCTCCAGACACGATTCCCAGAACACCCTGTTTCTTCAAATGA ACAGCCTGAGACCTGAGGACACGGCCGTGTATTATTGTGCGAGAGATACGGTGGTTTACGGG ATGGACGTCTGGGGCCAAGGGACCACGGTCATCGTCTCATCAG (SEQ ID NO: 191) | | | | |
| EVQLVESGGGLVQPGGSLRLSCAASGVIVSSNYMNWVRQAPGKGLEWVSVIYSGGSTFYADSV RGRFTISRHDSQNTLFLQMNSLRPEDTAVYYCARDTVVYGMDVWGQGTTVIVSS (SEQ ID NO: 25) | | | | |
| **Light Chain** | **IGKV1-9*01** | | **IGKJ5*01** | |
| GACATCCAGTTGACCCAGTCTCCATCCTTCCTGTCTGCATCTGTGGGAGACAGAGTCACCCTC ACTTGCCGGGCCAGTCAGGGCATTACCGCTGATTTAGCCTGGTATCAGCAAAAACCAGGGAA AGCCCCTAAGCTCCTGATCTATGCTGCATCCACTTTACAAAGTGGGGTCCCATCAAGGTTCA GCGGCAGTGGATCTGGGACAGAGTTCACTCTCACAGTCGGCAGCCTGCAGCCTGAGGATTTT GCGACTTATTACTGTCAACACCATGATACTTATTCGATCGCTTTCGGCCAAGGGACACGACT GGAGATTAAAC (SEQ ID NO: 192) | | | | |
| DIQLTQSPSFLSASVGDRVTLTCRASQGITADLAWYQQKPGKAPKLLIYAASTLQSGVPSRFSGS GSGTEFTLTVGSLQPEDFATYYCQHHDTYSIAFGQGTRLEIK (SEQ ID NO: 29) | | | | |

### Ab5. F768-104_G7

The amino acid sequences for the heavy and light chains of the expressed version of F768-104_G7can be found in Table 5. Variable heavy and light chain sequences were synthesized and cloned into immunoglobulin expression vectors containing human constant regions for the IgG1 heavy chain and kappa light chain. These vectors were used to express antibody that was purified.

**Table 5. F768-104_G7Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-53*02** | **IGHD3-16*01** | **IGHJ6*02** | |
| GAGGTGCAGCTGGTGGAGACTGGAGGAGGCTTGGTCCAGCCGGGGGGGTCCCTGAGACTCT CCTGTGCAGCCTCGGGAGTCATCGTCAGTAGGAACTACATGAGCTGGGTCCGCCAGGCTCCA GGGAAGGGGCTGGAGTGGGTCTCACTTATTTTGCCGGTGGTAGTACTTTCTACTCAGACTCC GTGAAGGGCCGATTCACCATCTCCAGAGACGATTCCAGAAACACGGTGTATCTGCAAATGAA CAGCCTGAGAGCCGAGGACACGGCCGTGTATTACTGTGCGAGAGATTTAGACGTTCGTGGCG GTTTGGACGTCTGGGGCCAAGGGACCACGGTCACCGTGTCTTCAG (SEQ ID NO: 193) | | | | |
| EVQLVETGGGLVQPGGSLRLSCAASGVIVSRNYMSWVRQAPGKGLEWVSLIFAGGSTFYSDSV KGRFTISRDDSRNTVYLQMNSLRAEDTAVYYCARDLDVRGGLDVWGQGTTVTVSS (SEQ ID NO: 33) | | | | |
| **Light Chain** | **IGKV1-39*01, IGKV1D-39*01** | | **IGKJ2*01** | |
| GACGTCCAGCTGACCCAGTTTCCATCCTCCCTGTCTGCATCTGTTGGAGACAGAGTCACCATC ACCTGCCGGGCAAGTCAGGACATTCCTAACTATATAAATTGGTATCAGCAAAAACCGGGGA AAGCCCCTAGGCTCCTGATCTATGCTGCATCCACTTTGCAAAGTGGGGTCCCATCAAGGTTC AGTGGCAGTGGATCTGGGACTGATTTCACTCTCACCATCAGCAGTCTGCAACCTGAAGATTT TGGAACTTACTACTGTCAACAGAGTGACACTAGGACTTTTGGCCCGGGGACCAAGTTGGAGA TCAAAC (SEQ ID NO: 194) | | | | |
| DVQLTQFPSSLSASVGDRVTITCRASQDIPNYINWYQQKPGKAPRLLIYAASTLQSGVPSRFSGSG SGTDFTLTISSLQPEDFGTYYCQQSDTRTFGPGTKLEIK (SEQ ID NO: 37) | | | | |

### Ab6. E184-105_H1

The amino acid sequences for the heavy and light chains of the expressed version of E184-105_H1 can be found in Table 6. Variable heavy and light chain sequences were synthesized and cloned into immunoglobulin expression vectors containing human constant regions for the IgG1 heavy chain and kappa light chain. These vectors were used to express antibody that was purified.

**Table 6. E184-105_H1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-66*01** | **IGHD6-19*01 F** | **IGHJ1*01** | |
| GAGGTGCAGCTGGTGGAGTCTGGGGGAGGCTTGGTCCAGCCTGGGGGGTCCCTGAGAGTCTC CTGTGCCGCCTCTGGACTTACCGTCAGTAGCAACTACATGACCTGGGTCCGCCAGGCTCCAG GGAAGGGGCTGGAGTGGGTCTCAGTTATTTATCCCGGTGGAAGTGCATTCTACGCAGACTCC GTGAAGGGCAGATTCAGCATCACCAGAGACAATTCCAAGAACACGTTGTATCTTCAGATGAA CAGCCTGAGAGCCGAGGACACGGCTGTGTATTATTGTGCGAGAGATCGGCCTAGCTTAGGTG TGGGTGGTGGGGGCCTGGCGCTCCGGGACTGGGGCCAGGGAACCTTGGTCACCGTCTCCTCA G (SEQ ID NO: 195) | | | | |
| EVQLVESGGGLVQPGGSLRVSCAASGLTVSSNYMTWVRQAPGKGLEWVSVIYPGGSAFYADS VKGRFSITRDNSKNTLYLQMNSLRAEDTAVYYCARDRPSLGVGGGGLALRDWGQGTLVTVSS (SEQ ID NO: 41) | | | | |
| **Light Chain** | **IGKV1D-33*01, IGKV1-33*01** | | **IGKJ4*01** | |
| GACATCCAGATGACCCAGTCTCCACCCTCCCTGTCTGCATCTGTAGGAGACAGAGTCACCAT CACTTGCCAGGCGAGTCAGGACATTAACAAGTTTTTAAATTGGTTTCAGCAGAAACCGGGGA AAGCCCCTAAGCTCCTGATCTACGATGCATCCAATGTGGAACCAGGGGTCCCGTCAAGGTTC AGTGGAAGTGGATCTGGGACAGATTTTACCTTCACCATCAGCAGCCTGCAGCCTGAAGATAT TGCAACATATTACTGTCAACAGTATGATAATCTCCCTCCGGGTTTCGGCGGAGGGACCAAGG TGGAGATCAGAC (SEQ ID NO: 196) | | | | |
| DIQMTQSPPSLSASVGDRVTITCQASQDINKFLNWFQQKPGKAPKLLIYDASNVEPGVPSRFSGS GSGTDFTFTISSLQPEDIATYYCQQYDNLPPGFGGGTKVEIR (SEQ ID NO: 45) | | | | |

### Ab7. F768-104_D10

The amino acid sequences for the heavy and light chains of the expressed version of F768-104_D10 can be found in Table 7 Variable heavy and light chain sequences were synthesized and cloned into immunoglobulin expression vectors containing human constant regions for the IgG1 heavy chain and kappa light chain. These vectors were used to express antibody that was purified.

**Table 7. F768-104_D10 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-66*01 or IGHV3-66*04** | **IGHD5-18*02 ORF** | **IGHJ6*02** | |
| GAAGTACAGCTGGTGGAGTCTGGGGGAGGCTTGGTCCAGCCTGGGGGGTCCCTGAGACTCTC CTGTGCAGCCTCTGGAATCGTCGTCAGTGCCAACTACATGACCTGGGTCCGCCAGGCTCCAG GGAAGGGGCTGGAGGGGGTCTCACTTCTTTTGCCGGTGGAAGCACATTCTACGCAGACTCC GTGAAGGGCAGATTCACGATCTCCAGAGACAATTCCAAGAACACGGTGTTTCTTCAAATGAA CAACCTGAGAGCCGAGGACACGGCTGTGTATTACTGTGCGAGGAATCTCGTTTACTACGGTA TGGACGTCTGGGGCCAGGGGACCACGGTCACCGTCTCCACAG (SEQ ID NO: 197) | | | | |
| EVQLVESGGGLVQPGGSLRLSCAASGIVVSANYMTWVRQAPGKGLEGVSLLFAGGSTFYADSV KGRFTISRDNSKNTVFLQMNNLRAEDTAVYYCARNLVYYGMDVWGQGTTVTVST (SEQ ID NO: 49) | | | | |
| **Light Chain** | **IGKV1-9*01** | | **IGKJ2*01** | |
| GACATCCAGTTGACCCAGTCTCCATCCTTCCTGTCTGCATCTGTAGGAGACAGAGTCACCATC ACTTGCCGGGCCAGTCAGGGCATTAGCGATTCTTTAGCCTGGTATCAGCAAAAACCAGGGAC AGCCCCTAAGCTCCTGATCTATGGTGCGTCCACTTTGCAAAGTGGGGTCCCATCAAGGTTCA GCGGCAGTGGATCTGCGACAGAATTCACTCTCACAATCAGCAGCCTGCAGCCTGAAGATTTT GCAACTTATTACTGTCAACAGCTTAATAGTGACTCGTACACTTTTGGCCAGGGGACCAAGCT GGAGATCAAAC (SEQ ID NO: 198) | | | | |
| DIQLTQSPSFLSASVGDRVTITCRASQGISDSLAWYQQKPGTAPKLLIYGASTLQSGVPSRFSGSG SATEFTLTISSLQPEDFATYYCQQLNSDSYTFGQGTKLEIK (SEQ ID NO: 53) | | | | |

### Ab8. A63-652-1.4

The amino acid sequences for the heavy and light chains of the expressed version of **A63-652-1.4** can be found in Table 8 Variable heavy and light chain sequences were synthesized and cloned into immunoglobulin expression vectors containing human constant regions for the IgG1 heavy chain and kappa light chain. These vectors were used to express antibody that was purified.

**Table 8 A63-652-1.4 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-66*01** | **IGHD1-26*01 F** | **IGHJ6*02** | |
| GAGGAGCAGCTGGTGGTGTCTGGGGGAGGCTTGGTCCAGCCGGGGGGGTCCCTGAGACTCT CCTGTACAGCCTCTGAAATCACCGTCATTAGGAATTACATGAACTGGGTCCGCCAGGCTCCA GGGAAGGGGCTGGAGTGGGTCTCAGTTATTTATCCCGGTGGGAGCACATTCTACGCCGACTC CGTGCAGGGCAGATTCACCATCTCCAGAGACAATTCCAAGAACACCCTGTATCTTCAAATGA ACAGCCTGAGAGCCGAGGACACGGCTGTTTATTACTGTTCGAGACCCATAGTGGGAGGTCGC CGGGGTATGGACGTCTGGGGCCAAGGGACCACGGTCACCGTCTCCTCAG (SEQ ID NO: 199) | | | | |
| EEQLVVSGGGLVQPGGSLRLSCTASEITVIRNYMNWVRQAPGKGLEWVSVIYPGGSTFYADSV QGRFTISRDNSKNTLYLQMNSLRAEDTAVYYCSRPIVGGRRGMDVWGQGTTVTVSS (SEQ ID NO: 57) | | | | |
| **Light Chain** | **IGKV1D-33*01, IGKV1-33*01** | | **IGKJ2*01** | |
| GACATCCAGATGACCCAGTCTCCATCCTCCCTGTCTGCATCTGTAGGAGACAGAGTCACCAT CACTTGCCAGGCGAGTCAGGACATTAACAACTTTTTAAATTGGTATCAACAGAAACCAGGGA AAGCCCCTAAACTCCTGATCTACGATGCATCCAATTTGGAAACAGGGGTCCCATCAAGGTTC AGTGGAAGTGGGTCTGGGACAGATTTTACTTTCACCATCATCAGCCTGCAGCCTGAAGATAT TGCAACATATTACTGTCAACACTATGATAATCCCCCGTACACTTTTGGCCAGGGGACCAAGC TGGAGATCAAAC (SEQ ID NO: 200) | | | | |
| DIQMTQSPSSLSASVGDRVTITCQASQDINNFLNWYQQKPGKAPKLLIYDASNLETGVPSRFSGS GSGTDFTFTIISLQPEDIATYYCQHYDNPPYTFGQGTKLEIK (SEQ ID NO: 61) | | | | |

### Ab9. E184-105_E5

The amino acid sequences for the heavy and light chains of the expressed version of **E184-105_E5** can be found in Table 9 Variable heavy and light chain sequences were synthesized and cloned into immunoglobulin expression vectors containing human constant regions for the IgG1 heavy chain and kappa light chain. These vectors were used to express antibody that was purified.

**Table 9. E184-105_E5 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV4-39*01** | **IGHD2-21*01 F** | **IGHJ4*02** | |
| CAGCTGCAGCTGCAGGAGTCGGGCCCAGGACTGGTGAAGCCTTCGGAGACCCTGTCCCTCAC CTGCACTGTCTCTGGTGGCTCCATCAGCAGGACTAGTTACTACTGGGGCTGGATCCGCCAGA CCCCAGGGAAGGGGCTGGAGTGGATTGGGCTATTTTTATAGTGGGAGCACCCACTACAAC CCGTCCCTCAAGAGTCGAGTCACCATATCCGTAGACACGTCCAAAGACCAGTTCTCCCTGAA GCTGAGCTCTGTGACCGCCGCGGACACGGCTATCTATTATTGTACGAGACAAATGGGCTTGA TTGACTACTGGGGCCAGGGAACCCTGGTCACCGTCTCCTCAG (SEQ ID NO: 201) | | | | |
| QLQLQESGPGLVKPSETLSLTCTVSGGSISRTSYYWGWIRQTPGKGLEWIGAIFYSGSTHYNPSL KSRVTISVDTSKDQFSLKLSSVTAADTAIYYCTRQMGLIDYWGQGTLVTVSS (SEQ ID NO: 65) | | | | |
| **Light Chain** | **IGKV1-NL1*01** | | **IGKJ3*01** | |
| GACATCCAGATGACCCAGTCTCCATTCTCCCTGTCTGCATCTGTGGGAGACAGAGTCACCAT CACTTGCCGGGCGAGTCAGGGCATTGGCAATTCTTTAGCCTGGTATCAGCAGAGACCAGGGA AAGCCCCTAAGCTCCTGCTCTATGCTGCATCCACATTGGAAAGTGGAGTCCCATCCAGGTTC GGTGGCAGTGGATCTGGGACGGATTTCACTCTCACCATCAGCAGCCTGCAGCCTGAAGATTT TGCAACTTATTACTGTCAACAGTATTATAGTACCCCTCCATTCACTTTCGGCCCTGGGACCAA GGTGGATTTCAAAC (SEQ ID NO: 202) | | | | |
| DIQMTQSPFSLSASVGDRVTITCRASQGIGNSLAWYQQRPGKAPKLLLYAASTLESGVPSRFGGS GSGTDFTLTISSLQPEDFATYYCQQYYSTPPFTFGPGTKVDFK (SEQ ID NO: 69) | | | | |

### Ab10. E184-105_H9

The amino acid sequences for the heavy and light chains of the expressed version of **E184-105_H9** can be found in Table 10 Variable heavy and light chain sequences were synthesized and cloned into immunoglobulin expression vectors containing human constant regions for the IgG1 heavy chain and kappa light chain. These vectors were used to express antibody that was purified.

**Table 10 E184-105_H9 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV4-39*01** | **IGHD2-8*01 F** | **IGHJ4*02** | |
| CAGTTGCAGCTGCAGGAGTCGGGCCCAGGACTGGTGAAGCCTTCGGAGACCCTGTCCCTCAC CTGCACTGTCTCTGGTGACTCCATCAGCAGGAGTAGTTATTACTGGGGCTGGGTCCGCCAGC CCCCAGGGAAGGGGCTGGAGTGGATTGGAAATATCTATTATAGTGGGACCACCTACTTCAAC CCGTCCCTCCAGAGTCGAGTCACCATATCCGTAGACACGTCCAAGAATCAGTTTTCCCTGAA GCTGAGCTCTGTGACCGCCGCAGACACGGCTGTGTATTACTGTGCGAGACAAATGGGCTTCG TTGACTACTGGGGCCAGGGAACCCTGGTCACCGTCTCCTCAG (SEQ ID NO: 203) | | | | |
| QLQLQESGPGLVKPSETLSLTCTVSGDSISRSSYYWGWVRQPPGKGLEWIGNIYYSGTTYFNPSL QSRVTISVDTSKNQFSLKLSSVTAADTAVYYCARQMGFVDYWGQGTLVTVSS (SEQ ID NO: 73) | | | | |
| **Light Chain** | **IGKV1-NL1*01** | | **IGKJ3*01** | |
| GACATCCAGATGACCCAGTCTCCATCCTCCCTGTCTGCATCTGTAGGAGACAGAGTCACCAT CACTTGCCGGGCGAGTCAGGGCATTAGCAATTCTTTAGCCTGGTATCAGCAGAAACCAGGGA GAGCCCCTAAGTTTCTGCTCTATGCTGCATCCACATTGGAAAGTGGGGTCCCATCCAGGTTCA GTGGCAGTGGATCTGAGACGGATTTCACTCTCACCATCAGCAGCCTGCAGCCTGAAGATTTT GCAACTTATTACTGTCAACAGTATTATAGTCCCCCTCCGTTCACTTTCGGCCCTGGGACCAAA GTGGATATCAAAC (SEQ ID NO: 204) | | | | |
| DIQMTQSPSSLSASVGDRVTITCRASQGISNSLAWYQQKPGRAPKFLLYAASTLESGVPSRFSGSG SETDFTLTISSLQPEDFATYYCQQYYSPPPFTFGPGTKVDIK (SEQ ID NO: 77) | | | | |

### Ab11. E184-105_H2

The amino acid sequences for the heavy and light chains of the expressed version of **E184-105_H2** can be found in Table 11 Variable heavy and light chain sequences were synthesized and cloned into immunoglobulin expression vectors containing human constant regions for the IgG1 heavy chain and kappa light chain. These vectors were used to express antibody that was purified.

**Table 11 E184-105_H2 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV4-39*01** | **IGHD2-8*01 F** | **IGHJ4*03** | |
| CAGCTGCAGTTGCAGGAGTCGGGCCCAGGACTGGTGAAGCCTTCGGAGACCCTGTCCCTCAC CTGCAGTGTCTCTGGTGGCTCCATCAGCAGTAGTAGTTACTACTGGGGCTGGATCCGCCAGC CCCCTGGGAAGGGGCTGGAGTGGATTGGAATATGTTTATAGTGGGAGCACCTACTACAAC CCGTCCCTCAAGAGTCGAGTCACCATATCCGTAGACACGTCCCAGAATCAAGTCTCCCTGAA GTTGAGCTCTGTGACCGCCTCAGACACGGCTGTATATTACTGTACGAGACAAATGGGCTTCG TTGACTACTGGGGCCAGGGAACCCTGGTCACCGTCTCCTCAG (SEQ ID NO: 205) | | | | |
| QLQLQESGPGLVKPSETLSLTCSVSGGSISSSSYYWGWIRQPPGKGLEWIGNMFYSGSTYYNPSL KSRVTISVDTSQNQVSLKLSSVTASDTAVYYCTRQMGFVDYWGQGTLVTVSS (SEQ ID NO: 81) | | | | |
| **Light Chain** | **IGKV1-NL1*01** | | **IGKJ3*01** | |
| GACATCCAGCTGACCCAGTCTCCATCCTCCCTGTCTGCATCTGTGGGAGACAGAGTCACCAT CACTTGCCGGGCGAGTGAGGGCATTAGCAATTCTTTAGCCTGGTATCAGCACAAACCAGGGA AAGCCCCTAGGTTCCTCCTCTATGCTGCATCCACGTTGGAGAGTGGGGTCCCTTCCAGGTTCA GTGGCAGTGGATCTGGGACGGATTTCACTCTCACCATCAGCGGCCTGCAGCCTGAAGACTTT GCAACTTATTACTGTCAACAATATTATAGTAGCCCTCCATTCACTTTCGGCCCTGGGACCACT GTGGATATCAGAC (SEQ ID NO: 206) | | | | |
| DIQLTQSPSSLSASVGDRVTITCRASEGISNSLAWYQHKPGKAPRFLLYAASTLESGVPSRFSGSG SGTDFTLTISGLQPEDFATYYCQQYYSSPPFTFGPGTTVDIR (SEQ ID NO: 185) | | | | |

### Ab12. A80-102+103_G1

The amino acid sequences for the heavy and light chains of the expressed version of **A80-102+103**_**G1** can be found in Table 12 Variable heavy and light chain sequences were synthesized and cloned into immunoglobulin expression vectors containing human constant regions for the IgG1 heavy chain and kappa light chain. These vectors were used to express antibody that was purified.

**Table 12 A80-102+103_G1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV5-51*01** | **IGHD3-10*01** | **IGHJ4*02** | |
| GAGGTGCAGCTGGTGCAGTCTGGAGCAGAGGTGAAAAAGCCCGGGGAGTCTCTGAAGATCT CCTGTAAGGGTTCTGGATACAGCTTTAGCAACTACTGGATCGGCTGGGTGCGCCAGATGCCC GGGAAAGGCCTGGAGTGGATGGGGATCGTCTATCCTTATGACTCTGATACCAGGTACAGCCC GTCCTTCCAAGGCCAGGTCACCATCTCAGCCGACAAGTCCCTCACCACCGCCTACCTGCAGT GGAGCAGTGTGAAGGCCTCGGACACCGCCATCTATTACTGTGCGAGACATGGGCATTACTAT GGTTCGGGGAGTTATGATGGCCCTGACTACTGGGGCCAGGGAACCCTGGTCACCGTCTCCTC AG (SEQ ID NO: 207) | | | | |
| EVQLVQSGAEVKKPGESLKISCKGSGYSFSNYWIGWVRQMPGKGLEWMGIVYPYDSDTRYSPS FQGQVTISADKSLTTAYLQWSSVKASDTAIYYCARHGHYYGSGSYDGPDYWGQGTLVTVSS (SEQ ID NO: 89) | | | | |
| **Light Chain** | **IGLV1-44*01** | | **IGKJ3*02** | |
| CAGTCTGTGCTGACTCAGCCACCCTCAGCGTCTGGGCCCCCGGGCAGAGGGTCACCATTTC TTGTTCTGGAAGCAGCTCCAACATCGGAGGTAATACTGTAAACTGGTACCAGCAGCTCCCAG GAACGGCCCCCAAACTCCTCATCTATAATAATAATCAGCGGCCCTCAGGGGTCCCTGACCGA TTCTCTGGCTCCAAGTCTGGCACCTCAGCCTCCCTGACCATCAGTGGGCTCCAGTCTGAGGAT GAGGCTGATTATTACTGTGCAGCATGGGATGACAGCCTGAATGGTCAAGTGTTCGGCGGAGG GACCAAGCTGACCGTCCTGA (SEQ ID NO: 208) | | | | |
| QSVLTQPPSASGPPGQRVTISCSGSSSNIGGNTVNWYQQLPGTAPKLLIYNNNQRPSGVPDRFSGS KSGTSASLTISGLQSEDEADYYCAAWDDSLNGQVFGGGTKLTVL (SEQ ID NO: 93) | | | | |

### Ab13. A80-103_E2

The amino acid sequences for the heavy and light chains of the expressed version of **A80-103**_**E2** can be found in Table 13 Variable heavy and light chain sequences were synthesized and cloned into immunoglobulin expression vectors containing human constant regions for the IgG1 heavy chain and kappa light chain. These vectors were used to express antibody that was purified.

**Table 13 A80-103_E2 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-7*04** | **IGHD6-19*01 F** | **IGHJ6*02** | |
| GAGGTGCAGTTGGTGGAGTCTGGGGGAAACTTGGTCCAGCCGGGGGGGTCCCTGAGACTCTC CTGTGCAGCCTCTGGATTCACCTTTAGTAGCTATTGGATGACCTGGGTCCGCCGGGCTCCAGG TAAAGGGCTGGAGTGGGTGGCCAATATAAAGGAAGATGGAAGTGAGAAATACTATGTGGAC TCTGTGAAGGGCCGATTCACCATCTCCAGAGACAACGCCAAGAACTCACTGTATCTGCACAT GAACAGCCTGAGAGGCGAGGACACGGCTGTGTATTACTGTGCGAGGGATCCCCATACCAAT GGCTGGTACAGGGAAAAGTACTACAACGGTATGGACGTCTGGGGCCAAGGGACCACGGTCA CCGTCTCCTCAG (SEQ ID NO: 209) | | | | |
| EVQLVESGGNLVQPGGSLRLSCAASGFTFSSYWMTWVRRAPGKGLEWVANIKEDGSEKYYVD SVKGRFTISRDNAKNSLYLHMNSLRGEDTAVYYCARDPHTNGWYREKYYNGMDVWGQGTT VTVSS (SEQ ID NO: 97) | | | | |
| **Light Chain** | **IGLV3-21*01** | | **IGKJ3*02** | |
| TCCTATGTGCTGACTCAGCCACCCTCAATGTCAGTGGCCGCAGGAAAGACGGCCAAGATTAC CTGTGGGGGAAACAACATTGGAAGTAAAAGTGTGCACTGGTACCAGCAGAAGCCAGGCCAG | | | | |
| GCCCCTGTATTGGTCATCTATTCTGATAGCGACCGGCCCTCAGGGATCCCTGAGCGATTCTCT GGCTCCAACTCTGGGAACACGGCCACCCTGACCATCAGCAGGGTCGAAGTCGAAGCCGGCG ATGAGGCCGACTATTACTGTCAGGTGTGGGATAGTAGTAGTGATCATTGGGTGTTCGGCGGA GGGACCAAGCTGACCGTCCTAG (SEQ ID NO: 210) | | | | |
| SYVLTQPPSMSVAAGKTAKITCGGNNIGSKSVHWYQQKPGQAPVLVIYSDSDRPSGIPERFSGSN SGNTATLTISRVEVEAGDEADYYCQVWDSSSDHWVFGGGTKLTVL (SEQ ID NO: 101) | | | | |

### Ab14. A80-103_B6

The amino acid sequences for the heavy and light chains of the expressed version of **A80-103**_**B6** can be found in Table 14. Variable heavy and light chain sequences were synthesized and cloned into immunoglobulin expression vectors containing human constant regions for the IgG1 heavy chain and kappa light chain. These vectors were used to express antibody that was purified.

**Table 14 A80-103_B6 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-9*01** | **IGHD3-10*01** | **IGHJ4*02** | |
| GAAGTGCAGCTGGTGGAGTCTGGGGGAAATTTGGTACAGCCTGGCAGGTCCCTGAGACTCTC CTGTGCAGCCTCTGGATTCACCTTTGATGATAATGCCATGCACTGGGTCCGGCAAGCTCCAG GGAAGGGCCTGGAGTGGGTCTCAAGTATTAATTGGAGTGGTGGTGACATAGCCTATGCGGAC TCTGTGAAGGGCCGATTCACCATCTCCAGAGACAACGCCAAGAACTCCCTCTATTTGCAAAT GAACAGTCTGAGAGCTGACGATACGGCCTTGTATTACTGTGCAAAAGGACTGTCGGGGAA ATATTATTTCCACTGGACTATTGGGGCCAGGGAACCCTGGTCACCGTCTCCTCAG (SEQ ID NO: 211) | | | | |
| EVQLVESGGNLVQPGRSLRLSCAASGFTFDDNAMHWVRQAPGKGLEWVSSINWSGGDIAYAD SVKGRFTISRDNAKNSLYLQMNSLRADDTALYYCAKGTVGEILFPLDYWGQGTLVTVSS (SEQ ID NO: 105) | | | | |
| **Light Chain** | **IGLV1-44*01** | | **IGLJ3*02** | |
| CAGTCTGTGCTGACTCAGCCACCCTCAGCGTCTGGGACCCCCGGGCAGAGGGTCACCATCTC TTGTTCTGGAAGCAGCTCCAACGTCGGAACTTATACTGTAAACTGGTACCAGCAGCTCCCAG GAACGGCCCCCAAACTCCTCGTCTACAGTTATAATCACCGGCCCTCAGGGGTCCCTGACCGA TTCTCTGGCTCCAAGTCTGGCACCTCAGCCTCCCTGGCCATCAGTGGGCTCCAGTCTGAGGAT GAGGCTGATTATTACTGTGCAGCATGGGATGACAGCCTGAATGGTTGGGTGTTCGGCGGAGG GACCAAGCTGAGCGTCCTGG (SEQ ID NO: 212) | | | | |
| QSVLTQPPSASGTPGQRVTISCSGSSSNVGTYTVNWYQQLPGTAPKLLVYSYNHRPSGVPDRFSG SKSGTSASLAISGLQSEDEADYYCAAWDDSLNGWVFGGGTKLSVL (SEQ ID NO: 109) | | | | |

### Ab15. A18-618-452.1

The amino acid sequences for the heavy and light chains of the expressed version of **A18-618-452.1** can be found in Table 15 Variable heavy and light chain sequences were synthesized and cloned into immunoglobulin expression vectors containing human constant regions for the IgG1 heavy chain and kappa light chain. These vectors were used to express antibody that was purified.

**Table 15 A18-618-452.1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-33*01 or IGHV3-33*05** | **IGHD4-23*01 ORF** | **IGHJ2*01** | |
| CAGGGACAACTGGTGGAGTCTGGGGGAGGCGTGGTCCAGCCTGGGAGGTCCCTGAGACTCT CCTGTGCAGCGTCTGGATTCACCTTCAGTAGTTATGCCATGCACTGGGTCCGCCAGACTCCAG GCAAGGGGCCGGAGTGGGTTGCTCTTATATCGGATGATGGCAGGATGAAAATGTATGAAGA CTCTGTGAAGGGCCGATTCATCATCTCCAGAGACAATCCCAAGAACACTCTGCATCTTCAAC TGAACACCCTGAGAGCCGAGGACACGGCTGTGTATTATTGTACGAGAGAAGACTCCAGAGG TCTTTACTGGTACTTCGATCTCTGGGGCCGTGGCACCCTGGTCACTGTCTCCTCAG (SEQ ID NO: 213) | | | | |
| QGQLVESGGGVVQPGRSLRLSCAASGFTFSSYAMHWVRQTPGKGPEWVALISDDGRMKMYED SVKGRFIISRDNPKNTLHLQLNTLRAEDTAVYYCTREDSRGLYWYFDLWGRGTLVTVSS (SEQ ID NO: 113) | | | | |
| **Light Chain** | **IGKV1D-39*01, IGKV1-39*01** | | **IGKJ1*01** | |
| GACATCCAGATGACCCAGTCTCCATCCTCCCTGTCTGCATCTGTAGGAGACAAAATCACGAT CACTTGCCGGGCAAGTCAGACCATTGGCATCTATTTAAACTGGTATCAACAAAAACCAGGGA AAGGCCCTAAGCTCCTGGTCTATGCTGCATCCAATTTGCACACTGGCGTCCCATCAAGGTTCA GTGGCAGTGGATCTGGGACAGATTTCACTCTCACCATCAGCAGTCTGCAACCTGAAGATTTT GCTCTCTACTACTGTCAACAGAGTTACATTACCCCTCAAGGGACGTTCGGCCAAGGGACCAA GGTGGAGATCAAAC (SEQ ID NO: 214) | | | | |
| DIQMTQSPSSLSASVGDKITITCRASQTIGIYLNWYQQKPGKGPKLLVYAASNLHTGVPSRFSGSG SGTDFTLTISSLQPEDFALYYCQQSYITPQGTFGQGTKVEIK (SEQ ID NO: 117) | | | | |

### Ab16. A80-102+103_C1

The amino acid sequences for the heavy and light chains of the expressed version of **A80-102+103**_**C1** can be found in Table 16 Variable heavy and light chain sequences were synthesized and cloned into immunoglobulin expression vectors containing human constant regions for the IgG1 heavy chain and kappa light chain. These vectors were used to express antibody that was purified.

**Table 16 A80-102+103_C1 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-30*04, IGHV3-30-3*01, IGHV3-30-3*03,** | **IGHD6-6*01 F** | **IGHJ4*02** | |
| CAGGAGCACCTGGTGGAGTCTGGGGGAGGCGTGGTCCAGCCTGGGAGGTCCCTGAGACTCT CTTGTGCAGTCTCTGGATTCACCTTCAGTCGCTATGCAATGCACTGGGTCCGCCAGGCTCCAG GCAAGGGGCTGGAGTGGGTGGCAGTTATCTCAGATGATGGAAGAACTAAATACTACGGAGA CTCCGCGGAGGGCCGATTCACCATCTCCAGAGACAATTCCAAGAACACACTGTTTCTGCAAA TGAATAGCCTGAGGGCTGACGACACGGCTGTGTTTTACTGTGCGAGAGATCGATGGCACCGC GTAACTCCCGCTTTTTATTACTTTGACCTCTGGGGCCAGGGAACCCTGGTCACCGTCTCCTCA G (SEQ ID NO: 215) | | | | |
| QEHLVESGGGVVQPGRSLRLSCAVSGFTFSRYAMHWVRQAPGKGLEWVAVISDDGRTKYYGD SAEGRFTISRDNSKNTLFLQMNSLRADDTAVFYCARDRWHRVTPAFYYFDLWGQGTLVTVSS (SEQ ID NO: 121) | | | | |
| **Light Chain** | **IGKV3-15*01, IGKV3D-15*01** | | **IGKJ2*01** | |
| GAAGTAGTGATGACGCAGTCTCCAGCCACCCTGTCTGTGTCTCCAGGGGAAAGAGCCACCCT CTCCTGTAGGGCCGGTCAGAGTGTTAACAGCAACTTAGCCTGGTACCAGCATAAACCTGGCC AGGCTCCCAGGCTCCTCATCTATGATGCATCCACCAGGGCCACTGGTATCCCAGCCAGGTTC AGTGGCAGTGGGTCTGGGACAGACTTCACTCTCACCATCAGCAGCCTGCAGTCTGACGATTT TGCACTTTATTACTGTCAGCAGTATGATAACTGGTACACTTTTGGCCAGGGGACCAAGCTGG AGATCAAAC (SEQ ID NO: 216) | | | | |
| EVVMTQSPATLSVSPGERATLSCRAGQSVNSNLAWYQHKPGQAPRLLIYDASTRATGIPARFSGS GSGTDFTLTISSLQSDDFALYYCQQYDNWYTFGQGTKLEIK (SEQ ID NO: 125) | | | | |

### Ab17. A43-d321.10.4

The amino acid sequences for the heavy and light chains of the expressed version of A43-d321.10.4 can be found in Table 17. Variable heavy and light chain sequences were synthesized and cloned into immunoglobulin expression vectors containing human constant regions for the IgG1 heavy chain and kappa light chain. These vectors were used to express antibody that was purified.

**Table 17 A43-d321.10.4 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-30-5*1,IGHV3-30*18** | **IGHD2-15*1** | **IGHJ4*03** | |
| CAGGTGCAGCTGGTGGAGTCTGGGGGAGGCGTGGTCCAGCCTGGGAAGTCCCTGAGACTCTC CTGTGCAGCCTCTGGATTCACCTTCAGAAACTATGGCATGGTCTGGGTCCGCCAGGCTCCAG GCAAGGGGCTGGAGTGGGTGGCAGATATAGTCTATGATGGAAGTGATAAATACTATGCAGA CTCCGTGAAGGGCCGATTCACCATCTCCAGAGACAATGCCAAGAACACGTTGTATTTGCAAA TGAACAGCCTGAGAACTGAGGACACGGCTGTGTATTTCTGCGCGAAACGCGGCGGCCCATAT TGTAGTGCTGGTCCCTGCTACGGAGGCTACTTTGACTACTGGGGCCAGGGATCCCTGGTCAC CGTCTCCTCAG (SEQ ID NO: 217) | | | | |
| QVQLVESGGGVVQPGKSLRLSCAASGFTFRNYGMVWVRQAPGKGLEWVADIVYDGSDKYYA DSVKGRFTISRDNAKNTLYLQMNSLRTEDTAVYFCAKRGGPYCSAGPCYGGYFDYWGQGSLV TVSS (SEQ ID NO: 129) | | | | |
| **Light Chain** | **IGKV1D-33*1,IGKV1-33*1** | | **IGKJ4*1** | |
| GACATGCAGATGACCCAGTCTCCATCCTCCCTGTCTGCATCTGTAGGAGACAGAGTCACTAT CACTTGCCAGGCGAGTCAGGACATTAGCAACTATTTAAATTGGTATCAGCAGAAACCAGGGA AAGCCCCTAAGCTCCTGATCTACGATGCATCCAATTTGGAAACAGGTGTCCCATCAAGGTTC AGTGGAAGTGGATCTGGGACAGATTTTACTTTCACCATCGGCAGCCTGCAGCCTGAAGACTT TGCAACATATTACTGTCAACAGTATGGTGATCTCCCGGTCACTTTCGGCGGAGGGACCAAGG TGGAGATCAAAC (SEQ ID NO: 218) | | | | |
| DMQMTQSPSSLSASVGDRVTITCQASQDISNYLNWYQQKPGKAPKLLIYDASNLETGVPSRFSGS GSGTDFTFTIGSLQPEDFATYYCQQYGDLPVTFGGGTKVEIK (SEQ ID NO: 133) | | | | |

### Ab18. F768-100-pt2_A05

The amino acid sequences for the heavy and light chains of the expressed version of **F768-100-pt2_A05** can be found in Table 18 Variable heavy and light chain sequences were synthesized and cloned into immunoglobulin expression vectors containing human constant regions for the IgG1 heavy chain and kappa light chain. These vectors were used to express antibody that was purified.

**Table 18 F768-100-pt2_A05 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV3-49*03** | **IGHD2-15*01** | **IGHJ4*02** | |
| GAGGCGCAGCTGGTGGAGTCTGGGGGAGGCTTGGTACAGCCAGGGCGGTCCCTGAGACTCT CCTGTACAGCTTCTGGATTCACCTTTGGTGATTCTGCCATGAGCTGGTTCCGCCAGGCTCCAG GGAAGGGGCTGGAGTGGGTAGGTTTCATTAGAAGCAAAGCTTGGGGTGGGACAACAGACTA CGCCGCGTCTGTGAAAGGCAGATTCACCATCTCAAGAGATGATTCCAAAAGCATCGCCTATC TGCAAATGAACAGCCTGAAAACCGAGGACACAGCCGTCTATTACTGTACTAGAGATCGTCGT TGTAGTGGTCTTAGCTGCTACCTCCTTGACTTCTTGGGCCAGGGAACCCTGGTCACCGTCTCC TCAG (SEQ ID NO: 219) | | | | |
| EAQLVESGGGLVQPGRSLRLSCTASGFTFGDSAMSWFRQAPGKGLEWVGFIRSKAWGGTTDY AASVKGRFTISRDDSKSIAYLQMNSLKTEDTAVYYCTRDRRCSGLSCYLLDFLGQGTLVTVSS (SEQ ID NO: 137) | | | | |
| **Light Chain** | **IGKV4-1*01** | | **IGKJ2*01** | |
| GACATCGTGATGACCCAGTCTCCAGACTCCCTGGCTGTGTCTCTGGGCGAGAGGGCCACCAT CAACTGCAAGTCCAGCCAGAGTGTTTTAGACAGCTCCAACAATAGGAACTACTTAGCTTGGT ACCACCAGAAACCGGGACAGCCTCCTAAGCTGCTCATTTACTGGGCATCTACCCGGGAGTCC GGGGTCCCTGACCGATTCAGTGGCAGCGGGTCTGGGACAGATTTCACTCTCACCATCAGCAG CCTGCAGGCTGAAGATGTGGCAGTTTATTACTGTCAGCAATATTATAGTACTCCTCAGACTTT TGGCCAGGGGACCAAGCTGGAGATCATAC (SEQ ID NO: 220) | | | | |
| DIVMTQSPDSLAVSLGERATINCKSSQSVLDSSNNRNYLAWYHQKPGQPPKLLIYWASTRESGVP DRFSGSGSGTDFTLTISSLQAEDVAVYYCQQYYSTPQTFGQGTKLEII (SEQ ID NO: 141) | | | | |

### Ab19. A80-102_G5

The amino acid sequences for the heavy and light chains of the expressed version of **A80-102_G5** can be found in Table 19 Variable heavy and light chain sequences were synthesized and cloned into immunoglobulin expression vectors containing human constant regions for the IgG1 heavy chain and kappa light chain. These vectors were used to express antibody that was purified.

**Table 19 A80-102_G5 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-3*01** | **IGHD3-22*01** | **IGHJ1*01** | |
| CAGGTCCAGCTTGTGCAGTCTGGGGCTGAGGTGAAGAAGCCTGGGGCCTCAGTGAAGGTTTC CTGCAAGGCTTCTGGATACAGCTTCACTACTAACTATGCCATGCATTGGGTGCGCCAGGCCC CCGGACAAAGGCTTGAGTGGATGGGATGGATCAACGTTGGCAATGATAACACAAAATATTC ACAGAAGTTCCAGGGCAGAGTCACCATTACCAGGGACACATCCGCGAGCACAGTCAACATG GAGCTGAGCAGCCTGAGATCTGAAGACACGGCTGTATACTACTGTGCCCGATCAACCCCTTT | | | | |
| CTATGATAGGAGGGGTTTTTACTTTCTGGGTTACTTCCAGCACTGGGGCCAGGGCACTCTGGT CACCGTCTCCTCAG (SEQ ID NO: 221) | | | | |
| QVQLVQSGAEVKKPGASVKVSCKASGYSFTTNYAMHWVRQAPGQRLEWMGWINVGNDNTK YSQKFQGRVTITRDTSASTVNMELSSLRSEDTAVYYCARSTPFYDRRGFYFLGYFQHWGQGTL VTVSS (SEQ ID NO: 145) | | | | |
| **Light Chain** | **IGKV1D-33*01, IGKV1-33*01** | | **IGKJ4*01** | |
| GACATCCAGATGACCCAGTCTCCATCCTCCCTGTCTGCATCTGTAGGAGACAGAGTCACCAT CACTTGCCAGGCGAGTCAGGACATTAGCAACTATTTAAATTGGTATCAGCAGAAACCAGGGA AAGCCCCTAAGGTCCTGATCTCCGATGCATCCAATTTGGAAACAGGGGTCCCGTCAAGGTTC AGTGGAAGTGGATCTGGGACAGATTTTATTTTCACCATCAGCAGCCTGCAGCTTGAGGATGT TGCAACATATTACTGTCAACAACATGATAATGTCCCTCTCACTTTCGGCGGAGGGACCAAGG TGGAGATCAAAC (SEQ ID NO: 222) | | | | |
| DIQMTQSPSSLSASVGDRVTITCQASQDISNYLNWYQQKPGKAPKVLISDASNLETGVPSRFSGSG SGTDFIFTISSLQLEDVATYYCQQHDNVPLTFGGGTKVEIK (SEQ ID NO: 149) | | | | |

### Ab20. A80-338-19.5

The amino acid sequences for the heavy and light chains of the expressed version of **A80-338-19.5** can be found in Table 20 Variable heavy and light chain sequences were synthesized and cloned into immunoglobulin expression vectors containing human constant regions for the IgG1 heavy chain and kappa light chain. These vectors were used to express antibody that was purified.

**Table 20 A80-338-19.5 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-3*01** | **IGHD3-22*01** | **IGHJ1*01** | |
| CAGGTCCAGCTTGTGCAGTCTGGGGCTGAGGTGAAGAAGCCTGGGGCCTCAGTGAAGGTTTC CTGCAAGGCTTCTGGATACATCTTCACTAGCTTTGCTATCCATTGGGTGCGCCAGGCCCCCGG ACACAGGCCTGAGTGGATGGGATGGATCAACCCCGGCAATGGTAACACACAGTATTCACAG AAGTTCCAGGGCAGAGTCACCATCACCAGGGACACATCCGCGAGCACAGCCAACATGGAGC TGAGCAGCCTGGGATCTGAAGACACGGCTGTTTATTTCTGTGCCCGATCAACCCCTTATTACG ATAGGACTGGTTCTTATTGGCTGGGTTACTTCCAGCACTGGGGCCAGGGCACCCTGGTCAGC GTCTCCTCAG (SEQ ID NO: 223) | | | | |
| QVQLVQSGAEVKKPGASVKVSCKASGYIFTSFAIHWVRQAPGHRPEWMGWINPGNGNTQYSQ KFQGRVTITRDTSASTANMELSSLGSEDTAVYFCARSTPYYDRTGSYWLGYFQHWGQGTLVS VSS (SEQ ID NO: 153) | | | | |
| **Light Chain** | **IGKV1D-33*01, IGKV1-33*01** | | **IGKJ4*01** | |
| GACGTCCAGATGACCCAGTCTCCATCCTCCCTGTCTGCATCTGTAGGAGACAGAGTCACCAT CACTTGCCAGGCGAGTCAGGACGTTACCAACTATTTAAATTGGTATCAGCAGAAACCAGGGC AAGCCCCTAAGGTCCTGATCTCCGATGCATCCGATTTGGAGACAGGGGTCCCGTCAAGGTTC AGTGGGTCTGGATCTGGGACAGATTTTACTTTCACCATCAGCAGCCTGCAGCCTGAAGATAT TGCAACATATTACTGTCAACAGTATGGTAATCTCCCTCTCACTTTCGGCGGAGGGACCAAGG TGGAGATCAAAC (SEQ ID NO: 224) | | | | |
| DVQMTQSPSSLSASVGDRVTITCQASQDVTNYLNWYQQKPGQAPKVLISDASDLETGVPSRFSG SGSGTDFTFTISSLQPEDIATYYCQQYGNLPLTFGGGTKVEIK (SEQ ID NO: 157) | | | | |

### Ab21. A80-338-19.6

The amino acid sequences for the heavy and light chains of the expressed version of **A80-338-19.6** can be found in Table 21 Variable heavy and light chain sequences were synthesized and cloned into immunoglobulin expression vectors containing human constant regions for the IgG1 heavy chain and kappa light chain. These vectors were used to express antibody that was purified.

**Table 21 A80-338-19.6 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-3*01** | **IGHD3-22*01** | **IGHJ1*01** | |
| CAGGTCCAGCTTGTGCAATCTGGGGCTGAGGTGAAGAAGCCCGGGGCCTCAGTGAAGGTTTC CTGCAAGGCTTCTGGATACATCTTCACTAGCTTTGCTCTTCATTGGGTGCGCCAGGCCCCCGG ACAAAGGCCTGAGTGGATGGGGTGGATCAACGCTGGCAATGGTGACACAGAATATTCACAG AAGTTCCAGGGCAGAGTCACCATTACCAGGGACACATCCGCGACCACAGCGACCATGGAGC TGAGCAGCCTGAGATCTGAAGACACGGCTGTTTATTTCTGTGCCCGGTCAACCCCTTACTATG ATAGGCGTGGTTATTACTGGCTAAATTACTTCCAGCACTGGGGCCAGGGCACCCTGGTCACC GTCTCCTCAG (SEQ ID NO: 225) | | | | |
| QVQLVQSGAEVKKPGASVKVSCKASGYIFTSFALHWVRQAPGQRPEWMGWINAGNGDTEYSQ KFQGRVTITRDTSATTATMELSSLRSEDTAVYFCARSTPYYDRRGYYWLNYFQHWGQGTLVT VSS (SEQ ID NO: 161) | | | | |
| **Light Chain** | **IGKV1D-33*01, IGKV1-33*01** | | **IGKJ4*01** | |
| GACATCCAGATGACCCAGTCTCCATCCTCCCTGTCTGCATCTGTAGGAGACAGAGTCACCAT CACTTGCCAGGCGAGTCAGGACATTAGCAACCATTTAAACTGGTATCAGCAGAAACCAGGA AAAGCCCCTAAGGTCCTGATCTCCGATGCATCCGATTTGGAAACAGGGGTCCCATCAAGGTT CAGTGGAAGTGGATCTGGGACAGATTTTACTTTCATCATCAACAGCCTGCAGCCTGAAGACT TTGCAACATATTACTGTCAGCAGTATGATAATCTCCCTCTCACTTTCGGCGGAGGGACCAAG GTGGAGACCAAAC (SEQ ID NO: 226) | | | | |
| DIQMTQSPSSLSASVGDRVTITCQASQDISNHLNWYQQKPGKAPKVLISDASDLETGVPSRFSGS GSGTDFTFIINSLQPEDFATYYCQQYDNLPLTFGGGTKVETK (SEQ ID NO: 165) | | | | |

### Ab22. A80-338-19.8

The amino acid sequences for the heavy and light chains of the expressed version of **A80-338-19.8** can be found in Table 22 Variable heavy and light chain sequences were synthesized and cloned into immunoglobulin expression vectors containing human constant regions for the IgG1 heavy chain and kappa light chain. These vectors were used to express antibody that was purified.

**Table 22 A80-338-19.8 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-3*01** | **IGHD3-22*01** | **IGHJ1*01** | |
| CAGGTCCAGCTCGTGCAGTCTGGGGCTGAGGTGAAGAAGCCTGGGGCCTCAGTGAAGGTTTC CTGCAAGTCTTCTGGATACACCTTCACAAGCCATAACTTTGGTATTCATTGGGTGCGCCTGGC CCCCGGACAACGGCTTGAGTGGATGGGGTGGATCAACACTGGCAACGGTAACACAAAGTAT | | | | |
| TCGCAGAAGTCCCAGGGCAGAGTCACCATTACTAGGGACACATCCGCGAGCACAGCCTACA TGGAGCTGAGCAACCTGAACTCTGAAGACACGGCTGTTTATTACTGTGCCCGATCAACCCCT TATTATGATAGAAGTGGTCATTACTGCCTGGGTTACTTCCAGCACTGGGGCCAGGGCACCCT GGTCTCCGTCTCCTCGG (SEQ ID NO: 227) | | | | |
| QVQLVQSGAEVKKPGASVKVSCKSSGYTFTSHNFGIHWVRLAPGQRLEWMGWINTGNGNTK YSQKSQGRVTITRDTSASTAYMELSNLNSEDTAVYYCARSTPYYDRSGHYCLGYFQHWGQGT LVSVSS (SEQ ID NO: 169) | | | | |
| **Light Chain** | **IGKV1D-33*01, IGKV1-33*01** | | **IGKJ4*01** | |
| GACATCCAGATGACCCAGTCGCCATCCTCCCTGTCTGCATCTGTAGGAGACAGAGTCACCAT CACTTGCCAGGCGAGTCAGGACATTAATAACTATTTAAATTGGTATCAGCAAAAACCAGGG AAGCCCCTAAGGTCGTGATCTTCGATGCAACCGATTTGGAAACAGGGGTCCCATCAAGGTTC AGTGGAAGTGGATCTGGGACAGATTTTACTTTCACCATTAGCAGCCTGCAGCCTGAAGATAT TGCAACATATTACTGTCAACAGGATGGTAATCTCCCTCTCACTTTCGGCGGAGGGACCAAGC TGGAGATCAAAC (SEQ ID NO: 228) | | | | |
| DIQMTQSPSSLSASVGDRVTITCQASQDINNYLNWYQQKPGEAPKVVIFDATDLETGVPSRFSGS GSGTDFTFTISSLQPEDIATYYCQQDGNLPLTFGGGTKLEIK (SEQ ID NO: 173) | | | | |

### Ab23. A80-338-19.9

The amino acid sequences for the heavy and light chains of the expressed version of **A80-338-19.9** can be found in Table 23 Variable heavy and light chain sequences were synthesized and cloned into immunoglobulin expression vectors containing human constant regions for the IgG1 heavy chain and kappa light chain. These vectors were used to express antibody that was purified.

**Table 23 A80-338-19.9 Gene families, nucleotide sequences and amino acid sequences for heavy and light chain are shown. Amino acid CDR regions are bolded.**

| | **V-gene Family** | **D-gene Family** | **J-gene Family** | |
|---|---|---|---|---|
| **Heavy Chain** | **IGHV1-3*01** | **IGHD3-22*01** | **IGHJ1*01** | |
| CAGGTCCAGCTTGTGCAGTCTGGGGCTGAGGTGAAGAAGCCTGGGGCCTCAGTGAAGGTTTC CTGCAAGGCTTCTGGATACATCTTCACTAGCTTTGCTATCCATTGGGTGCGCCAGGCCCCCGG ACAAAGGCTTGAGTGGATGGGATGGATCAACGCTGGCAATGGTAACACAGAATATTCACAG AAGTTCCAGGGCAGAGTCACCATTACCAGGGACACATCCGCGAGCACAGCCAACATGTACC TGAGCAGCCTGAGATCTGAAGACACGGCTGTGTATTATTGTGCCCGATCAACCCCTTATTAT GATAGGACTGGTTCTTATTGGCTGGGTTACTTCCAGCACTGGGGCCAGGGCACCCTGGTCAG CGTCTCCTCAG (SEQ ID NO: 229) | | | | |
| QVQLVQSGAEVKKPGASVKVSCKASGYIFTSFAIHWVRQAPGQRLEWMGWINAGNGNTEYSQ KFQGRVTITRDTSASTANMYLSSLRSEDTAVYYCARSTPYYDRTGSYWLGYFQHWGQGTLVS VSS (SEQ ID NO: 177) | | | | |
| **Light Chain** | **IGKV1D-33*01, IGKV1-33*01** | | **IGKJ4*01** | |
| GACATCCAGATGACCCAGTCTCCATCCTCCCTGTCTGCATCTGTAGGAGACAGAGTCACCAT CACTTGCCAGGCGAGTCAGGACGTTAGCAACTTTTTAAATTGGTATCAGCAGAAACCAGGTG AAGCCCCTAAGGTCCTGATCTCCGATGCATCCAGTTTGGAGACAGGGGTCCCATCAAGGTTC AGTGGGTCTGGATCTGGGACAGATTTTACTTTCACCATCAGCAGCCTGCAGCCTGAAGATAT TGCAACATATTACTGTCAACAGTATGGTAATCTCCCTCTCACTTTCGGCGGAGGGACCAAGG TGGAGATCAAAC (SEQ ID NO: 230) | | | | |
| DIQMTQSPSSLSASVGDRVTITCQASQDVSNFLNWYQQKPGEAPKVLISDASSLETGVPSRFSGSG SGTDFTFTISSLQPEDIATYYCQQYGNLPLTFGGGTKVEIK (SEQ ID NO: 181) | | | | |

### Example 4

### Combinations

The antibodies can be used as combinations, either as cocktails of two or more antibodies delivered as a mixture, or two or more combined in a single molecule as a multispecific. The advantages of such combinations include increased breadth of neutralization against variants, and synergistic effects leading to increased potency.

Combinations can have increased breadth of neutralization against variants compared to single antibodies, for example if certain variants are more sensitive to one or the other antibody in a combination. The disclosed antibodies can be used in combination to increase breadth across SARS-2 like viruses, SARS1 like, or both. For example, a useful combination would consist of: antibody A18-618-448.1, which is potently neutralizing against nearly all strains tested including SARS-1, but ineffective against the sarbecoviruses Pangolin GX-P2V and Pangolin GX-P5L; with a Group 4 antibody such as F768-104_G7, which neutralizes Pangolin GX-P2V and Pangolin GX-P5L but not SARS-1 and related viruses. Another example would be F768-104_B2 combined with a Group 3 antibody such as A80-102_G5 which potently neutralizes all tested SARS-CoV-2- related animal viruses and SARS-1 related viruses, and has breadth with modest potency against recent SARS-CoV-2 variants.

In addition, synergy may occur when binding of one antibody stabilizes or induces a spike conformation, for example locking the spike RBD domain into the Up conformation, that increases binding of a second antibody. This leads to improved potency of neutralization. The effects of combining antibodies were measured in a synergy test and found that antibodies F768-104_B2 combined with A18-448.1 showed synergistic improvement of neutralization potency against variants XBB.1.5 and EG.5.1 (Table 4; FIG. 6). F768-104_B2 also showed synergistic improvement of neutralization potency against variant XBB.1.5. when combined with antibody F769-E12, previously filed under # PCT 63/433,719. In addition, A18-448.1 showed synergistic improvement of neutralization potency against variants XBB.1.5 or EG.5.1 when combined with antibodies F769-E12, F770-G11, or F770-E8 previously filed under # PCT 63/433,719 (Table 4; FIG. 6).

### Example 5

### Additional Data

The SARS-CoV-2 JN.1 variant evolved in late 2023 and outcompeted the previously predominant XBB lineages in early 2024 (Kaku et al., Lancet Infect Dis 2024; 24(2): e82). Subsequently, JN.1 subvariants, including KP.2, KP.3, KP.2.3, LB.1, and KP.3.1.1, emerged concurrently (Kaku et al., Lancet Infect Dis 2024; 24(7): e416 ; Kaku et al., Lancet Infect Dis 2024; 24(8): e482-3; Kaku et al., Lancet Infect Dis 2024; 24(10): e609). In October 2024, KP.3.1.1 was the most predominant SARS-CoV-2 variant in the world (Kaku et al., Lancet Infect Dis 2024; 24(10): e609). XEC, first identified in Germany, quickly spread globally and has become a current concern (Kaku et al., Virological characteristics of the SARS-CoV-2 XEC variant, bioRxiv preprint, available on the internet, doi.org/10.1101/2024.10.16.618773). The KP.2, KP.2.3, KP.3, and LB.1 lineages evade neutralization by antibodies induced by infection or vaccination with greater efficiency than JN.1, particularly in individuals without hybrid immunity (Chen et al., Vaccines 2024, 12(11), 1236). Compared to KP.3, both KP.3.1.1 and XEC further evade neutralizing antibodies in convalescent plasma, even those elicited by KP.2-like breakthrough infections, and exhibit increased resistance against monoclonal neutralizing antibodies targeting various epitopes on the receptor-binding domain (RBD) (Liu et al., Enhanced immune evasion of SARS-CoV-2 KP.3.1.1 and XEC through NTD glycosylation. bioRxiv preprint, available on the internet, doi.org/10.1101/2024.10.23.619754).

Many of the disclosed antibodies have neutralizing activity against these variants (FIG. 7; Table 5). For example, A18-448.1 (class IV), F768-104_B2 (class III) and A63-1.4 (class I) potently neutralized all tested SARS-CoV-2 variants. In addition, other class IV (A80-102_G5 and A80-19.8) and site V (A80-103_E2, A43-10.4, amongst others) antibodies also neutralized all SARS-CoV-2 variants, albeit with lower potency. A18-448.1 potently neutralizes all the variants, including JN.1 sub-lineages, with single-digit ng/ml IC₅₀ values, making it 20-40 times more potent than the current EUA antibody, PEMGARDA^{™} (pemivibart) (See the FDA website, fda.gov/media/177067/download?utm_medium= email&utm_source=govdelivery). In contrast, A80-102_G5 weakly neutralizes SARS-CoV-2 variants and CR3022 does not neutralize the tested variants. F768-104_B2 and A63-1.4 also neutralized all the variants, including JN.1 sublineages, with IC₅₀s ranging from 24 to 591ng/ml).

Structural studies were also performed. A18-448.1, A80-102_G5 and CR3022 were shown to bind the same side of the RBD. Monoclonal antibody A18-448.1 binds to a distinct, smaller epitope compared to A80-102_G5 and CR3022 (FIGS. 8A-8C). As A18-448.1, F768-104_B2 and A63-1.4 target diverse epitopes of the RBD, they can be used in combination cocktails or in multi-specific antibodies to prevent or treat COVID-19.

A18-448.1, F768-104_B2, and A80-102_G5 protected hamsters against SARS-CoV-2 EG.5.1 challenge, showing significantly reduced loss of body weight and lung viral load, which correlated with *in vitro* neutralization potency (FIGS. 9A-9F).

In view of the many possible aspects to which the principles of the disclosed invention may be applied, it should be recognized that the illustrated aspects are only preferred examples of the invention and should not be taken as limiting the scope of the invention. Rather, the scope of the invention is defined by the following claims. We therefore claim as our invention all that comes within the scope and spirit of these claims.

Furthermore, the present invention relates to the following items:
1. An isolated monoclonal antibody or antigen binding fragment thereof, comprising a heavy chain variable (V_{H}) region and a light chain variable region (V_{L}) comprising a heavy chain complementarity determining region (HCDR)1, a HCDR2, and a HCDR3, and a light chain complementarity determining region (LCDR)1, a LCDR2, and a LCDR3 of the V_{H} and V_{L} set forth as any one of:
   a) SEQ ID NOs: 1 and 5, respectively (A18-618-448.1);
   b) SEQ ID NOs: 9 and 13, respectively (F768-104_B2);
   c) SEQ ID NOs: 17 and 21, respectively (E184-105_F3);
   d) SEQ ID NOs: 25 and 29, respectively (A63-652-32.1);
   e) SEQ ID NOs: 33 and 37, respectively (F768-104_G7);
   g) SEQ ID NOs: 41 and 45, respectively (E184-105_H1);
   f) SEQ ID NOs: 49 and 53, respectively (F768-104_D10);
   h) SEQ ID NOs: 57 and 61, respectively (A63-652-1.4);
   i) SEQ ID NOs: 65 and 69, respectively (E184-105_E5);
   j) SEQ ID NOs: 73 and 77, respectively (E184-105_H9);
   k) SEQ ID NOs: 81 and 85, respectively (E184-105_H2);
   l) SEQ ID NOs: 89 and 93, respectively (A80-102+103_G1);
   m) SEQ ID NOs: 97 and 101, respectively (A80-103_E2);
   n) SEQ ID NOs: 105 and 109, respectively (A80-103_B6);
   o) SEQ ID NOs: 113 and 117, respectively (A18-618-452.1);
   p) SEQ ID NOs: 121 and 125, respectively (A80-102+103_C1);
   q) SEQ ID NOs: 129 and 133, respectively (A43-d321.10.4);
   r) SEQ ID NOs: 137 and 141, respectively (F768-100-pt2_A05);
   s) SEQ ID NOs: 145 and 149, respectively (A80-102_G5);
   t) SEQ ID NOs: 153 and 157, respectively (A18-338-19.5);
   u) SEQ ID NOs: 161 and 165, respectively (A18-338-19.6);
   v) SEQ ID NOs: 169 and 173, respectively (A18-338-19.8); or
   w) SEQ ID NOs: 177 and 181, respectively (A18-338-19.9),
   wherein the monoclonal antibody or antigen binding fragment specifically binds severe acute respiratory syndrome coronavirus (SARS CoV)-2.
2. The isolated monoclonal antibody or antigen binding fragment of item 1, wherein the HCDR1, the HCDR2, the HCDR3, the LCDR1, the LCDR2, and the LCDR3 comprise the amino acids sequences set forth as any one of:
   a) SEQ ID NOs: 2, 3, 4, 6, 7 (AAS), and 8;
   b) SEQ ID NOs: 10, 11, 12, 14, 15 (GAS), and 16;
   c) SEQ ID NOs: 18, 19, 20, 22, 23 (GAS), and 24;
   d) SEQ ID NOs: 26, 27, 28, 30, 31 (AAS), and 32;
   e) SEQ ID NOs: 34, 35, 36, 38, 39 (AAS), and 40;
   f) SEQ ID NOs: 42, 43, 44, 46, 47 (DAS), and 48;
   g) SEQ ID NOs: 50, 51, 52, 54, 55 (GAS), and 56;
   h) SEQ ID NOs: 58, 59, 60, 62, 63 (DAS), and 64;
   i) SEQ ID NOs: 66, 67, 68, 70, 71 (AAS), and 72;
   j) SEQ ID NOs: 74, 75, 76, 78, 79 (AAS), and 80;
   k) SEQ ID NOs: 82, 83, 84, 86, 87 (AAS), and 88;
   l) SEQ ID NOs: 90, 91, 92, 94, 95 (NNN), and 96;
   m) SEQ ID NOs: 98, 99, 100, 102, 103 (SDS), and 104;
   n) SEQ ID NOs: 106, 107, 108, 110, 111 (SYN), and 112;
   o) SEQ ID NOs: 114, 115, 116, 118, 119 (AAS), and 120;
   p) SEQ ID NOs: 122, 123, 124, 126, 127 (DAS), and 128;
   q) SEQ ID NOs: 130, 131, 132, 134, 135 (DAS), and 136;
   r) SEQ ID NOs: 138, 139, 140, 142, 143 (WAS), and 144;
   s) SEQ ID NOs: 146, 147, 148, 150, 151 (DAS), and 152;
   t) SEQ ID NOs: 154, 155, 156, 158, 159 (DAS), and 160;
   u) SEQ ID NOs: 162, 163, 164, 166, 167 (DAS), and 168;
   v) SEQ ID NOs: 170, 171, 172, 174, 175 (DAT), and 176; or
   w) SEQ ID NOs: 178, 179, 180, 182, 183 (DAS), and 184,
   respectively.
3. The isolated monoclonal antibody or antigen binding fragment of item 1 or item 2, wherein the V_{H} and the V_{L} comprise the amino acid sequences at least 90% identical to the amino acid sequences set forth as any one of:
   a) SEQ ID NOs: 1 and 5;
   b) SEQ ID NOs: 9 and 13;
   c) SEQ ID NOs: 17 and 21;
   d) SEQ ID NOs: 25 and 29;
   e) SEQ ID NOs: 33 and 37;
   f) SEQ ID NOs: 41 and 45;
   g) SEQ ID NOs: 49 and 53;
   h) SEQ ID NOs: 57 and 61;
   i) SEQ ID NOs: 65 and 69;
   j) SEQ ID NOs: 73 and 77;
   k) SEQ ID NOs: 81 and 85;
   l) SEQ ID NOs: 89 and 93;
   m) SEQ ID NOs: 97 and 101;
   n) SEQ ID NOs: 105 and 109;
   o) SEQ ID NOs: 113 and 117;
   p) SEQ ID NOs: 121 and 125;
   q) SEQ ID NOs: 129 and 133;
   r) SEQ ID NOs: 137 and 141;
   s) SEQ ID NOs: 145 and 149;
   t) SEQ ID NOs: 153 and 157;
   u) SEQ ID NOs: 161 and 165;
   v) SEQ ID NOs: 169 and 173; or
   w) SEQ ID NOs: 177 and 181,
   respectively.
4. The isolated monoclonal antibody or antigen binding fragment of any one of the prior items, comprising a human framework region.
5. The isolated monoclonal antibody or antigen binding fragment of any one of the prior items, wherein the V_{H} and the V_{L} comprise the amino acid sequences set forth as any one of:
   a) SEQ ID NOs: 1 and 5;
   b) SEQ ID NOs: 9 and 13;
   c) SEQ ID NOs: 17 and 21;
   d) SEQ ID NOs: 25 and 29;
   e) SEQ ID NOs: 33 and 37;
   f) SEQ ID NOs: 41 and 45;
   g) SEQ ID NOs: 49 and 53;
   h) SEQ ID NOs: 57 and 61;
   i) SEQ ID NOs: 65 and 69;
   j) SEQ ID NOs: 73 and 77;
   k) SEQ ID NOs: 81 and 85;
   l) SEQ ID NOs: 89 and 93;
   m) SEQ ID NOs: 97 and 101;
   n) SEQ ID NOs: 105 and 109;
   o) SEQ ID NOs: 113 and 117;
   p) SEQ ID NOs: 121 and 125;
   q) SEQ ID NOs: 129 and 133;
   r) SEQ ID NOs: 137 and 141;
   s) SEQ ID NOs: 145 and 149;
   t) SEQ ID NOs: 153 and 157;
   u) SEQ ID NOs: 161 and 165;
   v) SEQ ID NOs: 169 and 173; or
   w) SEQ ID NOs: 177 and 181,
   respectively.
6. The isolated monoclonal antibody of any one of the prior items, wherein the monoclonal antibody comprises a human constant domain.
7. The isolated monoclonal antibody of any one of the prior items, wherein the monoclonal antibody is a human antibody.
8. The isolated monoclonal antibody of any one of the prior items, wherein the monoclonal antibody is an IgG.
9. The isolated monoclonal antibody of any one of the prior items comprising a recombinant constant domain comprising a modification that increases the half-life of the monoclonal antibody.
10. The isolated monoclonal antibody of item 9, wherein the modification increases binding to the neonatal Fc receptor.
11. The isolated monoclonal antibody or antigen binding fragment of any one of items 1-10, wherein the monoclonal antibody neutralizes SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 and/or SHC-014.
12. The antigen binding fragment of any one of items 1-5 or 11.
13. The antigen binding fragment of item 12, wherein the antigen binding fragment is a Fv, Fab, F(ab')₂, scFV or a scFV₂ fragment.
14. The isolated monoclonal antibody or antigen binding fragment of any one of items 1-13, conjugated to a detectable marker.
15. A multi-specific antibody comprising the monoclonal antibody or antigen binding fragment of any one of items 1-14.
16. An isolated nucleic acid molecule encoding the monoclonal antibody or antigen binding fragment of any one of items 1-15, or a V_{H} or V_{L} of the monoclonal antibody.
17. The nucleic acid molecule of item 16, wherein the nucleic acid molecule is a cDNA sequence encoding the V_{H} and the V_{L}.
18. The nucleic acid molecule of item 16 or 17, operably linked to a promoter.
19. A vector comprising the nucleic acid molecule of any one of items 16-18.
20. A host cell comprising the nucleic acid molecule of any one of items 16-20, or the vector of item 19.
21. A pharmaceutical composition for use in inhibiting a coronavirus infection, comprising an effective amount of the monoclonal antibody or antigen binding fragment of any one of items 1-14, the multispecific antibody of item 15, the nucleic acid molecule of any one of items 16-18, or the vector of item 19; and
   a pharmaceutically acceptable carrier.
22. A method of producing an antibody or antigen binding fragment that specifically binds to a coronavirus spike protein, comprising:
   expressing one or more nucleic acid molecules encoding the monoclonal antibody, antigen binding fragment of any one of items 1-14 in a host cell; and
   purifying the antibody or antigen binding fragment.
23. A method of detecting the presence of a SARS CoV-2 in a biological sample from a subject, comprising:
   contacting the biological sample with an effective amount of the monoclonal antibody or antigen binding fragment of any one of items 1-14 under conditions sufficient to form an immune complex; and
   detecting the presence of the immune complex in the biological sample, wherein the presence of the immune complex in the biological sample indicates the presence of the SARS CoV-2 in the sample.
24. The method of item 23, wherein detecting the detecting the presence of the immune complex in the biological sample indicates that the subject has a SARS-CoV-2 infection.
25. A method of inhibiting a SARS CoV-2 infection in a subject, comprising administering an effective amount of the monoclonal antibody or antigen binding fragment of any one of items 1-14, the multispecific antibody of item 15, the nucleic acid molecule of any one of items 16-18, or the vector of item 19, or the pharmaceutical composition of item 21 to the subject, wherein the subject has or is at risk of a SARS CoV-2 infection.
26. The method of item 25, wherein the coronavirus is SARS-CoV-2 D614G, BA.1, BA.2, BA.4/5, BA.2.75.2, BQ.1.1, XBB.1, XBB.1.5, XBB.1.16, CH.1.1, XBB.2.3.2, XBC.1, EG.5.1, XBB.1.16.6, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.
27. Use of the monoclonal antibody, antigen binding fragment, multispecific antibody, nucleic acid molecule, vector, or pharmaceutical composition of any one of items 1-21 to inhibit a SARS CoV-2 infection in a subject or to detect the presence of a SARS CoV-2 in a biological sample.
28. The use of item 27, wherein the SARS CoV-2 is SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.

## Claims

1. An isolated monoclonal antibody or antigen binding fragment thereof, comprising a heavy chain variable (V_{H}) region and a light chain variable region (V_{L}) comprising a heavy chain complementarity determining region (HCDR)1, a HCDR2, and a HCDR3, and a light chain complementarity determining region (LCDR)1, a LCDR2, and a LCDR3 of the V_{H} and V_{L} set forth as:
SEQ ID NOs: 9 and 13, respectively (F768-104_B2),
wherein the monoclonal antibody or antigen binding fragment specifically binds severe acute respiratory syndrome coronavirus (SARS CoV)-2.

2. The isolated monoclonal antibody or antigen binding fragment of claim 1, wherein the HCDR1, the HCDR2, the HCDR3, the LCDR1, the LCDR2, and the LCDR3 comprise the amino acids sequences set forth as:
SEQ ID NOs: 10, 11, 12, 14, 15 (GAS), and 16; and,
optionally comprising a human framework region.

3. The isolated monoclonal antibody or antigen binding fragment of any one of the prior claims, wherein the V_{H} and the V_{L} comprise the amino acid sequences set forth as:
SEQ ID NOs: 9 and 13;
or wherein the V_{H} and the V_{L} respectively comprise the amino acid sequences at least 90% identical to the amino acid sequences set forth as SEQ ID NOs: 9 and 13.

4. The isolated monoclonal antibody of any one of the prior claims, wherein
a) the monoclonal antibody comprises a human constant domain, preferably, wherein the monoclonal antibody is a human antibody, more preferably, wherein the monoclonal antibody is an IgG; and/or
b) the monoclonal antibody comprises a recombinant constant domain comprising a modification that increases the half-life of the monoclonal antibody, preferably, wherein the modification increases binding to the neonatal Fc receptor.

5. The isolated monoclonal antibody or antigen binding fragment of any one of claims 1-4, wherein the monoclonal antibody neutralizes SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 and/or SHC-014, and/or wherein the antigen binding fragment is a Fv, Fab, F(ab')₂, scFV or a scFV₂ fragment.

6. The isolated monoclonal antibody or antigen binding fragment of any one of claims 1-5, conjugated to a detectable marker.

7. A multi-specific antibody comprising the monoclonal antibody or antigen binding fragment of any one of claims 1-6.

8. An isolated nucleic acid molecule encoding the monoclonal antibody or antigen binding fragment of any one of claims 1-7, or a V_{H} or V_{L} of the monoclonal antibody, preferably, wherein the nucleic acid molecule is a cDNA sequence encoding the V_{H} and the V_{L}, optionally, wherein said nucleic acid molecule is operably linked to a promoter.

9. A vector comprising the nucleic acid molecule of claim 8.

10. A host cell comprising the nucleic acid molecule of claim 8, or the vector of claim 9.

11. A pharmaceutical composition for use in inhibiting a coronavirus infection, comprising an effective amount of the monoclonal antibody or antigen binding fragment of any one of claims 1-6, the multi-specific antibody of claim 7, the nucleic acid molecule of claim 8, or the vector of claim 9; and
a pharmaceutically acceptable carrier.

12. A method of producing an antibody or antigen binding fragment that specifically binds to a coronavirus spike protein, comprising:
expressing one or more nucleic acid molecules encoding the monoclonal antibody, antigen binding fragment of any one of claims 1-6 in a host cell; and
purifying the antibody or antigen binding fragment.

13. A method of detecting the presence of a SARS CoV-2 in a biological sample from a subject, comprising:
contacting the biological sample with an effective amount of the monoclonal antibody or antigen binding fragment of any one of claims 1-6 under conditions sufficient to form an immune complex; and
detecting the presence of the immune complex in the biological sample, wherein the presence of the immune complex in the biological sample indicates the presence of the SARS CoV-2 in the sample, preferably, wherein detecting the detecting the presence of the immune complex in the biological sample indicates that the subject has a SARS-CoV-2 infection.

14. An effective amount of the monoclonal antibody or antigen binding fragment of any one of claims 1-6, the multi-specific antibody of claim 7, the nucleic acid molecule of claim 8, or the vector of claim 9, or the pharmaceutical composition of claim 11 for use in a method of inhibiting a SARS CoV-2 infection in a subject, , wherein the subject has or is at risk of a SARS CoV-2 infection, preferably, wherein the coronavirus is SARS-CoV-2 D614G, BA.1, BA.2, BA.4/5, BA.2.75.2, BQ.1.1, XBB.1, XBB.1.5, XBB.1.16, CH.1.1, XBB.2.3.2, XBC.1, EG.5.1, XBB.1.16.6, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.

15. Use of the monoclonal antibody, antigen binding fragment, multi-specific antibody, nucleic acid molecule, vector, or pharmaceutical composition of any one of claims 1-11 to detect the presence of a SARS CoV-2 in a biological sample, preferably, wherein the SARS CoV-2 is SARS-CoV-2 BA.1, BA.2, BA.4/5, BA.2.75.2, XBB.1.5, EG.5.1, FL.1.5.1, BA.2.86, JN.1, KP.2, KP.3, KP.2.3, LB.1, KP.3.1.1 and/or XEC, RaTG13, Pangolin GD, Pangolin GX-P2V, WIV1 or SHC-014.
